# EUROPEAN PATENT SPECIFICATION

(11) **EP 4 190 352 B1**
(45) Date of publication and mention of the grant of the patent: **25.03.2026**
(21) Application number: 21849910.1
(22) Date of filing: 26.07.2021
(51) Int. Cl.: A61K 9/00, A61K 39/395, A61P 35/00, A61K 9/08, A61K 9/19, A61K 47/12, A61K 47/18, A61K 39/00, C07K 16/28

(54) **FORMULATION OF NOVEL BISPECIFIC ANTI-CD3/CD20 POLYPEPTIDE COMPLEX**
FORMULIERUNG EINES NEUARTIGEN BISPEZIFISCHEN ANTI-CD3/CD20-POLYPEPTIDKOMPLEXES
NOUVELLE FORMULATION DE COMPLEXE POLYPEPTIDIQUE ANTI-CD3/CD20 BISPÉCIFIQUE

(30) Priority: 27.07.2020 CN 202010733597
(43) Date of publication of application: 07.06.2023
(73) Proprietor: CHIA TAI TIANQING PHARMACEUTICAL GROUP CO., LTD., Lianyungang, Jiangsu 222062 (CN)
(72) Inventor: ZHU, Youwei, Lianyungang, Jiangsu 222062 (CN); LI, Yingchun, Lianyungang, Jiangsu 222062 (CN); CHENG, Yanju, Lianyungang, Jiangsu 222062 (CN)
(74) Representative: Kraus & Lederer PartGmbB
(86) International application number: PCT/CN2021/108455
(87) International publication number: WO 2022/022464

(56) References cited:
- WO-A1-2018/223004
- WO-A1-2019/160904
- WO-A1-2019/228406
- WO-A1-2020/084608
- CN-A- 111 108 119
- CN-A- 111 138 546
- WANG W ET AL: "ANTIBODY STRUCTURE, INSTABILITY, AND FORMULATION", JOURNAL OF PHARMACEUTICAL SCIENCES, WILEY, vol. 96, no. 1, 1 January 2007 (2007-01-01), pages 1 - 26, XP009084505, ISSN: 0022-3549, DOI: 10.1002/JPS.20727
- ANN L DAUGHERTY AND RANDALL J MRSNY ED - STEVEN J SHIRE ET AL: "Formulation and Delivery Issues for Monoclonal Antibody Therapeutics", 1 January 2010, CURRENT TRENDS IN MONOCLONAL ANTIBODY DEVELOPMENT AND MANUFACTURING, SPRINGER, US, PAGE(S) 103 - 129, ISBN: 978-0-387-76642-3, XP009133774

## Description

### CROSS-REFERENCE TO RELATED APPLICATIONS

The present disclosure claims priority to the Chinese Patent Application No. 202010733597.6 filed on Jul. 27, 2020.

### TECHNICAL FIELD

The present disclosure is in the field of pharmaceutical formulation, and particularly relates to a bispecific anti-CD3/CD20 polypeptide complex formulation and compositions for use thereof

### BACKGROUND

The statements in BACKGROUND provide solely information related to the present disclosure and do not necessarily constitute the prior art.

As biological drugs have continuously developed, therapeutic antibodies have become the main drugs of choice for patients with cancer, autoimmune diseases, inflammation and other diseases. However, monoclonal antibodies are directed against single targets, and since the development of diseases such as cancer is multifactorial and involves multiple signaling pathways, single-target immunotherapy appears to be not enough to destroy cancer cells.

CD20 is an activated glycosylated phosphoprotein expressed on the surfaces of B lymphocytes. Rituximab (a chimeric anti-CD20 monoclonal antibody (hereinafter also referred to as "mAb")) was approved by FDA for the treatment of lymphoproliferative disorders as early as 1997. As the protein structure and molecular function of CD20 have been studied, a new-generation CD20-targeting therapeutic antibody ofatumumab (a fully human anti-CD20 therapeutic antibody) is developed, which targets a different CD20 epitope of greater proximity to cell surface than Rituximab, resulting in slower dissociation and more stably binding than rituximab (Laurenti L, Innocenti I, Autore F, et al. New developments in the management of chronic lymphocytic leukemia: role of ofatumumab. Onco Targets Ther. 2016 Jan 20; 9: 421-9). However, nearly all follicular lymphoma patients and CLL patients and about half of the patients with aggressive B cell lymphoma (e.g., diffuse large B cell lymphoma) will relapse or suffer a recurrence after anti-CD20 mAb treatment (Lim SH, Beers SA, French RR, et al. Anti-CD20 monoclonal antibodies: historical and future perspectives. Haematologica. 2010 Jan; 95(1):135-43).

The CD3 T cell receptor is a protein complex consisting of four distinct chains (a CD3 γ chain, a CD3 δ chain and two CD3 ε chains). These four chains associate with a molecule called T cell receptor (TCR) and an intracellular ζ chain to generate activation signals in T lymphocytes. The TCR, ζ chain and CD3 molecule constitute a TCR complex; TCR serves as a subunit for antigen recognition and binding, and CD3 as a subunit responsible for the transmission of antigenic stimulation to a signaling pathway and ultimately the regulation of T cell activity. The CD3 protein is present in nearly all T cells. The CD3-TCR complex modulates the T cell function in both innate and adaptive immune responses, as well as cellular and humoral immune functions, including elimination of pathogenic organisms and control of tumor growth through cytotoxic effects. Mouse monoclonal antibodies targeting human CD3, such as OKT3 (Kung et al, Science, 206: 347-9 (1979)), are the first generation of CD3 antibodies developed for therapy. Despite the strong immunosuppressive potency of OKT3, its clinical applications have been hampered by severe side effects associated with its immunogenicity and mitogenic potential (Chatenoud, Nature Reviews, 3:123-132 (2003)). OKT3 induces anti-globulin responses, promoting its quick clearance and neutralization (Chatenoud et al., Eur. J. Immunol.,137:830-8 (1982)). In addition, OKT3 induces extensive release of cytokines in the body (Hirsch et al., J. Immunol, 142:737-43 (1989)). Patent document WO 2018/223004 discloses compositions comprising bispecific anti-CD3/CD20 antibodies.

A bispecific antibody targeting CD3 and CD20 can bind to both a T cell and a B cell. Once the bispecific antibody binds to both a CD3-positive T cell and a CD20-positive B cell, the formation of cytolytic synapses will be promoted, and the cytotoxic T cell thereby release perforin and granzymes, inducing apoptosis of the B cell. However, as bispecific antibody drugs are large in molecular weight and complicated in structure, they are susceptible to degradation, aggregation or unwanted chemical modifications and the like and become unstable, particularly under high-temperature conditions or shear stress (for example, during transportation). In terms of rendering bispecific antibodies suitable for production and administration and maintaining their biological activity and stability during storage and subsequent use, the development of stable bispecific antibody pharmaceutical formulations is of particular importance.

### BRIEF SUMMARY

According to the present disclosure, a bispecific anti-CD3/CD20 polypeptide complex according to the claims can be formulated in the form of a liquid or lyophilized powder. The formulation of the present disclosure is capable of achieving a stabilizing effect, wherein the bispecific anti-CD3/CD20 polypeptide complex substantially retains its physical and/or chemical stability and/or biological activity after storage. Specifically, the formulation of the present disclosure with low level of bispecific anti-CD3/CD20 polypeptide complex aggregation, and has few MFI microparticles and insoluble microparticles, as well as good biological activity, rendering it suitable for administration in a subject.

In one aspect, the present disclosure provides a liquid formulation comprising a bispecific anti-CD3/CD20 polypeptide complex and a buffer, wherein the buffer includes one of or a combination of more of a histidine salt buffer, a succinate buffer and a citrate buffer.

In some embodiments, the histidine salt buffer is a histidine-histidine hydrochloride buffer, the succinate buffer is a succinic acid-sodium succinate buffer, and the citrate buffer is a citric acid-sodium citrate buffer.

In some embodiments, the buffer in the aforementioned liquid formulation is one of or a combination of more of a histidine-histidine hydrochloride buffer, a succinic acid-sodium succinate buffer and a citric acid-sodium citrate buffer. In some embodiments, the buffer in the aforementioned liquid formulation is a citric acid-sodium citrate buffer.

According to the claims, the bispecific anti-CD3/C20 polypeptide complex in the aforementioned liquid formulation is at a concentration of about 0.1 mg/mL to about 50 mg/mL. In some embodiments, the bispecific anti-CD3/C20 polypeptide complex in the aforementioned liquid formulation is at a concentration of about 0.5 mg/mL to about 20 mg/mL, e.g., about 0.5 mg/mL, about 1 mg/mL, about 1.5 mg/mL, about 2 mg/mL, about 2.5 mg/mL, about 3 mg/mL, about 3.5 mg/mL, about 4 mg/mL, about 4.5 mg/mL, about 5 mg/mL, about 5.5 mg/mL, about 6 mg/mL, about 6.5 mg/mL, about 7 mg/mL, about 7.5 mg/mL, about 8 mg/mL, about 8.5 mg/mL, about 9 mg/mL, about 9.5 mg/mL, about 10 mg/mL, about 11 mg/mL, about 12 mg/mL, about 13 mg/mL, about 14 mg/mL, about 15 mg/mL, about 16 mg/mL, about 17 mg/mL, about 18 mg/mL, about 19 mg/mL or about 20 mg/mL. In some embodiments, the bispecific anti-CD3/C20 polypeptide complex in the aforementioned liquid formulation is at a concentration of about 1 mg/mL to about 20 mg/mL, about 2 mg/mL to about 10 mg/mL, about 2 mg/mL to about 5 mg/mL, or about 2 mg/mL to about 3 mg/mL. In some embodiments, the bispecific anti-CD3/C20 polypeptide complex in the aforementioned liquid formulation is at a concentration of about 2 mg/mL. In some embodiments, the bispecific anti-CD3/C20 polypeptide complex in the aforementioned liquid formulation is at a concentration of about 3 mg/mL. In some embodiments, the bispecific anti-CD3/C20 polypeptide complex in the aforementioned liquid formulation is at a concentration of about 5 mg/mL. In some embodiments, the bispecific anti-CD3/C20 polypeptide complex in the aforementioned liquid formulation is at a concentration of about 10 mg/mL.

According to the claims, the bispecific anti-CD3/C20 polypeptide complex in the aforementioned liquid formulation is at a concentration of 0.1 mg/mL to 50 mg/mL. In some embodiments, the bispecific anti-CD3/C20 polypeptide complex in the aforementioned liquid formulation is at a concentration of 0.5 mg/mL to 20 mg/mL, e.g., 0.5 mg/mL, 1 mg/mL, 1.5 mg/mL, 2 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL or 20 mg/mL. In some embodiments, the bispecific anti-CD3/C20 polypeptide complex in the aforementioned liquid formulation is at a concentration of 1 mg/mL to 20 mg/mL, 2 mg/mL to 10 mg/mL, 2 mg/mL to 5 mg/mL, or 2 mg/mL to 3 mg/mL. In some embodiments, the bispecific anti-CD3/C20 polypeptide complex in the aforementioned liquid formulation is at a concentration of 2 mg/mL. In some embodiments, the bispecific anti-CD3/C20 polypeptide complex in the aforementioned liquid formulation is at a concentration of 3 mg/mL. In some embodiments, the bispecific anti-CD3/C20 polypeptide complex in the aforementioned liquid formulation is at a concentration of 5 mg/mL. In some embodiments, the bispecific anti-CD3/C20 polypeptide complex in the aforementioned liquid formulation is at a concentration of 10 mg/mL.

In some embodiments, the buffer in the aforementioned liquid formulation has a pH of about 5.0 to about 7.5. In some embodiments, the buffer in the aforementioned liquid formulation has a pH of about 5.0 to about 6.5, e.g., about 5.0, about 5.1, about 5.2, about 5.3, about 5.4, about 5.5, about 5.6, about 5.7, about 5.8, about 5.9, about 6.0, about 6.1, about 6.2, about 6.3, about 6.4 or about 6.5. In some embodiments, the buffer in the aforementioned liquid formulation has a pH of about 6.0 to about 6.5. In some embodiments, the buffer in the aforementioned liquid formulation has a pH of about 6. In some embodiments, the buffer in the aforementioned liquid formulation has a pH of about 6.2. In some embodiments, the buffer in the aforementioned liquid formulation has a pH of about 6.5.

In some embodiments, the buffer in the aforementioned liquid formulation has a pH of 5.0 to 7.5. In some embodiments, the buffer in the aforementioned liquid formulation has a pH of 5.0 to 6.5, e.g., 5.0, 5.1, 5.2, 5.3, 5.4, 5.5, 5.6, 5.7, 5.8, 5.9, 6.0, 6.1, 6.2, 6.3, 6.4 or 6.5. In some embodiments, the buffer in the aforementioned liquid formulation has a pH of 6.0 to 6.5. In some embodiments, the buffer in the aforementioned liquid formulation has a pH of 6. In some embodiments, the buffer in the aforementioned liquid formulation has a pH of 6.2. In some embodiments, the buffer in the aforementioned liquid formulation has a pH of 6.5.

According to the claims, the buffer in the aforementioned liquid formulation has a concentration of about 1 mM to about 30 mM. In some embodiments, the buffer in the aforementioned liquid formulation has a concentration of about 5 mM to about 20 mM, e.g., about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM or about 20 mM. In some embodiments, the buffer in the aforementioned liquid formulation has a concentration of about 10 mM.

According to the claims, the buffer in the aforementioned liquid formulation has a concentration of 1 mM to 30 mM. In some embodiments, the buffer in the aforementioned liquid formulation has a concentration of 5 mM to 20 mM, e.g., 5 mM, 6 mM, 7 mM, 8 mM, 9 mM, 10 mM, 11 mM, 12 mM, 13 mM, 14 mM, 15 mM, 16 mM, 17 mM, 18 mM, 19 mM or 20 mM. In some embodiments, the buffer in the aforementioned liquid formulation has a concentration of 10 mM.

In some embodiments, the aforementioned liquid formulation has a pH of about 5.0 to about 7.5. In some embodiments, the aforementioned liquid formulation has a pH of about 5.0 to about 6.5, e.g., about 5.0, about 5.1, about 5.2, about 5.3, about 5.4, about 5.5, about 5.6, about 5.7, about 5.8, about 5.9, about 6.0, about 6.1, about 6.2, about 6.3, about 6.4 or about 6.5. In some embodiments, the aforementioned liquid formulation has a pH of about 6.0 to about 6.5. In some embodiments, the aforementioned liquid formulation has a pH of about 6. In some embodiments, the aforementioned liquid formulation has a pH of about 6.2. In some embodiments, the aforementioned liquid formulation has a pH of about 6.5.

In some embodiments, the aforementioned liquid formulation has a pH of 5.0 to 7.5. In some embodiments, the aforementioned liquid formulation has a pH of 5.0 to 6.5, e.g., 5.0, 5.1, 5.2, 5.3, 5.4, 5.5, 5.6, 5.7, 5.8, 5.9, 6.0, 6.1, 6.2, 6.3, 6.4 or 6.5. In some embodiments, the aforementioned liquid formulation has a pH of 6.0 to 6.5. In some embodiments, the aforementioned liquid formulation has a pH of 6. In some embodiments, the aforementioned liquid formulation has a pH of 6.2. In some embodiments, the aforementioned liquid formulation has a pH of 6.5. In some embodiments, the aforementioned liquid formulation further comprises an osmotic pressure regulator and/or a stabilizer. According to the claims, the osmotic pressure regulator and/or the stabilizer is a saccharide. As used herein, "saccharide" can be a monosaccharide, disaccharide, trisaccharide, polysaccharide, sugar alcohol or reducing sugar and non-reducing sugar, e.g., glucose, sucrose, trehalose, lactose, fructose, dextran, glycerol, erythritol, glycerol, arabitol, xylitol, sorbitol, mannitol, melibiose, melezitose, raffinose, mannotriose, stachyose, maltose, lactulose, maltulose, sorbitol, maltitol, lactitol, isomaltulose, and the like. In some embodiments, the osmotic pressure regulator and/or the stabilizer further comprise one or more of sodium chloride, arginine-hydrochloride, glycine and proline. In some embodiments, the osmotic pressure regulator and/or the stabilizer in the aforementioned liquid formulation are disaccharides, such as trehalose or sucrose. In some embodiments, the aforementioned liquid formulation comprises trehalose. In some embodiments, the aforementioned liquid formulation comprises trehalose and one or more of sodium chloride, arginine-hydrochloride, glycine and proline. In some embodiments, the aforementioned liquid formulation comprises sucrose and one or more of sodium chloride, arginine-hydrochloride, glycine and proline. In some embodiments, the aforementioned liquid formulation comprises trehalose and sodium chloride. In some embodiments, the aforementioned liquid formulation comprises trehalose and arginine-hydrochloride. In some embodiments, the aforementioned liquid formulation comprises sucrose and sodium chloride. In some embodiments, the aforementioned liquid formulation comprises sucrose and arginine-hydrochloride. In the formulation of the present application, saccharides include saccharides and/or hydrates thereof; for example, trehalose includes trehalose and/or a hydrate thereof such as trehalose dihydrate.

According to the claims, the saccharide in the aforementioned liquid formulation is at a concentration of about 150 mM to about 320 mM. In some embodiments, the saccharide in the aforementioned liquid formulation is at a concentration of about 180 mM to about 265 mM, e.g., about 180 mM, about 185 mM, about 198 mM, about 211 mM, about 214 mM, about 217 mM, about 219 mM, about 222 mM, about 225 mM, about 227 mM, about 230 mM, about 233 mM, about 235 mM, about 238 mM, about 241 mM, about 243 mM, about 246 mM, about 248 mM, about 251 mM, 254 mM, about 256 mM, about 259 mM, about 262 mM or about 264 mM. In some embodiments, the saccharide in the aforementioned liquid formulation is at a concentration of about 230 mM to about 240 mM, or about 230 mM to about 235 mM. In some embodiments, the saccharide in the aforementioned liquid formulation is at a concentration of about 233 mM. In some embodiments, the saccharide in the aforementioned liquid formulation is at a concentration of about 234 mM. In some embodiments, the saccharide in the aforementioned liquid formulation is at a concentration of about 180 mM to about 265 mM, e.g., about 180 mM, about 185.02 mM, about 198.24 mM, about 211.46 mM, about 214.1 mM, about 216.74 mM, about 219.39 mM, about 222.03 mM, about 224.67 mM, about 227.31 mM, about 229.96 mM, about 232.6 mM, about 235.24 mM, about 237.89 mM, about 240.53 mM, about 243.17 mM, about 245.82 mM, about 248.46 mM, about 251.1 mM, 253.75 mM, about 256.39 mM, about 259.03 mM, about 261.68 mM or about 264.32 mM. In some embodiments, the saccharide in the aforementioned liquid formulation is at a concentration of about 232.6 mM. In some embodiments, the saccharide in the aforementioned liquid formulation is at a concentration of about 233.7 mM.

In some embodiments, the trehalose in the aforementioned liquid formulation is at a concentration of about 150 mM to about 320 mM. In some embodiments, the trehalose in the aforementioned liquid formulation is at a concentration of about 180 mM to about 265 mM, e.g., about 180 mM, about 185 mM, about 198 mM, about 211 mM, about 214 mM, about 217 mM, about 219 mM, about 222 mM, about 225 mM, about 227 mM, about 230 mM, about 233 mM, about 235 mM, about 238 mM, about 241 mM, about 243 mM, about 246 mM, about 248 mM, about 251 mM, 254 mM, about 256 mM, about 259 mM, about 262 mM or about 264 mM. In some embodiments, the trehalose in the aforementioned liquid formulation is at a concentration of about 230 mM to about 240 mM, or about 230 mM to about 235 mM. In some embodiments, the trehalose in the aforementioned liquid formulation is at a concentration of about 233 mM. In some embodiments, the trehalose in the aforementioned liquid formulation is at a concentration of about 180 mM to about 265 mM, e.g., about 180 mM, about 185.02 mM, about 198.24 mM, about 211.46 mM, about 214.1 mM, about 216.74 mM, about 219.39 mM, about 222.03 mM, about 224.67 mM, about 227.31 mM, about 229.96 mM, about 232.6 mM, about 235.24 mM, about 237.89 mM, about 240.53 mM, about 243.17 mM, about 245.82 mM, about 248.46 mM, about 251.1 mM, 253.75 mM, about 256.39 mM, about 259.03 mM, about 261.68 mM or about 264.32 mM. In some embodiments, the trehalose in the aforementioned liquid formulation is at a concentration of about 232.6 mM.

In some embodiments, the sodium chloride, arginine-hydrochloride, glycine or proline in the aforementioned liquid formulation is at a concentration of about 10 mM to about 300 mM. In some embodiments, the sodium chloride, arginine-hydrochloride, glycine or proline in the aforementioned liquid formulation is at a concentration of about 20 mM to about 200 mM, e.g., about 20 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM, about 100 mM, about 105 mM, about 110 mM, about 115 mM, about 120 mM, about 125 mM, about 130 mM, about 135 mM, about 140 mM, about 145 mM, about 150 mM, about 160 mM, about 170 mM, about 180 mM, about 190 mM or about 200 mM. In some embodiments, the sodium chloride, arginine-hydrochloride, glycine or proline in the aforementioned liquid formulation is at a concentration of about 50 mM to about 150 mM, about 100 mM to about 110 mM, or about 100 mM to about 105 mM. In some embodiments, the sodium chloride in the aforementioned liquid formulation is at a concentration of about 102.7 mM. In some embodiments, the sodium chloride in the aforementioned liquid formulation is at a concentration of about 103 mM. In some embodiments, the sodium chloride in the aforementioned liquid formulation is at a concentration of about 6 mg/mL. In some embodiments, the arginine-hydrochloride in the aforementioned liquid formulation is at a concentration of about 100 mM.

In some embodiments, the sodium chloride, arginine-hydrochloride, glycine or proline in the aforementioned liquid formulation is at a concentration of 10 mM to 300 mM. In some embodiments, the sodium chloride, arginine-hydrochloride, glycine or proline in the aforementioned liquid formulation is at a concentration of 20 mM to 200 mM, e.g., 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, 100 mM, 105 mM, 110 mM, 115 mM, 120 mM, 125 mM, 130 mM, 135 mM, 140 mM, 145 mM, 150 mM, 160 mM, 170 mM, 180 mM, 190 mM or 200 mM. In some embodiments, the sodium chloride, arginine-hydrochloride, glycine or proline in the aforementioned liquid formulation is at a concentration of 50 mM to 150 mM, 100 mM to 110 mM, or 100 mM to 105 mM. In some embodiments, the sodium chloride in the aforementioned liquid formulation is at a concentration of about 102.7 mM. In some embodiments, the sodium chloride in the aforementioned liquid formulation is at a concentration of about 103 mM. In some embodiments, the sodium chloride in the aforementioned liquid formulation is at a concentration of 6 mg/mL. In some embodiments, the arginine-hydrochloride in the aforementioned liquid formulation is at a concentration of 100 mM.

According to the claims, the aforementioned liquid formulation further comprises a surfactant, which can be selected from the group consisting of polysorbate 20, polysorbate 80, poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauroamidopropyl-betaine, cocamidopropyl-betaine, linoleamidopropyl-betaine, myristamidopropyl-betaine, palmidopropyl-betaine, isostearamidopropyl-betaine, myristamidopropyl-dimethylamine, palmidopropyl-dimethylamine, isostearamidopropyl-dimethylamine, sodium methyl cocoyl, sodium methyl oleyl taurate, polyethylene glycol, polypropylene glycol, a copolymer of ethylene and propylene glycol, and the like. According to the claims, the surfactant is polysorbate 80 or polysorbate 20. In some embodiments, the aforementioned liquid formulation comprises polysorbate 80 (also known as tween 80).

According to the claims, the polysorbate 80 in the aforementioned liquid formulation is at a concentration of about 0.001% to about 0.1% (w/v). In some embodiments, the polysorbate 80 in the aforementioned liquid formulation is at a concentration of about 0.005% to about 0.08% (w/v), e.g., about 0.005% (w/v), about 0.006% (w/v), about 0.007% (w/v), about 0.008% (w/v), about 0.009% (w/v), about 0.01% (w/v), about 0.015% (w/v), about 0.02% (w/v), about 0.025% (w/v), about 0.03% (w/v), about 0.035% (w/v), about 0.04% (w/v), about 0.045% (w/v), about 0.05% (w/v), about 0.06% (w/v), about 0.07% (w/v) or about 0.08% (w/v). In some embodiments, the polysorbate 80 in the aforementioned liquid formulation is at a concentration of about 0.01% to about 0.04% (w/v). In some embodiments, the polysorbate 80 in the aforementioned liquid formulation is at a concentration of about 0.02% (w/v).

According to the claims, the polysorbate 80 in the aforementioned liquid formulation is at a concentration of 0.001% to 0.1% (w/v). In some embodiments, the polysorbate 80 in the aforementioned liquid formulation is at a concentration of 0.005% to 0.08% (w/v), e.g., 0.005% (w/v), 0.006% (w/v), 0.007% (w/v), 0.008% (w/v), 0.009% (w/v), 0.01% (w/v), 0.015% (w/v), 0.02% (w/v), 0.025% (w/v), 0.03% (w/v), 0.035% (w/v), 0.04% (w/v), 0.045% (w/v), 0.05% (w/v), 0.06% (w/v), 0.07% (w/v) or 0.08% (w/v). In some embodiments, the polysorbate 80 in the aforementioned liquid formulation is at a concentration of 0.01% to 0.04% (w/v). In some embodiments, the polysorbate 80 in the aforementioned liquid formulation is at a concentration of 0.02% (w/v).

In one aspect, the present disclosure provides a liquid formulation comprising a bispecific anti-CD3/CD20 polypeptide complex and one or more of a buffer, an osmotic pressure regulator and/or a stabilizer, and a surfactant. In some embodiments, the liquid formulation of the present disclosure comprises a bispecific anti-CD3/CD20 polypeptide complex and a buffer, an osmotic pressure regulator and/or stabilizer, and a surfactant.

In some embodiments, the aforementioned liquid formulation comprises:
(a) about 0.1 mg/mL to about 50 mg/mL bispecific anti-CD3/CD20 polypeptide complex,
(b) about 1 mM to about 30 mM citrate buffer,
(c) about 150 mM to about 320 mM saccharide; optionally, one or more of the following can be comprised: about 10 mM to about 300 mM sodium chloride, about 10 mM to about 300 mM arginine-hydrochloride, about 10 mM to about 300 mM glycine and about 10 mM to about 300 mM proline; and
(d) about 0.001% to about 0.1% (w/v) surfactant,
wherein the liquid formulation has a pH of about 5.0 to about 7.5, preferably about 6.0 to about 6.5.

In some embodiments, the aforementioned liquid formulation comprises:
(a) about 0.1 mg/mL to about 50 mg/mL bispecific anti-CD3/CD20 polypeptide complex,
(b) about 1 mM to about 30 mM citrate buffer,
(c) about 150 mM to about 320 mM trehalose; optionally, one or more of the following can be comprised: about 10 mM to about 300 mM sodium chloride, about 10 mM to about 300 mM arginine-hydrochloride, about 10 mM to about 300 mM glycine and about 10 mM to about 300 mM proline; and
(d) about 0.001% to about 0.1% (w/v) polysorbate 80,
wherein the liquid formulation has a pH of about 5.0 to about 7.5, preferably about 6.0 to about 6.5.

In some embodiments, the aforementioned liquid formulation comprises:
(a) about 0.5 mg/mL to about 20 mg/mL bispecific anti-CD3/CD20 polypeptide complex,
(b) about 5 mM to about 20 mM citrate buffer,
(c) about 180 mM to about 265 mM trehalose; optionally, one or more of the following can be comprised: about 20 mM to about 200 mM sodium chloride, about 20 mM to about 200 mM arginine-hydrochloride, about 20 mM to about 200 mM glycine and about 20 mM to about 200 mM proline; and
(d) about 0.005% to about 0.08% (w/v) polysorbate 80,
wherein the liquid formulation has a pH of about 6.0 to about 6.5.

In some embodiments, the aforementioned liquid formulation comprises:
(a) about 0.5 mg/mL to about 20 mg/mL bispecific anti-CD3/CD20 polypeptide complex (e.g., about 0.5 mg/mL, about 1 mg/mL, about 1.5 mg/mL, about 2 mg/mL, about 2.5 mg/mL, about 3 mg/mL, about 3.5 mg/mL, about 4 mg/mL, about 4.5 mg/mL, about 5 mg/mL, about 5.5 mg/mL, about 6 mg/mL, about 6.5 mg/mL, about 7 mg/mL, about 7.5 mg/mL, about 8 mg/mL, about 8.5 mg/mL, about 9 mg/mL, about 9.5 mg/mL, about 10 mg/mL, about 11 mg/mL, about 12 mg/mL, about 13 mg/mL, about 14 mg/mL, about 15 mg/mL, about 16 mg/mL, about 17 mg/mL, about 18 mg/mL, about 19 mg/mL or about 20 mg/mL),
(b) about 5 mM to about 20 mM citric acid-sodium citrate buffer (e.g., about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM or about 20 mM),
(c) about 180 mM to about 265 mM trehalose (e.g., about 180 mM, about 185 mM, about 198 mM, about 211 mM, about 214 mM, about 217 mM, about 219 mM, about 222 mM, about 225 mM, about 227 mM, about 230 mM, about 233 mM, about 235 mM, about 238 mM, about 241 mM, about 243 mM, about 246 mM, about 248 mM, about 251 mM, 254 mM, about 256 mM, about 259 mM, about 262 mM or about 264 mM; or about 180 mM, about 185.02 mM, about 198.24 mM, about 211.46 mM, about 214.1 mM, about 216.74 mM, about 219.39 mM, about 222.03 mM, about 224.67 mM, about 227.31 mM, about 229.96 mM, about 232.6 mM, about 235.24 mM, about 237.89 mM, about 240.53 mM, about 243.17 mM, about 245.82 mM, about 248.46 mM, about 251.1 mM, 253.75 mM, about 256.39 mM, about 259.03 mM, about 261.68 mM or about 264.32 mM); optionally, one or more of the following can be comprised: about 20 mM to about 200 mM sodium chloride, about 20 mM to about 200 mM arginine-hydrochloride, about 20 mM to about 200 mM glycine and about 20 mM to about 200 mM proline; and
(d) about 0.005% to about 0.08% (w/v) polysorbate 80 (e.g., about 0.005% (w/v), about 0.006% (w/v), about 0.007% (w/v), about 0.008% (w/v), about 0.009% (w/v), about 0.01% (w/v), about 0.015% (w/v), about 0.02% (w/v), about 0.025% (w/v), about 0.03% (w/v), about 0.035% (w/v), about 0.04% (w/v), about 0.045% (w/v), about 0.05% (w/v), about 0.06% (w/v), about 0.07% (w/v) or about 0.08% (w/v)),
wherein the liquid formulation has a pH of about 6.0 to about 6.5 (e.g., about 6.0, about 6.1, about 6.2, about 6.3, about 6.4 or about 6.5).

In some embodiments, the aforementioned liquid formulation comprises:
(a) about 0.1 mg/mL to about 50 mg/mL bispecific anti-CD3/CD20 polypeptide complex,
(b) about 1 mM to about 30 mM buffer (e.g., one or more of histidine-histidine hydrochloride buffer, succinic acid-sodium succinate buffer and citric acid-sodium citrate buffer),
(c) about 150 mM to about 320 mM saccharide, and one of the following can be comprised: about 10 mM to about 300 mM sodium chloride, about 10 mM to about 300 mM arginine-hydrochloride, about 10 mM to about 300 mM glycine and about 10 mM to about 300 mM proline, and
(d) about 0.001% to about 0.1% (w/v) surfactant,
wherein the liquid formulation has a pH of about 5.0 to about 7.5, preferably about 6.0 to about 6.5.

In some embodiments, the aforementioned liquid formulation comprises:
(a) about 0.5 mg/mL to about 20 mg/mL bispecific anti-CD3/CD20 polypeptide complex (e.g., about 0.5 mg/mL, about 1 mg/mL, about 1.5 mg/mL, about 2 mg/mL, about 2.5 mg/mL, about 3 mg/mL, about 3.5 mg/mL, about 4 mg/mL, about 4.5 mg/mL, about 5 mg/mL, about 5.5 mg/mL, about 6 mg/mL, about 6.5 mg/mL, about 7 mg/mL, about 7.5 mg/mL, about 8 mg/mL, about 8.5 mg/mL, about 9 mg/mL, about 9.5 mg/mL, about 10 mg/mL, about 11 mg/mL, about 12 mg/mL, about 13 mg/mL, about 14 mg/mL, about 15 mg/mL, about 16 mg/mL, about 17 mg/mL, about 18 mg/mL, about 19 mg/mL or about 20 mg/mL),
(b) about 5 mM to about 20 mM histidine-histidine hydrochloride or citric acid-sodium citrate buffer (e.g., about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM or about 20 mM),
(c) about 180 mM to about 265 mM trehalose or sucrose (e.g., about 180 mM, about 185 mM, about 198 mM, about 211 mM, about 214 mM, about 217 mM, about 219 mM, about 222 mM, about 225 mM, about 227 mM, about 230 mM, about 233 mM, about 235 mM, about 238 mM, about 241 mM, about 243 mM, about 246 mM, about 248 mM, about 251 mM, 254 mM, about 256 mM, about 259 mM, about 262 mM or about 264 mM; or about 180 mM, about 185.02 mM, about 198.24 mM, about 211.46 mM, about 214.1 mM, about 216.74 mM, about 219.39 mM, about 222.03 mM, about 224.67 mM, about 227.31 mM, about 229.96 mM, about 232.6 mM, about 235.24 mM, about 237.89 mM, about 240.53 mM, about 243.17 mM, about 245.82 mM, about 248.46 mM, about 251.1 mM, 253.75 mM, about 256.39 mM, about 259.03 mM, about 261.68 mM or about 264.32 mM), and about 20 mM to about 200 mM sodium chloride and/or about 20 mM to about 200 mM arginine-hydrochloride (e.g., about 20 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM, about 100 mM, about 105 mM, about 110 mM, about 115 mM, about 120 mM, about 125 mM, about 130 mM, about 135 mM, about 140 mM, about 145 mM, about 150 mM, about 160 mM, about 170 mM, about 180 mM, about 190 mM or about 200 mM), and
(d) about 0.005% to about 0.08% (w/v) polysorbate 80 or polysorbate 20 (e.g., about 0.005% (w/v), about 0.006% (w/v), about 0.007% (w/v), about 0.008% (w/v), about 0.009% (w/v), about 0.01% (w/v), about 0.015% (w/v), about 0.02% (w/v), about 0.025% (w/v), about 0.03% (w/v), about 0.035% (w/v), about 0.04% (w/v), about 0.045% (w/v), about 0.05% (w/v), about 0.06% (w/v), about 0.07% (w/v) or about 0.08% (w/v)),
wherein the liquid formulation has a pH of about 6.0 to about 6.5 (e.g., about 6.0, about 6.1, about 6.2, about 6.3, about 6.4 or about 6.5).

In some embodiments, the aforementioned liquid formulation comprises:
(a) about 0.1 mg/mL to about 50 mg/mL bispecific anti-CD3/CD20 polypeptide complex,
(b) about 1 mM to about 30 mM histidine-histidine hydrochloride or citric acid-sodium citrate buffer,
(c) about 150 mM to about 320 mM trehalose or sucrose, and about 20 mM to about 200 mM sodium chloride and/or about 20 mM to about 200 mM arginine-hydrochloride, and
(d) about 0.001% to about 0.1% (w/v) polysorbate 80 or polysorbate 20,
wherein the liquid formulation has a pH of about 5.0 to about 7.5, preferably about 6.0 to about 6.5.

In some embodiments, the aforementioned liquid formulation comprises:
(a) about 0.5 mg/mL to about 20 mg/mL bispecific anti-CD3/CD20 polypeptide complex,
(b) about 5 mM to about 20 mM histidine-histidine hydrochloride or citric acid-sodium citrate buffer,
(c) about 180 mM to about 265 mM trehalose or sucrose, and about 20 mM to about 200 mM sodium chloride and/or about 20 mM to about 200 mM arginine-hydrochloride, and
(d) about 0.005% to about 0.08% (w/v) polysorbate 80 or polysorbate 20,
wherein the liquid formulation has a pH of about 6.0 to about 6.5.

In some embodiments, the aforementioned liquid formulation comprises:
(a) about 0.1 mg/mL to about 50 mg/mL bispecific anti-CD3/CD20 polypeptide complex,
(b) about 1 mM to about 30 mM citric acid-sodium citrate buffer,
(c) about 150 mM to about 320 mM trehalose, and about 20 mM to about 200 mM sodium chloride and/or about 20 mM to about 200 mM arginine-hydrochloride, and
(d) about 0.001% to about 0.1% (w/v) polysorbate 80 or polysorbate 20,
wherein the liquid formulation has a pH of about 5.0 to about 7.5, preferably about 6.0 to about 6.5.

In some embodiments, the aforementioned liquid formulation comprises:
(a) about 0.5 mg/mL to about 20 mg/mL bispecific anti-CD3/CD20 polypeptide complex,
(b) about 5 mM to about 20 mM citric acid-sodium citrate buffer,
(c) about 180 mM to about 265 mM trehalose, and about 20 mM to about 200 mM sodium chloride and/or about 20 mM to about 200 mM arginine-hydrochloride, and
(d) about 0.005% to about 0.08% (w/v) polysorbate 80 or polysorbate 20,
wherein the liquid formulation has a pH of about 6.0 to about 6.5.

In some embodiments, the aforementioned liquid formulation comprises:
(a) about 0.1 mg/mL to about 50 mg/mL bispecific anti-CD3/CD20 polypeptide complex,
(b) about 1 mM to about 30 mM citrate buffer,
(c) about 150 mM to about 320 mM trehalose, and about 10 mM to about 300 mM sodium chloride, and
(d) about 0.001% to about 0.1% (w/v) polysorbate 80 or polysorbate 20,
wherein the liquid formulation has a pH of about 5.0 to about 7.5, preferably about 6.0 to about 6.5.

In some embodiments, the aforementioned liquid formulation comprises:
(a) about 0.5 mg/mL to about 20 mg/mL bispecific anti-CD3/CD20 polypeptide complex,
(b) about 5 mM to about 20 mM citrate buffer,
(c) about 180 mM to about 265 mM trehalose, and about 20 mM to about 200 mM sodium chloride, and
(d) about 0.005% to about 0.08% (w/v) polysorbate 80 or polysorbate 20,
wherein the liquid formulation has a pH of about 6.0 to about 6.5.

In some embodiments, the aforementioned liquid formulation comprises:
(a) about 1 mg/mL to about 20 mg/mL bispecific anti-CD3/CD20 polypeptide complex,
(b) about 5 mM to about 20 mM citrate buffer,
(c) about 180 mM to about 265 mM trehalose, and about 20 mM to about 200 mM sodium chloride, and
(d) about 0.01% to about 0.04% (w/v) polysorbate 80 or polysorbate 20,
wherein the liquid formulation has a pH of about 6.0 to about 6.5.

In some embodiments, the aforementioned liquid formulation comprises:
(a) about 2 mg/mL to about 3 mg/mL bispecific anti-CD3/CD20 polypeptide complex,
(b) about 10 mM citric acid-sodium citrate buffer,
(c) about 233 mM trehalose, and
(d) about 0.02% (w/v) polysorbate 80,
wherein the liquid formulation has a pH of about 6.2.

In some embodiments, the aforementioned liquid formulation comprises:
(a) about 2 mg/mL to about 3 mg/mL bispecific anti-CD3/CD20 polypeptide complex,
(b) about 10 mM citric acid-sodium citrate buffer,
(c) about 232.6 mM trehalose, and
(d) about 0.02% (w/v) polysorbate 80,
wherein the liquid formulation has a pH of about 6.2.

In some embodiments, the aforementioned liquid formulation comprises:
(a) about 5 mg/mL bispecific anti-CD3/CD20 polypeptide complex,
(b) about 10 mM citrate buffer,
(c) about 233 mM trehalose, and about 103 mM sodium chloride, and
(d) about 0.02% (w/v) polysorbate 80,
wherein the liquid formulation has a pH of about 6.

In some embodiments, the aforementioned liquid formulation comprises:
(a) about 5 mg/mL bispecific anti-CD3/CD20 polypeptide complex,
(b) about 10 mM citrate buffer,
(c) about 232.6 mM trehalose, and about 102.7 mM sodium chloride, and
(d) about 0.02% (w/v) polysorbate 80,
wherein the liquid formulation has a pH of about 6.

In some embodiments, the aforementioned liquid formulation comprises:
(a) about 5 mg/mL bispecific anti-CD3/CD20 polypeptide complex,
(b) about 10 mM citrate buffer,
(c) about 88 mg/mL trehalose dihydrate, and about 6 mg/mL sodium chloride, and
(d) about 0.02% (w/v) polysorbate 80,
wherein the liquid formulation has a pH of about 6.

In some embodiments, the aforementioned liquid formulation comprises:
(a) about 5 mg/mL bispecific anti-CD3/CD20 polypeptide complex,
(b) about 10 mM citric acid-sodium citrate buffer,
(c) about 88 mg/mL trehalose dihydrate, and about 6 mg/mL sodium chloride, and
(d) about 0.02% (w/v) polysorbate 80,
wherein the liquid formulation has a pH of about 6.

The present disclosure (not claimed) also provides a method for preparing the aforementioned liquid formulation, which comprises a step of contacting the bispecific anti-CD3/CD20 polypeptide complex described above with a buffer, e.g., exchanging the bispecific anti-CD3/CD20 polypeptide complex into a buffer, wherein the buffer is preferably a citrate buffer, more preferably a citrate acid-sodium citrate buffer, and has a concentration of about 5 mM to about 20 mM, e.g. about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM or about 20 mM. In some embodiments, the liquid has a concentration of about 10 mM. The buffer has a pH of about 5.0 to about 6.5, e.g., about 5.0, about 5.1, about 5.2, about 5.3, about 5.4, about 5.5, about 5.6, about 5.7, about 5.8, about 5.9, about 6.0, about 6.1, about 6.2, about 6.3, about 6.4 or about 6.5. In some embodiments, the buffer has a pH of about 6. In some embodiments, the buffer has a pH of about 6.2. In some embodiments, the buffer has a pH of about 6.5.

The present disclosure (not claimed) also provides a method for preparing the aforementioned liquid formulation, which comprises contacting the bispecific anti-CD3/CD20 polypeptide complex described above with a buffer, and further comprises adding to the resulting solution an osmotic pressure regulator and/or a stabilizer and a surfactant, such as trehalose and polysorbate 80; optionally, one or more of the following can also be added in any order: sodium chloride, arginine-hydrochloride, glycine and proline; wherein the buffer has a preferred concentration of about 5 nM to about 20 mM, e.g., about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM or about 20 mM. In some embodiments, the buffer has a concentration of about 10 mM. The buffer has a pH of about 5.0 to about 6.5, e.g., about 5.0, about 5.1, about 5.2, about 5.3, about 5.4, about 5.5, about 5.6, about 5.7, about 5.8, about 5.9, about 6.0, about 6.1, about 6.2, about 6.3, about 6.4 or about 6.5. In some embodiments, the buffer has a pH of about 6. In some embodiments, the buffer has a pH of about 6.2. In some embodiments, the buffer has a pH of about 6.5.

The present disclosure (not claimed) also provides a method for preparing a lyophilizate comprising a bispecific anti-CD3/CD20 polypeptide complex, which comprises a step of lyophilizing the aforementioned liquid formulation. In some embodiments, the lyophilization is carried out using a method well known in the art, and comprises, but is not limited to, steps of pre-freezing, primary drying and secondary drying. Those skilled understand that any method of removing water from the liquid formulation described in the present disclosure is suitable for use in the present disclosure.

The present disclosure also provides a lyophilizate comprising a bispecific anti-CD3/CD20 polypeptide complex, which is prepared using the aforementioned method for preparing a lyophilizate.

The present disclosure also provides a lyophilizate comprising a bispecific anti-CD3/CD20 polypeptide complex, which can form the aforementioned liquid formulation upon reconstitution.

In some embodiments, the aforementioned lyophilizate comprises:
(a) about 2 mg of bispecific anti-CD3/CD20 polypeptide complex,
(b) about 0.0013 mmol or about 0.0014 mmol of citric acid,
(c) about 0.0086 mmol of sodium citrate,
(d) about 0.233 mmol of trehalose, and
(e) about 0.2 mg of polysorbate 80;

or comprises multiples of (a), (b), (c), (d) and (e), e.g., an integer multiple or a fraction multiple of the amount of each of (a), (b), (c), (d) and (e) components, or 1.5 times, 2 times, 3 times or 4 times of the amount of each of (a), (b), (c), (d) and (e) components, or an integer multiple of more than 5 of the amount of each of (a), (b), (c), (d) and (e) components,
wherein the lyophilizate has a pH of about 6.2.

In some embodiments, the aforementioned lyophilizate comprises:
(a) about 2 mg of bispecific anti-CD3/CD20 polypeptide complex,
(b) about 0.0013 mmol or about 0.0014 mmol of citric acid,
(c) about 0.0086 mmol of sodium citrate,
(d) about 0.2326 mmol of trehalose, and
(e) about 0.2 mg of polysorbate 80;

or comprises multiples of (a), (b), (c), (d) and (e), e.g., an integer multiple or a fraction multiple of the amount of each of (a), (b), (c), (d) and (e) components, or 1.5 times, 2 times, 3 times or 4 times of the amount of each of (a), (b), (c), (d) and (e) components, or an integer multiple of more than 5 of the amount of each of (a), (b), (c), (d) and (e) components,
wherein the lyophilizate has a pH of about 6.2.

In some embodiments, the aforementioned lyophilizate comprises:
(a) about 2 mg of bispecific anti-CD3/CD20 polypeptide complex,
(b) about 0.001 mmol of citric acid,
(c) about 0.009 mmol of sodium citrate,
(d) about 0.23 mmol of trehalose, and
(e) about 0.2 mg of polysorbate 80;

or comprises multiples of (a), (b), (c), (d) and (e), e.g., an integer multiple or a fraction multiple of the amount of each of (a), (b), (c), (d) and (e) components, or 1.5 times, 2 times, 3 times or 4 times of the amount of each of (a), (b), (c), (d) and (e) components, or an integer multiple of more than 5 of the amount of each of (a), (b), (c), (d) and (e) components,
wherein the lyophilizate has a pH of about 6.2.

In some embodiments, the aforementioned lyophilizate comprises:
(a) about 5 mg of bispecific anti-CD3/CD20 polypeptide complex,
(b) about 0.0013 mmol or about 0.0014 mmol of citric acid,
(c) about 0.0086 mmol of sodium citrate,
(d) about 0.233 mmol of trehalose, and 6 mg of sodium chloride, and
(e) about 0.2 mg of polysorbate 80;

or comprises multiples of (a), (b), (c), (d) and (e), e.g., an integer multiple or a fraction multiple of the amount of each of (a), (b), (c), (d) and (e) components, or 1.5 times, 2 times, 3 times or 4 times of the amount of each of (a), (b), (c), (d) and (e) components, or an integer multiple of more than 5 of the amount of each of (a), (b), (c), (d) and (e) components,
wherein the lyophilizate has a pH of about 6.

In some embodiments, the aforementioned lyophilizate comprises:
(a) about 5 mg of bispecific anti-CD3/CD20 polypeptide complex,
(b) about 0.0013 mmol or about 0.0014 mmol of citric acid,
(c) about 0.0086 mmol of sodium citrate,
(d) about 0.2326 mmol of trehalose, and 6 mg of sodium chloride, and
(e) about 0.2 mg of polysorbate 80;

or comprises multiples of (a), (b), (c), (d) and (e), e.g., an integer multiple or a fraction multiple of the amount of each of (a), (b), (c), (d) and (e) components, or 1.5 times, 2 times, 3 times or 4 times of the amount of each of (a), (b), (c), (d) and (e) components, or an integer multiple of more than 5 of the amount of each of (a), (b), (c), (d) and (e) components,
wherein the lyophilizate has a pH of about 6.

In some embodiments, the aforementioned lyophilizate comprises:
(a) about 5 mg of bispecific anti-CD3/CD20 polypeptide complex,
(b) about 0.001 mmol of citric acid,
(c) about 0.009 mmol of sodium citrate,
(d) about 0.23 mmol of trehalose, and 6 mg of sodium chloride, and
(e) about 0.2 mg of polysorbate 80;

or comprises multiples of (a), (b), (c), (d) and (e), e.g., an integer multiple or a fraction multiple of the amount of each of (a), (b), (c), (d) and (e) components, or 1.5 times, 2 times, 3 times or 4 times of the amount of each of (a), (b), (c), (d) and (e) components, or an integer multiple of more than 5 of the amount of each of (a), (b), (c), (d) and (e) components,
wherein the lyophilizate has a pH of about 6.

In some embodiments, the lyophilizate is stable at 2-8 °C for at least 1 month, at least 6 months, at least 12 months, at least 18 months or at least 24 months. In some embodiments, the lyophilizate is stable at 25 °C for at least 1 month or at least 6 months. In some embodiments, the lyophilizate is stable at 40 °C for at least 7 days, at least 14 days or at least 28 days. The present disclosure also provides a pharmaceutical composition containing a bispecific anti-CD3/CD20 polypeptide complex, which is a solution obtained by reconstituting the aforementioned lyophilizate.

The present disclosure (not claimed) also provides a method for preparing the aforementioned pharmaceutical composition, which comprises a step of reconstituting the aforementioned lyophilizate, wherein a solution for the reconstitution includes, but is not limited to, water for injection, normal saline or glucose solution; water for injection is preferred.

The present disclosure (not claimed) also provides an article of manufacture comprising a container containing the liquid formulation or the lyophilizate thereof or the pharmaceutical composition obtained by reconstituting the lyophilizate disclosed herein.

In some embodiments, the aforementioned liquid formulation is stable at 2-8 °C for at least 1 month, at least 6 months, at least 12 months, at least 18 months or at least 24 months. In some embodiments, the aforementioned liquid formulation is stable at 40 °C for at least 1 week, at least 2 weeks, at least 3 weeks, at least 4 weeks or at least 6 weeks.

The liquid formulation or the lyophilizate thereof or the pharmaceutical composition obtained by reconstituting the lyophilizate disclosed herein can be administered intravenously (e.g., intravenous drip), intramuscularly, subcutaneously, parenterally, spinally or epicutaneously (e.g., by injection or bolus injection). The liquid formulation or the lyophilizate thereof or the pharmaceutical composition obtained by reconstituting the lyophilizate disclosed herein can be diluted with a suitable diluent to a suitable concentration before administration to provide the best desired response (e.g., therapeutic response).

The present disclosure (not claimed) also provides use of the liquid formulation or the lyophilizate or the pharmaceutical composition obtained by reconstituting the lyophilizate or the article of manufacture disclosed herein for manufacturing a medicament for treating a CD20-related disease or disorder.

In some embodiments, the CD20-related disease or disorder is cancer; preferably the cancer is selected from the group consisting of, but is not limited to, lymphoma, lung cancer, liver cancer, cervical cancer, colon cancer, breast cancer, ovarian cancer, pancreatic cancer, melanoma, glioblastoma, prostate cancer, esophageal cancer and gastric cancer.

In some embodiments, the CD20-related disease or disorder is B cell lymphoma, optionally Hodgkin lymphoma or non-Hodgkin lymphoma, wherein the non-Hodgkin lymphoma includes: diffuse large B-cell lymphoma (DLBCL), follicular lymphoma, marginal zone B-cell lymphoma (MZL), mucosa-associated lymphoid tissue lymphoma (MALT), small lymphocytic lymphoma (chronic lymphocytic leukemia, CLL), or mantle cell lymphoma (MCL), acute lymphocytic leukemia (ALL), or Waldenström macroglobulinemia (WM).

In another aspect, the present disclosure provides a pharmaceutical composition comprising a bispecific anti-CD3/CD20 polypeptide complex and further comprising one or more of a buffering agent, an osmotic pressure regulator and/or a stabilizer, and a surfactant. In some embodiments, the pharmaceutical composition of the present disclosure is a lyophilized formulation comprising a bispecific anti-CD3/CD20 polypeptide complex, and a buffering agent, an osmotic pressure regulator and/or a stabilizer, and a surfactant.

In another aspect, the present disclosure provides a stable lyophilized formulation comprising a bispecific anti-CD3/CD20 polypeptide complex and a buffering agent, wherein the buffering agent includes one of or a combination of more of a histidine salt buffering agent, a succinate buffering agent and a citrate buffering agent. In some embodiments, the histidine salt buffering agent is histidine-histidine hydrochloride, the succinate buffering agent is succinic acid-sodium succinate, and the citrate buffering agent is citric acid-sodium citrate.

In some embodiments, the buffering agent in the aforementioned lyophilized formulation is one of or a combination of more of histidine-histidine hydrochloride, succinic acid-sodium succinate and citric acid-sodium citrate buffering agents. In some embodiments, the buffering agent in the aforementioned lyophilized formulation is a citric acid-sodium citrate buffer.

According to the claims, the bispecific anti-CD3/C20 polypeptide complex in the aforementioned lyophilized formulation is at a concentration of about 0.1 mg/mL to about 50 mg/mL. In some embodiments, the bispecific anti-CD3/C20 polypeptide complex in the aforementioned lyophilized formulation is at a concentration of about 0.5 mg/mL to about 20 mg/mL, e.g., about 0.5 mg/mL, about 1 mg/mL, about 1.5 mg/mL, about 2 mg/mL, about 2.5 mg/mL, about 3 mg/mL, about 3.5 mg/mL, about 4 mg/mL, about 4.5 mg/mL, about 5 mg/mL, about 5.5 mg/mL, about 6 mg/mL, about 6.5 mg/mL, about 7 mg/mL, about 7.5 mg/mL, about 8 mg/mL, about 8.5 mg/mL, about 9 mg/mL, about 9.5 mg/mL, about 10 mg/mL, about 11 mg/mL, about 12 mg/mL, about 13 mg/mL, about 14 mg/mL, about 15 mg/mL, about 16 mg/mL, about 17 mg/mL, about 18 mg/mL, about 19 mg/mL or about 20 mg/mL. In some embodiments, the bispecific anti-CD3/C20 polypeptide complex in the aforementioned lyophilized formulation is at a concentration of about 1 mg/mL to about 20 mg/mL, about 2 mg/mL to about 10 mg/mL, about 2 mg/mL to about 5 mg/mL, or about 2 mg/mL to about 3 mg/mL. In some embodiments, the bispecific anti-CD3/C20 polypeptide complex in the aforementioned lyophilized formulation is at a concentration of about 2 mg/mL. In some embodiments, the bispecific anti-CD3/C20 polypeptide complex in the aforementioned lyophilized formulation is at a concentration of about 3 mg/mL. In some embodiments, the bispecific anti-CD3/C20 polypeptide complex in the aforementioned lyophilized formulation is at a concentration of about 5 mg/mL. In some embodiments, the bispecific anti-CD3/C20 polypeptide complex in the aforementioned lyophilized formulation is at a concentration of about 10 mg/mL.

According to the claims, the bispecific anti-CD3/C20 polypeptide complex in the aforementioned lyophilized formulation is at a concentration of 0.1 mg/mL to 50 mg/mL. In some embodiments, the bispecific anti-CD3/C20 polypeptide complex in the aforementioned lyophilized formulation is at a concentration of 0.5 mg/mL to 20 mg/mL, e.g., 0.5 mg/mL, 1 mg/mL, 1.5 mg/mL, 2 mg/mL, 2.5 mg/mL, 3 mg/mL, 3.5 mg/mL, 4 mg/mL, 4.5 mg/mL, 5 mg/mL, 5.5 mg/mL, 6 mg/mL, 6.5 mg/mL, 7 mg/mL, 7.5 mg/mL, 8 mg/mL, 8.5 mg/mL, 9 mg/mL, 9.5 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL or 20 mg/mL. In some embodiments, the bispecific anti-CD3/C20 polypeptide complex in the aforementioned lyophilized formulation is at a concentration of 1 mg/mL to 20 mg/mL, 2 mg/mL to 10 mg/mL, 2 mg/mL to 5 mg/mL, or 2 mg/mL to 3 mg/mL. In some embodiments, the bispecific anti-CD3/C20 polypeptide complex in the aforementioned lyophilized formulation is at a concentration of 2 mg/mL. In some embodiments, the bispecific anti-CD3/C20 polypeptide complex in the aforementioned lyophilized formulation is at a concentration of 3 mg/mL. In some embodiments, the bispecific anti-CD3/C20 polypeptide complex in the aforementioned lyophilized formulation is at a concentration of 5 mg/mL. In some embodiments, the bispecific anti-CD3/C20 polypeptide complex in the aforementioned lyophilized formulation is at a concentration of 10 mg/mL.

In some embodiments, the buffering agent in the aforementioned lyophilized formulation maintains the pH of the lyophilized formulation at about 5.0 to about 7.5. In some embodiments, the buffering agent in the aforementioned lyophilized formulation maintains the pH of the lyophilized formulation at about 5.0 to about 6.5, e.g., about 5.0, about 5.1, about 5.2, about 5.3, about 5.4, about 5.5, about 5.6, about 5.7, about 5.8, about 5.9, about 6.0, about 6.1, about 6.2, about 6.3, about 6.4 or about 6.5. In some embodiments, the buffering agent in the aforementioned lyophilized formulation maintains the pH of the lyophilized formulation at about 6.0 to about 6.5. In some embodiments, the buffering agent in the aforementioned lyophilized formulation maintains the pH of the lyophilized formulation at about 6. In some embodiments, the buffering agent in the aforementioned lyophilized formulation maintains the pH of the lyophilized formulation at about 6.2. In some embodiments, the buffering agent in the aforementioned lyophilized formulation maintains the pH of the lyophilized formulation at about 6.5. In some embodiments, the buffering agent in the aforementioned lyophilized formulation maintains the pH of the lyophilized formulation at 5.0 to 7.5. In some embodiments, the buffering agent in the aforementioned lyophilized formulation maintains the pH of the lyophilized formulation at 5.0 to 6.5, e.g., 5.0, 5.1, 5.2, 5.3, 5.4, 5.5, 5.6, 5.7, 5.8, 5.9, 6.0, 6.1, 6.2, 6.3, 6.4 or 6.5. In some embodiments, the buffering agent in the aforementioned lyophilized formulation maintains the pH of the lyophilized formulation at 6.0 to 6.5. In some embodiments, the buffering agent in the aforementioned lyophilized formulation maintains the pH of the lyophilized formulation at 6. In some embodiments, the buffering agent in the aforementioned lyophilized formulation maintains the pH of the lyophilized formulation at 6.2. In some embodiments, the buffering agent in the aforementioned lyophilized formulation maintains the pH of the lyophilized formulation at 6.5.

According to the claims, the buffering agent in the aforementioned lyophilized formulation is at a concentration of about 1 mM to about 30 mM. In some embodiments, the buffering agent in the aforementioned lyophilized formulation is at a concentration of about 5 mM to about 20 mM, e.g., about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM or about 20 mM. In some embodiments, the buffering agent in the aforementioned lyophilized formulation is at a concentration of about 10 mM.

According to the claims, the buffering agent in the aforementioned lyophilized formulation is at a concentration of 1 mM to 30 mM. In some embodiments, the buffering agent in the aforementioned lyophilized formulation is at a concentration of 5 mM to 20 mM, e.g., 5 mM, 6 mM, 7 mM, 8 mM, 9 mM, 10 mM, 11 mM, 12 mM, 13 mM, 14 mM, 15 mM, 16 mM, 17 mM, 18 mM, 19 mM or 20 mM. In some embodiments, the buffering agent in the aforementioned lyophilized formulation is at a concentration of 10 mM.

In some embodiments, the aforementioned lyophilized formulation has a pH of about 5.0 to about 7.5. In some embodiments, the aforementioned lyophilized formulation has a pH of about 5.0 to about 6.5, e.g., about 5.0, about 5.1, about 5.2, about 5.3, about 5.4, about 5.5, about 5.6, about 5.7, about 5.8, about 5.9, about 6.0, about 6.1, about 6.2, about 6.3, about 6.4 or about 6.5. In some embodiments, the aforementioned lyophilized formulation has a pH of about 6.0 to about 6.5. In some embodiments, the aforementioned lyophilized formulation has a pH of about 6. In some embodiments, the aforementioned lyophilized formulation has a pH of about 6.2. In some embodiments, the aforementioned lyophilized formulation has a pH of about 6.5.

In some embodiments, the aforementioned lyophilized formulation has a pH of 5.0 to 7.5. In some embodiments, the aforementioned lyophilized formulation has a pH of 5.0 to 6.5, e.g., 5.0, 5.1, 5.2, 5.3, 5.4, 5.5, 5.6, 5.7, 5.8, 5.9, 6.0, 6.1, 6.2, 6.3, 6.4 or 6.5. In some embodiments, the aforementioned lyophilized formulation has a pH of 6.0 to 6.5. In some embodiments, the aforementioned lyophilized formulation has a pH of 6. In some embodiments, the aforementioned lyophilized formulation has a pH of 6.2. In some embodiments, the aforementioned lyophilized formulation has a pH of 6.5.

According to the claims, the aforementioned lyophilized formulation further comprises an osmotic pressure regulator and/or a stabilizer. The osmotic pressure regulator and/or the stabilizer is a saccharide. As used herein, "saccharide" can be a monosaccharide, disaccharide, trisaccharide, polysaccharide, sugar alcohol or reducing sugar and non-reducing sugar, e.g., glucose, sucrose, trehalose, lactose, fructose, dextran, glycerol, erythritol, glycerol, arabitol, xylitol, sorbitol, mannitol, melibiose, melezitose, raffinose, mannotriose, stachyose, maltose, lactulose, maltulose, sorbitol, maltitol, lactitol, isomaltulose, and the like. In some embodiments, the osmotic pressure regulator and/or the stabilizer further comprise one or more of sodium chloride, arginine-hydrochloride, glycine and proline. In some embodiments, the osmotic pressure regulator and/or the stabilizer in the aforementioned lyophilized formulation are disaccharides, such as trehalose or sucrose. In some embodiments, the aforementioned lyophilized formulation comprises trehalose. In some embodiments, the aforementioned lyophilized formulation comprises trehalose and one or more of sodium chloride, arginine-hydrochloride, glycine and proline. In some embodiments, the aforementioned lyophilized formulation comprises sucrose and one or more of sodium chloride, arginine-hydrochloride, glycine and proline. In some embodiments, the aforementioned lyophilized formulation comprises trehalose and sodium chloride. In some embodiments, the aforementioned lyophilized formulation comprises trehalose and arginine-hydrochloride. In some embodiments, the aforementioned lyophilized formulation comprises sucrose and sodium chloride. In some embodiments, the aforementioned lyophilized formulation comprises sucrose and arginine-hydrochloride. In the formulation of the present application, saccharides include saccharides and/or hydrates thereof; for example, trehalose includes trehalose and/or a hydrate thereof such as trehalose dihydrate.

According to the claims, the saccharide in the aforementioned lyophilized formulation is at a concentration of about 150 mM to about 320 mM. In some embodiments, the saccharide in the aforementioned lyophilized formulation is at a concentration of about 180 mM to about 265 mM, e.g., about 180 mM, about 185 mM, about 198 mM, about 211 mM, about 214 mM, about 217 mM, about 219 mM, about 222 mM, about 225 mM, about 227 mM, about 230 mM, about 233 mM, about 235 mM, about 238 mM, about 241 mM, about 243 mM, about 246 mM, about 248 mM, about 251 mM, 254 mM, about 256 mM, about 259 mM, about 262 mM or about 264 mM. In some embodiments, the saccharide in the aforementioned lyophilized formulation is at a concentration of about 230 mM to about 240 mM, or about 230 mM to about 235 mM. In some embodiments, the saccharide in the aforementioned lyophilized formulation is at a concentration of about 233 mM. In some embodiments, the saccharide in the aforementioned lyophilized formulation is at a concentration of about 234 mM. In some embodiments, the saccharide in the aforementioned lyophilized formulation is at a concentration of about 180 mM to about 265 mM, e.g., about 180 mM, about 185.02 mM, about 198.24 mM, about 211.46 mM, about 214.1 mM, about 216.74 mM, about 219.39 mM, about 222.03 mM, about 224.67 mM, about 227.31 mM, about 229.96 mM, about 232.6 mM, about 235.24 mM, about 237.89 mM, about 240.53 mM, about 243.17 mM, about 245.82 mM, about 248.46 mM, about 251.1 mM, 253.75 mM, about 256.39 mM, about 259.03 mM, about 261.68 mM or about 264.32 mM. In some embodiments, the saccharide in the aforementioned lyophilized formulation is at a concentration of about 232.6 mM. In some embodiments, the saccharide in the aforementioned lyophilized formulation is at a concentration of about 233.7 mM.

In some embodiments, the trehalose in the aforementioned lyophilized formulation is at a concentration of about 150 mM to about 320 mM. In some embodiments, the trehalose in the aforementioned lyophilized formulation is at a concentration of about 180 mM to about 265 mM, e.g., about 180 mM, about 185 mM, about 198 mM, about 211 mM, about 214 mM, about 217 mM, about 219 mM, about 222 mM, about 225 mM, about 227 mM, about 230 mM, about 233 mM, about 235 mM, about 238 mM, about 241 mM, about 243 mM, about 246 mM, about 248 mM, about 251 mM, 254 mM, about 256 mM, about 259 mM, about 262 mM or about 264 mM. In some embodiments, the trehalose in the aforementioned lyophilized formulation is at a concentration of about 230 mM to about 240 mM, or about 230 mM to about 235 mM. In some embodiments, the trehalose in the aforementioned lyophilized formulation is at a concentration of about 233 mM. In some embodiments, the trehalose in the aforementioned lyophilized formulation is at a concentration of about 180 mM to about 265 mM, e.g., about 180 mM, about 185.02 mM, about 198.24 mM, about 211.46 mM, about 214.1 mM, about 216.74 mM, about 219.39 mM, about 222.03 mM, about 224.67 mM, about 227.31 mM, about 229.96 mM, about 232.6 mM, about 235.24 mM, about 237.89 mM, about 240.53 mM, about 243.17 mM, about 245.82 mM, about 248.46 mM, about 251.1 mM, 253.75 mM, about 256.39 mM, about 259.03 mM, about 261.68 mM or about 264.32 mM. In some embodiments, the trehalose in the aforementioned lyophilized formulation is at a concentration of about 232.6 mM.

In some embodiments, the sodium chloride, arginine-hydrochloride, glycine or proline in the aforementioned lyophilized formulation is at a concentration of about 10 mM to about 300 mM. In some embodiments, the sodium chloride, arginine-hydrochloride, glycine or proline in the aforementioned lyophilized formulation is at a concentration of about 20 mM to about 200 mM, e.g., about 20 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM, about 100 mM, about 105 mM, about 110 mM, about 115 mM, about 120 mM, about 125 mM, about 130 mM, about 135 mM, about 140 mM, about 145 mM, about 150 mM, about 160 mM, about 170 mM, about 180 mM, about 190 mM or about 200 mM. In some embodiments, the sodium chloride, arginine-hydrochloride, glycine or proline in the aforementioned lyophilized formulation is at a concentration of about 50 mM to about 150 mM, about 100 mM to about 110 mM, or about 100 mM to about 105 mM. In some embodiments, the sodium chloride in the aforementioned lyophilized formulation is at a concentration of about 102.7 mM. In some embodiments, the sodium chloride in the aforementioned lyophilized formulation is at a concentration of about 103 mM. In some embodiments, the sodium chloride in the aforementioned lyophilized formulation is at a concentration of about 6 mg/mL. In some embodiments, the arginine-hydrochloride in the aforementioned lyophilized formulation is at a concentration of about 100 mM.

In some embodiments, the sodium chloride, arginine-hydrochloride, glycine or proline in the aforementioned lyophilized formulation is at a concentration of 10 mM to 300 mM. In some embodiments, the sodium chloride, arginine-hydrochloride, glycine or proline in the aforementioned lyophilized formulation is at a concentration of 20 mM to 200 mM, e.g., 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, 100 mM, 105 mM, 110 mM, 115 mM, 120 mM, 125 mM, 130 mM, 135 mM, 140 mM, 145 mM, 150 mM, 160 mM, 170 mM, 180 mM, 190 mM or 200 mM. In some embodiments, the sodium chloride, arginine-hydrochloride, glycine or proline in the aforementioned lyophilized formulation is at a concentration of 50 mM to 150 mM, 100 mM to 110 mM, or 100 mM to 105 mM. In some embodiments, the sodium chloride in the aforementioned lyophilized formulation is at a concentration of about 102.7 mM. In some embodiments, the sodium chloride in the aforementioned lyophilized formulation is at a concentration of about 103 mM. In some embodiments, the sodium chloride in the aforementioned lyophilized formulation is at a concentration of 6 mg/mL. In some embodiments, the arginine-hydrochloride in the aforementioned lyophilized formulation is at a concentration of 100 mM.

According to the claims, the aforementioned lyophilized formulation further comprises a surfactant, which can be selected from the group consisting of polysorbate 20, polysorbate 80, poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauroamidopropyl-betaine, cocamidopropyl-betaine, linoleamidopropyl-betaine, myristamidopropyl-betaine, palmidopropyl-betaine, isostearamidopropyl-betaine, myristamidopropyl-dimethylamine, palmidopropyl-dimethylamine, isostearamidopropyl-dimethylamine, sodium methyl cocoyl, sodium methyl oleyl taurate, polyethylene glycol, polypropylene glycol, a copolymer of ethylene and propylene glycol, and the like. According to the claims, the surfactant is polysorbate 80 or polysorbate 20. In some embodiments, the aforementioned lyophilized formulation comprises polysorbate 80 (also known as tween 80).

According to the claims, the polysorbate 80 in the aforementioned lyophilized formulation is at a concentration of about 0.001% to about 0.1% (w/v). In some embodiments, the polysorbate 80 in the aforementioned lyophilized formulation is at a concentration of about 0.005% to about 0.08% (w/v), e.g., about 0.005% (w/v), about 0.006% (w/v), about 0.007% (w/v), about 0.008% (w/v), about 0.009% (w/v), about 0.01% (w/v), about 0.015% (w/v), about 0.02% (w/v), about 0.025% (w/v), about 0.03% (w/v), about 0.035% (w/v), about 0.04% (w/v), about 0.045% (w/v), about 0.05% (w/v), about 0.06% (w/v), about 0.07% (w/v) or about 0.08% (w/v). In some embodiments, the polysorbate 80 in the aforementioned lyophilized formulation is at a concentration of about 0.01% to about 0.04% (w/v). In some embodiments, the polysorbate 80 in the aforementioned lyophilized formulation is at a concentration of about 0.02% (w/v).

According to the claims, the polysorbate 80 in the aforementioned lyophilized formulation is at a concentration of 0.001% to 0.1% (w/v). In some embodiments, the polysorbate 80 in the aforementioned lyophilized formulation is at a concentration of 0.005% to 0.08% (w/v), e.g., 0.005% (w/v), 0.006% (w/v), 0.007% (w/v), 0.008% (w/v), 0.009% (w/v), 0.01% (w/v), 0.015% (w/v), 0.02% (w/v), 0.025% (w/v), 0.03% (w/v), 0.035% (w/v), 0.04% (w/v), 0.045% (w/v), 0.05% (w/v), 0.06% (w/v), 0.07% (w/v) or 0.08% (w/v). In some embodiments, the polysorbate 80 in the aforementioned lyophilized formulation is at a concentration of 0.01% to 0.04% (w/v). In some embodiments, the polysorbate 80 in the aforementioned lyophilized formulation is at a concentration of 0.02% (w/v).

In some embodiments, the aforementioned lyophilized formulation comprises:
(a) about 0.1 mg/mL to about 50 mg/mL bispecific anti-CD3/CD20 polypeptide complex,
(b) about 1 mM to about 30 mM citrate buffering agent,
(c) about 150 mM to about 320 mM saccharide; optionally, one or more of the following can be comprised: about 10 mM to about 300 mM sodium chloride, about 10 mM to about 300 mM arginine-hydrochloride, about 10 mM to about 300 mM glycine and about 10 mM to about 300 mM proline; and
(d) about 0.001% to about 0.1% (w/v) surfactant,
wherein the lyophilized formulation has a pH of about 5.0 to about 7.5, preferably about 6.0 to about 6.5.

In some embodiments, the aforementioned lyophilized formulation comprises:
(a) about 0.1 mg/mL to about 50 mg/mL bispecific anti-CD3/CD20 polypeptide complex,
(b) about 1 mM to about 30 mM citrate buffering agent,
(c) about 150 mM to about 320 mM trehalose; optionally, one or more of the following can be comprised: about 10 mM to about 300 mM sodium chloride, 10 mM to about 300 mM arginine-hydrochloride, 10 mM to about 300 mM glycine and 10 mM to about 300 mM proline; and
(d) about 0.001% to about 0.1% (w/v) polysorbate 80,
wherein the lyophilized formulation has a pH of about 5.0 to about 7.5, preferably about 6.0 to about 6.5.

In some embodiments, the aforementioned lyophilized formulation comprises:
(a) about 0.5 mg/mL to about 20 mg/mL bispecific anti-CD3/CD20 polypeptide complex,
(b) about 5 mM to about 20 mM citrate buffering agent,
(c) about 180 mM to about 265 mM trehalose; optionally, one or more of the following can be comprised: about 20 mM to about 200 mM sodium chloride, 20 mM to about 200 mM arginine-hydrochloride, 20 mM to about 200 mM glycine and 20 mM to about 200 mM proline; and
(d) about 0.005% to about 0.08% (w/v) polysorbate 80,
wherein the lyophilized formulation has a pH of about 6.0 to about 6.5.

In some embodiments, the aforementioned lyophilized formulation comprises:
(a) about 0.5 mg/mL to about 20 mg/mL bispecific anti-CD3/CD20 polypeptide complex (e.g., about 0.5 mg/mL, about 1 mg/mL, about 1.5 mg/mL, about 2 mg/mL, about 2.5 mg/mL, about 3 mg/mL, about 3.5 mg/mL, about 4 mg/mL, about 4.5 mg/mL, about 5 mg/mL, about 5.5 mg/mL, about 6 mg/mL, about 6.5 mg/mL, about 7 mg/mL, about 7.5 mg/mL, about 8 mg/mL, about 8.5 mg/mL, about 9 mg/mL, about 9.5 mg/mL, about 10 mg/mL, about 11 mg/mL, about 12 mg/mL, about 13 mg/mL, about 14 mg/mL, about 15 mg/mL, about 16 mg/mL, about 17 mg/mL, about 18 mg/mL, about 19 mg/mL or about 20 mg/mL),
(b) about 5 mM to about 20 mM citric acid-sodium citrate buffering agent (e.g., about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM or about 20 mM),
(c) about 180 mM to about 265 mM trehalose (e.g., about 180 mM, about 185 mM, about 198 mM, about 211 mM, about 214 mM, about 217 mM, about 219 mM, about 222 mM, about 225 mM, about 227 mM, about 230 mM, about 233 mM, about 235 mM, about 238 mM, about 241 mM, about 243 mM, about 246 mM, about 248 mM, about 251 mM, 254 mM, about 256 mM, about 259 mM, about 262 mM or about 264 mM; or about 180 mM, about 185.02 mM, about 198.24 mM, about 211.46 mM, about 214.1 mM, about 216.74 mM, about 219.39 mM, about 222.03 mM, about 224.67 mM, about 227.31 mM, about 229.96 mM, about 232.6 mM, about 235.24 mM, about 237.89 mM, about 240.53 mM, about 243.17 mM, about 245.82 mM, about 248.46 mM, about 251.1 mM, 253.75 mM, about 256.39 mM, about 259.03 mM, about 261.68 mM or about 264.32 mM); optionally, one or more of the following can be comprised: about 20 mM to about 200 mM sodium chloride, 20 mM to about 200 mM arginine-hydrochloride, 20 mM to about 200 mM glycine and 20 mM to about 200 mM proline; and
(d) about 0.005% to about 0.08% (w/v) polysorbate 80 (e.g., about 0.005% (w/v), about 0.006% (w/v), about 0.007% (w/v), about 0.008% (w/v), about 0.009% (w/v), about 0.01% (w/v), about 0.015% (w/v), about 0.02% (w/v), about 0.025% (w/v), about 0.03% (w/v), about 0.035% (w/v), about 0.04% (w/v), about 0.045% (w/v), about 0.05% (w/v), about 0.06% (w/v), about 0.07% (w/v) or about 0.08% (w/v)),
wherein the lyophilized formulation has a pH of about 6.0 to about 6.5 (e.g., about 6.0, about 6.1, about 6.2, about 6.3, about 6.4 or about 6.5).

In some embodiments, the aforementioned lyophilized formulation comprises:
(a) about 0.1 mg/mL to about 50 mg/mL bispecific anti-CD3/CD20 polypeptide complex,
(b) about 1 mM to about 30 mM buffering agent (e.g., one or more of histidine-histidine hydrochloride buffering agent, succinic acid-sodium succinate buffering agent and citric acid-sodium citrate buffering agent),
(c) about 150 mM to about 320 mM saccharide, and one or more of about 10 mM to about 300 mM sodium chloride, 10 mM to about 300 mM arginine-hydrochloride, 10 mM to about 300 mM glycine and 10 mM to about 300 mM proline, and
(d) about 0.001% to about 0.1% (w/v) surfactant,
wherein the lyophilized formulation has a pH of about 5.0 to about 7.5, preferably about 6.0 to about 6.5.

In some embodiments, the aforementioned lyophilized formulation comprises:
(a) about 0.5 mg/mL to about 20 mg/mL bispecific anti-CD3/CD20 polypeptide complex (e.g., about 0.5 mg/mL, about 1 mg/mL, about 1.5 mg/mL, about 2 mg/mL, about 2.5 mg/mL, about 3 mg/mL, about 3.5 mg/mL, about 4 mg/mL, about 4.5 mg/mL, about 5 mg/mL, about 5.5 mg/mL, about 6 mg/mL, about 6.5 mg/mL, about 7 mg/mL, about 7.5 mg/mL, about 8 mg/mL, about 8.5 mg/mL, about 9 mg/mL, about 9.5 mg/mL, about 10 mg/mL, about 11 mg/mL, about 12 mg/mL, about 13 mg/mL, about 14 mg/mL, about 15 mg/mL, about 16 mg/mL, about 17 mg/mL, about 18 mg/mL, about 19 mg/mL or about 20 mg/mL),
(b) about 5 mM to about 20 mM histidine-histidine hydrochloride or citric acid-sodium citrate buffering agent (e.g., about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM or about 20 mM),
(c) about 180 mM to about 265 mM trehalose or sucrose (e.g., about 180 mM, about 185 mM, about 198 mM, about 211 mM, about 214 mM, about 217 mM, about 219 mM, about 222 mM, about 225 mM, about 227 mM, about 230 mM, about 233 mM, about 235 mM, about 238 mM, about 241 mM, about 243 mM, about 246 mM, about 248 mM, about 251 mM, 254 mM, about 256 mM, about 259 mM, about 262 mM or about 264 mM; or about 180 mM, about 185.02 mM, about 198.24 mM, about 211.46 mM, about 214.1 mM, about 216.74 mM, about 219.39 mM, about 222.03 mM, about 224.67 mM, about 227.31 mM, about 229.96 mM, about 232.6 mM, about 235.24 mM, about 237.89 mM, about 240.53 mM, about 243.17 mM, about 245.82 mM, about 248.46 mM, about 251.1 mM, 253.75 mM, about 256.39 mM, about 259.03 mM, about 261.68 mM or about 264.32 mM), and about 20 mM to about 200 mM sodium chloride and/or about 20 mM to about 200 mM arginine-hydrochloride (e.g., about 20 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM, about 100 mM, about 105 mM, about 110 mM, about 115 mM, about 120 mM, about 125 mM, about 130 mM, about 135 mM, about 140 mM, about 145 mM, about 150 mM, about 160 mM, about 170 mM, about 180 mM, about 190 mM or about 200 mM), and
(d) about 0.005% to about 0.08% (w/v) polysorbate 80 or polysorbate 20 (e.g., about 0.005% (w/v), about 0.006% (w/v), about 0.007% (w/v), about 0.008% (w/v), about 0.009% (w/v), about 0.01% (w/v), about 0.015% (w/v), about 0.02% (w/v), about 0.025% (w/v), about 0.03% (w/v), about 0.035% (w/v), about 0.04% (w/v), about 0.045% (w/v), about 0.05% (w/v), about 0.06% (w/v), about 0.07% (w/v) or about 0.08% (w/v)),

wherein the lyophilized formulation has a pH of about 6.0 to about 6.5 (e.g., about 6.0, about 6.1, about 6.2, about 6.3, about 6.4 or about 6.5). In some embodiments, the aforementioned lyophilized formulation comprises:
   (a) about 0.1 mg/mL to about 50 mg/mL bispecific anti-CD3/CD20 polypeptide complex,
   (b) about 1 mM to about 30 mM histidine-histidine hydrochloride or citric acid-sodium citrate buffering agent,
   (c) about 150 mM to about 320 mM trehalose or sucrose, and about 20 mM to about 200 mM sodium chloride and/or about 20 mM to about 200 mM arginine-hydrochloride, and
   (d) about 0.001% to about 0.1% (w/v) polysorbate 80 or polysorbate 20,
wherein the lyophilized formulation has a pH of about 5.0 to about 7.5, preferably about 6.0 to about 6.5.

In some embodiments, the aforementioned lyophilized formulation comprises:
(a) about 0.5 mg/mL to about 20 mg/mL bispecific anti-CD3/CD20 polypeptide complex,
(b) about 5 mM to about 20 mM histidine-histidine hydrochloride or citric acid-sodium citrate buffering agent,
(c) about 180 mM to about 265 mM trehalose or sucrose, and about 20 mM to about 200 mM sodium chloride and/or about 20 mM to about 200 mM arginine-hydrochloride, and
(d) about 0.005% to about 0.08% (w/v) polysorbate 80 or polysorbate 20,
wherein the lyophilized formulation has a pH of about 6.0 to about 6.5.

In some embodiments, the aforementioned lyophilized formulation comprises:
(a) about 0.1 mg/mL to about 50 mg/mL bispecific anti-CD3/CD20 polypeptide complex,
(b) about 1 mM to about 30 mM citric acid-sodium citrate buffering agent,
(c) about 150 mM to about 320 mM trehalose, and about 20 mM to about 200 mM sodium chloride and/or about 20 mM to about 200 mM arginine-hydrochloride, and
(d) about 0.001% to about 0.1% (w/v) polysorbate 80 or polysorbate 20,
wherein the lyophilized formulation has a pH of about 5.0 to about 7.5, preferably about 6.0 to about 6.5.

In some embodiments, the aforementioned lyophilized formulation comprises:
(a) about 0.5 mg/mL to about 20 mg/mL bispecific anti-CD3/CD20 polypeptide complex,
(b) about 5 mM to about 20 mM citric acid-sodium citrate buffering agent,
(c) about 180 mM to about 265 mM trehalose, and about 20 mM to about 200 mM sodium chloride and/or about 20 mM to about 200 mM arginine-hydrochloride, and
(d) about 0.005% to about 0.08% (w/v) polysorbate 80 or polysorbate 20,
wherein the lyophilized formulation has a pH of about 6.0 to about 6.5.

In some embodiments, the aforementioned lyophilized formulation comprises:
(a) about 0.1 mg/mL to about 50 mg/mL bispecific anti-CD3/CD20 polypeptide complex,
(b) about 1 mM to about 30 mM citrate buffering agent,
(c) about 150 mM to about 320 mM trehalose, and about 10 mM to about 300 mM sodium chloride, and
(d) about 0.001% to about 0.1% (w/v) polysorbate 80 or polysorbate 20,
wherein the lyophilized formulation has a pH of about 5.0 to about 7.5, preferably about 6.0 to about 6.5.

In some embodiments, the aforementioned lyophilized formulation comprises:
(a) about 0.5 mg/mL to about 20 mg/mL bispecific anti-CD3/CD20 polypeptide complex,
(b) about 5 mM to about 20 mM citrate buffering agent,
(c) about 180 mM to about 265 mM trehalose, and about 20 mM to about 200 mM sodium chloride, and
(d) about 0.005% to about 0.08% (w/v) polysorbate 80 or polysorbate 20,
wherein the lyophilized formulation has a pH of about 6.0 to about 6.5.

In some embodiments, the aforementioned lyophilized formulation comprises:
(a) about 1 mg/mL to about 20 mg/mL bispecific anti-CD3/CD20 polypeptide complex,
(b) about 5 mM to about 20 mM citrate buffering agent,
(c) about 180 mM to about 265 mM trehalose, and about 20 mM to about 200 mM sodium chloride, and
(d) about 0.01% to about 0.04% (w/v) polysorbate 80 or polysorbate 20,
wherein the lyophilized formulation has a pH of about 6.0 to about 6.5.

In some embodiments, the aforementioned lyophilized formulation comprises:
(a) about 2 mg/mL to about 3 mg/mL bispecific anti-CD3/CD20 polypeptide complex,
(b) about 10 mM citric acid-sodium citrate buffering agent,
(c) about 233 mM trehalose, and
(d) about 0.02% (w/v) polysorbate 80,
wherein the lyophilized formulation has a pH of about 6.2.

In some embodiments, the aforementioned lyophilized formulation comprises:
(a) about 2 mg/mL to about 3 mg/mL bispecific anti-CD3/CD20 polypeptide complex,
(b) about 10 mM citric acid-sodium citrate buffering agent,
(c) about 232.6 mM trehalose, and
(d) about 0.02% (w/v) polysorbate 80,
wherein the lyophilized formulation has a pH of about 6.2.

In some embodiments, the aforementioned lyophilized formulation comprises:
(a) about 5 mg/mL bispecific anti-CD3/CD20 polypeptide complex,
(b) about 10 mM citrate buffering agent,
(c) about 233 mM trehalose, and about 103 mM sodium chloride, and
(d) about 0.02% (w/v) polysorbate 80,
wherein the lyophilized formulation has a pH of about 6.

In some embodiments, the aforementioned lyophilized formulation comprises:
(a) about 5 mg/mL bispecific anti-CD3/CD20 polypeptide complex,
(b) about 10 mM citrate buffering agent,
(c) about 232.6 mM trehalose, and about 102.7 mM sodium chloride, and
(d) about 0.02% (w/v) polysorbate 80,
wherein the lyophilized formulation has a pH of about 6.

In some embodiments, the aforementioned lyophilized formulation comprises:
(a) about 2 mg of bispecific anti-CD3/CD20 polypeptide complex,
(b) about 0.0013 mmol or about 0.0014 mmol of citric acid,
(c) about 0.0086 mmol of sodium citrate,
(d) about 0.233 mmol of trehalose, and
(e) about 0.2 mg of polysorbate 80;

or comprises multiples of (a), (b), (c), (d) and (e), e.g., an integer multiple or a fraction multiple of the amount of each of (a), (b), (c), (d) and (e) components, or 1.5 times, 2 times, 3 times or 4 times of the amount of each of (a), (b), (c), (d) and (e) components, or an integer multiple of more than 5 of the amount of each of (a), (b), (c), (d) and (e) components,
wherein the lyophilized formulation has a pH of about 6.2.

In some embodiments, the aforementioned lyophilized formulation comprises:
(a) about 2 mg of bispecific anti-CD3/CD20 polypeptide complex,
(b) about 0.0013 mmol or about 0.0014 mmol of citric acid,
(c) about 0.0086 mmol of sodium citrate,
(d) about 0.2326 mmol of trehalose, and
(e) about 0.2 mg of polysorbate 80;

or comprises multiples of (a), (b), (c), (d) and (e), e.g., an integer multiple or a fraction multiple of the amount of each of (a), (b), (c), (d) and (e) components, or 1.5 times, 2 times, 3 times or 4 times of the amount of each of (a), (b), (c), (d) and (e) components, or an integer multiple of more than 5 of the amount of each of (a), (b), (c), (d) and (e) components,
wherein the lyophilized formulation has a pH of about 6.2.

In some embodiments, the aforementioned lyophilized formulation comprises:
(a) about 2 mg of bispecific anti-CD3/CD20 polypeptide complex,
(b) about 0.001 mmol of citric acid,
(c) about 0.009 mmol of sodium citrate,
(d) about 0.23 mmol of trehalose, and
(e) about 0.2 mg of polysorbate 80;

or comprises multiples of (a), (b), (c), (d) and (e), e.g., an integer multiple or a fraction multiple of the amount of each of (a), (b), (c), (d) and (e) components, or 1.5 times, 2 times, 3 times or 4 times of the amount of each of (a), (b), (c), (d) and (e) components, or an integer multiple of more than 5 of the amount of each of (a), (b), (c), (d) and (e) components,
wherein the lyophilized formulation has a pH of about 6.2.

In some embodiments, the aforementioned lyophilized formulation comprises:
(a) about 5 mg of bispecific anti-CD3/CD20 polypeptide complex,
(b) about 0.0013 mmol or about 0.0014 mmol of citric acid,
(c) about 0.0086 mmol of sodium citrate,
(d) about 0.233 mmol of trehalose, and 6 mg of sodium chloride, and
(e) about 0.2 mg of polysorbate 80;

or comprises multiples of (a), (b), (c), (d) and (e), e.g., an integer multiple or a fraction multiple of the amount of each of (a), (b), (c), (d) and (e) components, or 1.5 times, 2 times, 3 times or 4 times of the amount of each of (a), (b), (c), (d) and (e) components, or an integer multiple of more than 5 of the amount of each of (a), (b), (c), (d) and (e) components,
wherein the lyophilized formulation has a pH of about 6.

In some embodiments, the aforementioned lyophilized formulation comprises:
(a) about 5 mg of bispecific anti-CD3/CD20 polypeptide complex,
(b) about 0.0013 mmol or about 0.0014 mmol of citric acid,
(c) about 0.0086 mmol of sodium citrate,
(d) about 0.2326 mmol of trehalose, and 6 mg of sodium chloride, and
(e) about 0.2 mg of polysorbate 80;

or comprises multiples of (a), (b), (c), (d) and (e), e.g., an integer multiple or a fraction multiple of the amount of each of (a), (b), (c), (d) and (e) components, or 1.5 times, 2 times, 3 times or 4 times of the amount of each of (a), (b), (c), (d) and (e) components, or an integer multiple of more than 5 of the amount of each of (a), (b), (c), (d) and (e) components,
wherein the lyophilized formulation has a pH of about 6.

In some embodiments, the aforementioned lyophilized formulation comprises:
(a) about 5 mg of bispecific anti-CD3/CD20 polypeptide complex,
(b) about 0.001 mmol of citric acid,
(c) about 0.009 mmol of sodium citrate,
(d) about 0.23 mmol of trehalose, and 6 mg of sodium chloride, and
(e) about 0.2 mg of polysorbate 80;

or comprises multiples of (a), (b), (c), (d) and (e), e.g., an integer multiple or a fraction multiple of the amount of each of (a), (b), (c), (d) and (e) components, or 1.5 times, 2 times, 3 times or 4 times of the amount of each of (a), (b), (c), (d) and (e) components, or an integer multiple of more than 5 of the amount of each of (a), (b), (c), (d) and (e) components,
wherein the lyophilized formulation has a pH of about 6.

In some embodiments, the lyophilized formulation is stable at 2-8 °C for at least 1 month, at least 6 months, at least 12 months, at least 18 months or at least 24 months. In some embodiments, the lyophilized formulation is stable at 25 °C for at least 1 month or at least 6 months. In some embodiments, the lyophilized formulation is stable at 40 °C for at least 7 days, at least 14 days or at least 28 days.

The present disclosure also provides a pharmaceutical composition containing a bispecific anti-CD3/CD20 polypeptide complex, which is a solution obtained by reconstituting the aforementioned lyophilized formulation. The present disclosure (not claimed) also provides a method for preparing the aforementioned pharmaceutical composition, which comprises a step of reconstituting the aforementioned lyophilized formulation, wherein a solution for the reconstitution includes, but is not limited to, water for injection, normal saline or glucose solution; water for injection is preferred.

The present disclosure (not claimed) also provides an article of manufacture comprising a container containing the lyophilized formulation or the pharmaceutical composition obtained by reconstituting the lyophilized formulation disclosed herein.

The lyophilized formulation or the pharmaceutical composition obtained by reconstituting the lyophilized formulation disclosed herein can be administered intravenously (e.g., intravenous drip), intramuscularly, subcutaneously, parenterally, spinally or epicutaneously (e.g., by injection or bolus injection). The lyophilized formulation or the pharmaceutical composition obtained by reconstituting the lyophilized formulation disclosed herein can be diluted with a suitable diluent to a suitable concentration before administration to provide the best desired response (e.g., therapeutic response).

In another aspect, the present disclosure (not claimed) provides use of the lyophilized formulation or the pharmaceutical composition obtained by reconstituting the lyophilized formulation or the article of manufacture disclosed herein for manufacturing a medicament for treating a CD20-related disease or disorder.

In some embodiments, the CD20-related disease or disorder is cancer; preferably the cancer is selected from the group consisting of, but is not limited to, lymphoma, lung cancer, liver cancer, cervical cancer, colon cancer, breast cancer, ovarian cancer, pancreatic cancer, melanoma, glioblastoma, prostate cancer, esophageal cancer and gastric cancer.

In some embodiments, the CD20-related disease or disorder is B cell lymphoma, optionally Hodgkin lymphoma or non-Hodgkin lymphoma, wherein the non-Hodgkin lymphoma includes: diffuse large B-cell lymphoma (DLBCL), follicular lymphoma, marginal zone B-cell lymphoma (MZL), mucosa-associated lymphoid tissue lymphoma (MALT), small lymphocytic lymphoma (chronic lymphocytic leukemia, CLL), or mantle cell lymphoma (MCL), acute lymphocytic leukemia (ALL), or Waldenström macroglobulinemia (WM).

In optional embodiments, the bispecific anti-CD3/CD20 polypeptide complex in the aforementioned liquid formulation, or lyophilizate or pharmaceutical composition obtained by reconstituting the lyophilizate, or lyophilized formulation or pharmaceutical composition obtained by reconstituting the lyophilized formulation, or article of manufacture, comprises a first antigen-binding portion associated with a second antigen-binding portion, wherein:
the first antigen-binding portion comprises:
a first polypeptide, comprising from N-terminus to C-terminus a first heavy chain variable domain (VH) of a first antibody operably linked to a first T Cell Receptor (TCR) constant region (C1), and
a second polypeptide, comprising from N-terminus to C-terminus a first light chain variable domain (VL) of the first antibody operably linked to a second TCR constant region (C2),
wherein C1 and C2 can form a dimer comprising at least one non-native interchain bond between C1 and C2, and
the non-native interchain bond can stabilize the dimer, and
the second antigen-binding portion comprises:
   a second heavy chain variable domain (VH2) of a second antibody operably linked to an antibody heavy chain CH1 domain, and
   a second light chain variable domain (VL2) of the second antibody operably linked to an antibody light chain constant (CL) domain,
   wherein one of the first antigen-binding portion and the second antigen-binding portion is an anti-CD3 binding portion, and the other one is an anti-CD20 binding portion;
   the anti-CD3 binding portion is derived from an anti-CD3 antibody, comprising:
      a) heavy chain CDR1 comprising an amino acid sequence selected from the group consisting of SEQ ID NOs: 1 and 13,
      b) heavy chain CDR2 comprising an amino acid sequence selected from the group consisting of SEQ ID NOs: 2 and 14,
      c) heavy chain CDR3 comprising an amino acid sequence selected from the group consisting of SEQ ID NOs: 3 and 15,
      d) κ light chain CDR1 comprising an amino acid sequence selected from the group consisting of SEQ ID NOs: 4 and 16,
      e) κ light chain CDR2 comprising an amino acid sequence selected from the group consisting of SEQ ID NOs: 5 and 17, and
      f) κ light chain CDR3 comprising an amino acid sequence selected from the group consisting of SEQ ID NOs: 6 and 18,
   the anti-CD20 binding portion is derived from an anti-CD20 antibody, comprising:
      a) heavy chain CDR1 comprising an amino acid sequence selected from the group consisting of SEQ ID NOs: 7 and 19,
      b) heavy chain CDR2 comprising an amino acid sequence selected from the group consisting of SEQ ID NOs: 8 and 20,
      c) heavy chain CDR3 comprising an amino acid sequence selected from the group consisting of SEQ ID NOs: 9 and 21,
      d) κ light chain CDR1 comprising an amino acid sequence selected from the group consisting of SEQ ID NOs: 10 and 22,
      e) κ light chain CDR2 comprising an amino acid sequence selected from the group consisting of SEQ ID NOs: 11 and 23, and
      f) κ light chain CDR3 comprising an amino acid sequence selected from the group consisting of SEQ ID NOs: 12 and 24.

In some embodiments, the anti-CD3 binding portion is derived from an anti-CD3 antibody, comprising:
a) heavy chain CDR1 comprising an amino acid sequence of SEQ ID NO: 13,
b) heavy chain CDR2 comprising an amino acid sequence of SEQ ID NO: 14,
c) heavy chain CDR3 comprising an amino acid sequence of SEQ ID NO: 15,
d) κ light chain CDR1 comprising an amino acid sequence of SEQ ID NO: 16,
e) κ light chain CDR2 comprising an amino acid sequence of SEQ ID NO: 17, and
f) κ light chain CDR3 comprising an amino acid sequence of SEQ ID NO: 18.

In some embodiments, the anti-CD20 binding portion is derived from an anti-CD20 antibody, comprising:
a) heavy chain CDR1 comprising an amino acid sequence of SEQ ID NO: 19,
b) heavy chain CDR2 comprising an amino acid sequence of SEQ ID NO: 20,
c) heavy chain CDR3 comprising an amino acid sequence of SEQ ID NO: 21,
d) κ light chain CDR1 comprising an amino acid sequence of SEQ ID NO: 22,
e) κ light chain CDR2 comprising an amino acid sequence of SEQ ID NO: 23, and
f) κ light chain CDR3 comprising an amino acid sequence of SEQ ID NO: 24.

In optional embodiments, the anti-CD3 binding portion of the bispecific anti-CD3/CD20 polypeptide complex in the aforementioned liquid formulation, or lyophilizate or pharmaceutical composition obtained by reconstituting the lyophilizate, or lyophilized formulation or pharmaceutical composition obtained by reconstituting the lyophilized formulation, or article of manufacture, is derived from an anti-CD3 antibody, comprising: a heavy chain variable domain sequence comprising an amino acid sequence selected from the group consisting of SEQ ID NOs: 25 and 27, and a light chain variable domain sequence comprising an amino acid sequence selected from the group consisting of SEQ ID NOs: 26 and 28. In some embodiments, the anti-CD3 binding portion is derived from an anti-CD3 antibody, comprising: a heavy chain variable domain sequence comprising a variant of an amino acid sequence selected from the group consisting of SEQ ID NOs: 25 and 27, e.g., a heavy chain variable domain sequence comprising an amino acid sequence of SEQ ID NO: 25 or 27 with C-terminal deletions of amino acids SS, and a light chain variable domain sequence comprising an amino acid sequence selected from the group consisting of SEQ ID NOs: 26 and 28. In some embodiments, the anti-CD3 binding portion is derived from an anti-CD3 antibody, comprising: a heavy chain variable domain sequence comprising an amino acid sequence of SEQ ID NO: 27, and a light chain variable domain sequence comprising an amino acid sequence of SEQ ID NO: 28. In some embodiments, the anti-CD3 binding portion is derived from an anti-CD3 antibody, comprising: a heavy chain variable domain sequence comprising a variant of an amino acid sequence of SEQ ID NO: 27, e.g., a heavy chain variable domain sequence comprising an amino acid sequence of SEQ ID NO: 27 with C-terminal deletions of amino acids SS, and a light chain variable domain sequence comprising an amino acid sequence of SEQ ID NO: 28.

In optional embodiments, the anti-CD20 binding portion of the bispecific anti-CD3/CD20 polypeptide complex in the aforementioned liquid formulation, or lyophilizate or pharmaceutical composition obtained by reconstituting the lyophilizate, or lyophilized formulation or pharmaceutical composition obtained by reconstituting the lyophilized formulation, or article of manufacture, is derived from an anti-CD20 antibody, comprising: a heavy chain variable domain sequence comprising an amino acid sequence selected from the group consisting of SEQ ID NO: 29 and 31, and a light chain variable domain sequence comprising an amino acid sequence selected from the group consisting of SEQ ID NO: 30 and 32. In some embodiments, the anti-CD20 binding portion is derived from an anti-CD20 antibody, comprising: a heavy chain variable domain sequence comprising an amino acid sequence of SEQ ID NO: 31, and a light chain variable domain sequence comprising an amino acid sequence of SEQ ID NO: 32.

In optional embodiments, the first antigen-binding portion is linked to a first dimerization domain and the second antigen-binding portion is linked to a second dimerization domain, wherein the first dimerization domain and the second dimerization domain are associated. In optional embodiments, the association is achieved via a linker, a disulfide bond, a hydrogen bond, an electrostatic interaction, a salt bridge or a hydrophobic-hydrophilic interaction or a combination thereof.

In optional embodiments, the first dimerization domain and/or the second dimerization domain comprise at least a portion of an antibody hinge region, which is optionally from IgG1, IgG2 or IgG4.

In optional embodiments, the first dimerization domain and/or the second dimerization domain comprise an antibody CH2 domain and/or an antibody CH3 domain.

In optional embodiments, the first dimerization domain is operably linked to the first TCR constant region (C1) at a third junction domain.

In optional embodiments, the second dimerization domain is operably linked to a heavy chain variable domain of the second antigen-binding portion.

In optional embodiments, the first dimerization domain and the second dimerization domain are different and are associated in a way that discourages homodimerization and/or favors heterodimerization.

In optional embodiments, the first dimerization domain and the second dimerization domain can be associated to form a heterodimer via knobs-into-holes, a hydrophobic interaction, an electrostatic interaction, a hydrophilic interaction or increased flexibility.

In optional embodiments, the bispecific anti-CD3/CD20 polypeptide complex in the aforementioned liquid formulation, or lyophilizate or pharmaceutical composition obtained by reconstituting the lyophilizate, or lyophilized formulation or pharmaceutical composition obtained by reconstituting the lyophilized formulation, or article of manufacture, comprises a combination of the following four polypeptide sequences: SEQ ID NO: 33, SEQ ID NO: 34, SEQ ID NO: 35 and SEQ ID NO: 36.

In optional embodiments, the bispecific anti-CD3/CD20 polypeptide complex in the aforementioned liquid formulation, or lyophilizate or pharmaceutical composition obtained by reconstituting the lyophilizate, or lyophilized formulation or pharmaceutical composition obtained by reconstituting the lyophilized formulation, or article of manufacture, comprises a combination of the following four polypeptide sequences: SEQ ID NO: 37, SEQ ID NO: 38, SEQ ID NO: 39 and SEQ ID NO: 40.

In optional embodiments, the bispecific anti-CD3/CD20 polypeptide complex in the aforementioned liquid formulation, or lyophilizate or pharmaceutical composition obtained by reconstituting the lyophilizate, or lyophilized formulation or pharmaceutical composition obtained by reconstituting the lyophilized formulation, or article of manufacture, comprises a combination of the following four polypeptide sequences: SEQ ID NO: 41, SEQ ID NO: 42, SEQ ID NO: 43 and SEQ ID NO: 44.

In optional embodiments, the bispecific anti-CD3/CD20 polypeptide complex in the aforementioned liquid formulation, or lyophilizate or pharmaceutical composition obtained by reconstituting the lyophilizate, or lyophilized formulation or pharmaceutical composition obtained by reconstituting the lyophilized formulation, or article of manufacture, comprises a combination of the following four polypeptide sequences: SEQ ID NO: 45, SEQ ID NO: 46, SEQ ID NO: 47 and SEQ ID NO: 48.

In optional embodiments, the bispecific anti-CD3/CD20 polypeptide complex in the aforementioned liquid formulation, or lyophilizate or pharmaceutical composition obtained by reconstituting the lyophilizate, or lyophilized formulation or pharmaceutical composition obtained by reconstituting the lyophilized formulation, or article of manufacture, comprises a combination of the following four polypeptide sequences: SEQ ID NO: 49, SEQ ID NO: 50, SEQ ID NO: 51 and SEQ ID NO: 52.

In optional embodiments, one or more of the amino acids at positions 193, 182, 203, 206 and 207 in the polypeptide set forth in SEQ ID NO: 44 are modified to any amino acids other than serine and/or threonine so glycosylation sites are eliminated; preferably, the amino acid at position 193 is modified. In optional embodiments, the amino acid at position 193 in the polypeptide set forth in SEQ ID NO: 44 is modified to alanine, glycine, proline or valine.

The foregoing and other features as well as advantages of the present disclosure will become apparent from the following detailed description of several embodiments which proceeds with reference to the accompanying figures.

### BRIEF DESCRIPTION OF THE DRAWINGS

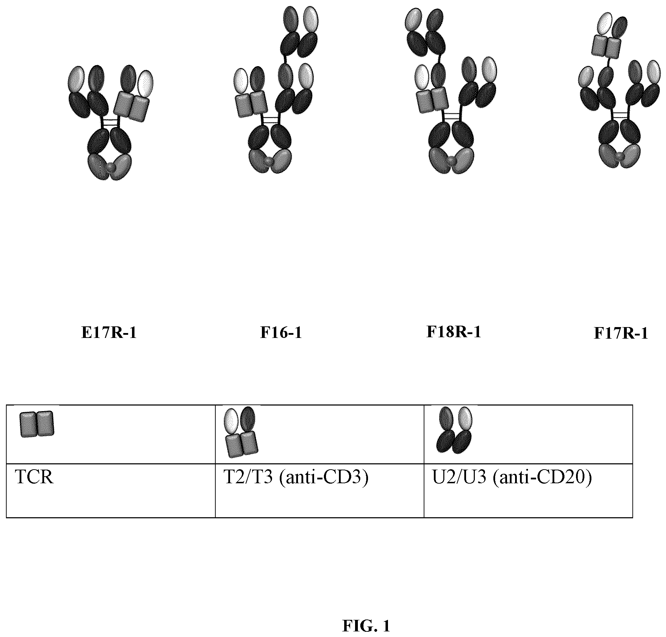

FIG. 1 presents a schematic of the studied antibodies formats, wherein "E17R-1", "F16-1" and "F17R-1" schematically represent the formats of bispecific antibodies W3278-T2U3.E17R-1.uIgG4.SP, W3278-T3U2.F16-1.uIgG4.SP and W3278-T3U2.F17R-1.ulgG4.SP, respectively, and "F18R-1" schematically represents the formats of bispecific antibodies W3278-U2T3.F18R-1.uIgG4.SP and W3278-U3T2.F18R-1.uIgG4.SP. In the context of the present disclosure, "U" in the names of bispecific antibodies refers to an anti-CD20 antibody or an anti-CD20 binding portion, and "T" in the names of bispecific antibodies refers to an anti-CD3 antibody or an anti-CD3 binding portion. A unique light-heavy chain interface that is orthogonal to conventional antibodies is designed by replacing the constant regions of "T" (CL and CH1) with the constant domains of TCR. The TCR-modified "T" and native "U" are used in conjunction with the "knobs-into-holes" mutations in the Fc domain to design bispecific antibody formats "E17R-1", "F16-1", "F17R-1" and "F18R-1".
FIG. 2 shows target binding detected by FACS: (A) binding assay of the bispecific antibody of the present disclosure (WBP3278 BsAb) to Jurkat cells, and (B) binding assay of the bispecific antibody of the present disclosure (WBP3278 BsAb) to Raji cells.
FIG. 3 shows simultaneous dual target binding detected by FACS.
FIG. 4 shows T cell killing.
FIGs. 5A and 5B show T cell activation indicated by CD25 expression and by CD69 expression, respectively.
FIG. 6 shows IL-2 (A) and TNF-α (B) releases from CD4⁺ T cells.
FIG. 7 shows the results of serum stability.
FIG. 8 shows the dose-dependent anti-tumor activity of BsAb of the present disclosure in an *in vivo* therapeutic treatment model.
FIG. 9 shows the depletion of circulating B cells in peripheral blood following administration of WBP3278 lead Ab (i.e., W3278-U2T3.F18R-1.uIgG4) to male cynomolgus monkeys first used for immunization.
FIGs. 10A, 10B and 10C show changes in the level of circulating T cells in peripheral blood following WBP3278 lead Ab treatment.
FIG. 11 shows changes in circulating cytokine levels following WBP3278 lead Ab treatment.
FIG. 12 shows changes in serum concentration of WBP3278 lead Ab over time.

### DETAILED DESCRIPTION

The following description of the present disclosure is intended to be only illustrative of various embodiments of the present disclosure.

### Definitions

"Histidine salt buffer" is a buffer comprising histidine ions, or a buffer comprising histidine and/or a histidine salt, wherein the histidine includes histidine and/or a hydrate thereof, and the histidine salt includes the histidine salt and/or a hydrate thereof. Examples of histidine salt buffers include such buffers as histidine-hydrochloride, histidine-acetate, histidine-phosphate, histidine-sulfate and histidine-histidine hydrochloride; histidine-histidine hydrochloride is preferred. Histidine-histidine hydrochloride refers to a combination of histidine and histidine hydrochloride, wherein the histidine includes histidine and/or a hydrate thereof, and the histidine hydrochloride includes histidine hydrochloride and/or a hydrate thereof; as an example, the histidine-histidine hydrochloride buffer is prepared with histidine and histidine hydrochloride monohydrate (C₆H₉N₃O₂•HCl•H₂O).

"Succinate buffer" is a buffer comprising succinate ions, or a buffer comprising succinic acid and/or a succinate salt, wherein the succinic acid includes succinic acid and/or a hydrate thereof, and the succinate salt includes the succinate salt and/or a hydrate thereof. Examples of succinate buffers include succinic acid-sodium succinate, succinic acid-potassium succinate and succinic acid-calcium succinate; preferably, the succinate buffer is succinic acid-sodium succinate. Succinic acid-sodium succinate refers to a combination of succinic acid and sodium succinate, wherein the succinic acid includes succinic acid and/or a hydrate thereof, and the sodium succinate includes sodium succinate and/or a hydrate thereof.

"Citrate buffer" is a buffer comprising citrate ions, or a buffer comprising citric acid and/or a citrate salt, wherein the citric acid includes citric acid and/or a hydrate thereof, and the citrate salt includes the citrate salt and/or a hydrate thereof. Examples of citrate buffers include citric acid-sodium citrate, citric acid-potassium citrate, citric acid-calcium citrate and citric acid-magnesium citrate; preferably, the citrate buffer is citric acid-sodium citrate. Citric acid-sodium citrate refers to a combination of citric acid and sodium citrate, wherein the citric acid includes citric acid and/or a hydrate thereof, and the sodium citrate includes sodium citrate and/or a hydrate thereof; as an example, the citric acid-sodium citrate buffer is prepared by mixing citric acid monohydrate (C₆H₈O₇•H₂O) with sodium citrate dihydrate (C₆H₅ Na₃O₇•2H₂O).

"Buffer" and "buffering agent" are to aid in maintaining the pH of the formulation within a range that approximates physiological conditions. Suitable buffers/buffering agents for the present disclosure include inorganic acids paired with salts thereof or organic acids paired with salts thereof, e.g., citrate buffers/buffering agents (e.g., a citric acid-potassium citrate mixture, or a citric acid-sodium citrate mixture), succinate buffers/ buffering agents (e.g., a succinic acid-sodium succinate mixture, or a succinic acid-sodium hydroxide mixture), histidine salt buffers/buffering agents (e.g., a histidine-histidine hydrochloride mixture). As an example, the citric acid-sodium citrate buffer/buffering agent is prepared by mixing citric acid monohydrate (C₆H₈O₇•H₂O) with sodium citrate dihydrate (C₆H₅ Na₃O₇•2H₂O).

"Pharmaceutical composition" and "formulation" are intended to encompass products comprising a specified ingredient (e.g., a bispecific anti-CD3/CD20 polypeptide complex), optionally in a specified amount, and any product which results, directly or indirectly, from the combination of specified ingredients, optionally in specified amounts. "Stable" or "stabilized" pharmaceutical compositions or formulations are those in which the active ingredient (e.g., a protein or an antibody) substantially retains its physical and/or chemical stability and/or biological activity during storage. Various analytical techniques for determining the stability of an active ingredient are known in the art, for example, reviewed in Peptide and Protein Drug Delivery, 247-301, Vincent Lee Ed., Marcel Dekker, Inc., New York, N.Y., Pubs. (1991) and Jones, A. Adv. Drug Delivery Rev. 10:29-90 (1993). Stability can be measured at selected temperatures and under other storage conditions over a selected period of time. For example, an active ingredient "retains its physical stability" in a pharmaceutical formulation if it does not exhibit a significant increase in aggregation, precipitation and/or denaturation upon visual inspection of color and/or clarity, or as measured by UV light scattering, size exclusion chromatography (SEC) and differential scanning calorimetry (DSC). Preferably, when the formulation of the present disclosure is used, 5% or less, 4% or less, preferably 3% or less of the active ingredient forms aggregates, as measured by, for example, SEC-HPLC or any other suitable method for measuring aggregate formation. An active ingredient (e.g., a protein or an antibody) "retains its chemical stability" in a pharmaceutical formulation if the active ingredient (e.g., the protein or the antibody) does not exhibit a significant chemical change. Chemical stability can be assessed by detecting and quantifying the chemically altered formats of protein or antibody. Degradation processes that often alter the chemical structure of a protein include hydrolysis or truncation (assessed by methods such as size exclusion chromatography and SDS-PAGE), oxidation (assessed by methods such as peptide mapping coupled with mass spectrometry or MALDI/TOF/MS), deamidation (assessed by methods such as ion exchange chromatography, capillary isoelectric focusing, peptide mapping and isoaspartic acid measurement) and isomerization (assessed by measuring isoaspartic acid content, by peptide mapping, etc.). An active ingredient (e.g., a protein or an antibody) "retains its biological activity" in a pharmaceutical formulation at a given time, e.g., as determined by an antigen-binding assay, if the biological activity of the active ingredient (e.g., the protein or the antibody) at the given time is at least about 90% (within assay error) of that at the time of preparing the pharmaceutical formulation. In some specific embodiments, the pharmaceutical composition or the formulation is a water-soluble injection, including but not limited to a water-soluble formulation not lyophilized or a water-soluble formulation obtained by reconstituting a lyophilized powder. In other embodiments, the pharmaceutical composition or the formulation is a lyophilized formulation. The lyophilized formulation refers to a formulation prepared by subjecting an aqueous solution to a lyophilization process. Lyophilization is a stabilization process, in which a substance is first frozen, and then the amount of a solvent is reduced by sublimation (primary drying process) and then by desorption (secondary drying process) until the amount of the solvent is reduced to a value that no longer supports a biological activity or a chemical reaction. The lyophilized formulation of the present disclosure can also be dried by other methods known in the art, such as spray drying and bubbledrying.

The articles "a", "an" and "the" are used herein to refer to one or more (i.e., at least one) of the grammatical objects of the article. For example, "a formulation" refers to one or more formulations.

The term "about" or "approximately" means a numerical value is within an acceptable error range for the particular value determined by those of ordinary skill in the art, and the numerical value depends in part on how the measurement or determination is carried out (i.e., the limits of the measurement system). For example, "about" or "approximately" means a range of ±5%. Furthermore, particularly for a biological system or process, the term can mean at most an order of magnitude or at most 5 times the value. In the present disclosure, unless otherwise stated, "about XX" or "approximately XX" or "substantially comprising XX" means a numeral value within an acceptable error range for the particular value "XX" (including the numeral value "XX" itself, as well as those within an acceptable error range for the determination of the numeral value by those of ordinary skill in the art). Throughout the present disclosure, unless the context dictates otherwise, the words "comprise/include", "comprises/includes" and "comprising/including" will be understood as comprising/including the steps or elements or a group of steps or elements but not excluding any other steps or elements or groups of steps or elements. "Consisting of..." means comprising and being limited to what follows the phrase "consisting of". Thus, the phrase "consisting of..." means that the listed elements are required or necessary and that no other elements can be present. "Substantially consisting of..." means comprising any listed element that follows the phrase and being limited to other elements that do not interfere with or are favorable for the listed elements' activity or effects as detailed in the present disclosure. Thus, the phrase "substantially consisting of..." means that the listed elements are required or necessary but other elements are optional and can be present or absent depending on whether they affect the listed elements' activity or effects.

"One embodiment", "an embodiment", "a particular embodiment", "a related embodiment", "a certain embodiment", "another embodiment" or "a further embodiment" referred to herein, or a combination thereof, means particular features, structures or characteristics described in connection with the embodiment are included in at least one embodiment of the present disclosure. Thus, the expression "aforementioned/forgoing" that appears throughout the specification does not necessarily refer to the same embodiment. Furthermore, the particular features, structures or characteristics may be combined in any suitable manner in one or more embodiments.

The terms "polypeptide", "peptide" and "protein" are used interchangeably in the present disclosure and refer to an aggregate of amino acid residues, or to a collection of multiple aggregates of amino acid residues. These terms are applied to amino acid aggregates in which one or more amino acid residues are artificial chemical mimics of corresponding naturally-occurring amino acids, and to naturally-occurring amino acid aggregates and non-naturally-occurring amino acid aggregates. The term "amino acid" refers to naturally-occurring amino acids and synthetic amino acids, as well as amino acid analogs and amino acid mimics that function similarly to the naturally-occurring amino acids. Naturally-occurring amino acids are those encoded by the genetic codes, as well as those amino acids that are subsequently modified, e.g., hydroxyproline, γ-carboxyglutamic acid and O-phosphoserine. Amino acid analogs refer to compounds having the same basic chemical structure as naturally-occurring amino acids (i.e., those in which the α-carbon is linked to hydrogen, carboxyl, amino or an R group), such as homoserine, norleucine, methionine sulfoxide and methionine methyl sulfonium. Such analogs have modified R groups (e.g., norleucine) or modified peptide frameworks, but retain the same basic chemical structure as a naturally-occurring amino acid. An α-carbon refers to the first carbon atom attached to a functional group (such as carbonyl). A β-carbon refers to the second carbon atom attached to an α-carbon, and in this system, carbon atoms are continuously named in Greek alphabetical order. Amino acid mimics refer to chemical compounds which have a structure that is different from the general chemical structure of amino acids but which function similarly to naturally-occurring amino acids. The term "protein" generally refers to a large polypeptide. The term "peptide" generally refers to a short polypeptide. In a polypeptide sequence, the left end is typically referred to as the amino terminus (N-terminus) and the right end as the carboxy terminus (C-terminus). "Polypeptide complex" as used herein refers to a complex comprising one or more polypeptides that are associated with fulfilling certain functions. In certain embodiments, the polypeptides are immunologically relevant.

The term "antibody" as used herein encompasses any immunoglobulin, monoclonal antibody, polyclonal antibody, multispecific antibody or bispecific (bivalent) antibody capable of binding to a particular antigen. A native, intact antibody comprises two heavy chains and two light chains. Each heavy chain consists of a variable region ("HCVR") and a first constant region, a second constant region and a third constant region (CH1, CH2 and CH3, respectively), and each light chain consists of a variable region ("LCVR") and a constant region (CL). Mammalian heavy chains can be classified into α, δ, ε, γ and µ, and mammalian light chains into λ or κ. Antibodies are Y-shaped, and the stem of the Y-shaped structure consists of the second constant regions and third constant regions of the two heavy chains, which are joined by disulfide bonds. Each arm of the Y-shaped structure comprises the variable region and the first constant region of one of the heavy chains, which are coupled with the variable region and the constant region of one of the light chains. The variable regions of the light chain and heavy chain are responsible for antigen-binding. The variable region of each chain comprises three hypervariable loops called complementarity determining regions (CDRs) (light (L) chain CDRs include LCDR1, LCDR2 and LCDR3, and heavy (H) chain CDRs include HCDR1, HCDR2 and HCDR3). The CDR limits of an antibody can be named or identified using the Kabat, Chothia, or Al-Lazikani scheme (Al-Lazikani, B., Chothia, C., Lesk, A. M., J. Mol. Biol., 273(4), 927 (1997); Chothia, C. et al., J Mol Biol. Dec 5; 186(3):651-63 (1985); Chothia, C. and Lesk, A.M., J. Mol.Biol., 196,901 (1987); Chothia, C. et al., Nature. Dec 21-28; 342(6252):877-83 (1989); Kabat E.A. et al., National Institutes of Health, Bethesda, Md. (1991)). The three CDRs are separated by flanking continuous portions called framework regions (FRs), which are more highly conserved than the CDRs and form a scaffold to support the hypervariable loops. Each HCVR and LCVR comprise four FRs, and the CDRs and FRs are arranged from amino-terminus to carboxy-terminus in the following order: FR1, CDR1, FR2, CDR2, FR3, CDR3, FR4. The constant regions of the heavy chain and light chain are not involved in antigen binding, but have various effector functions. Antibodies can be divided into several classes based on the amino acid sequences of the heavy chain constant regions. Antibodies can be divided into five major classes or isotypes based on the presence of α, δ, ε, γ and µ heavy chains, and they are IgA, IgD, IgE, IgG and IgM, respectively. Several of the major antibody classes can also be divided into subclasses, such as IgG1 (γ1 heavy chain), IgG2 (γ2 heavy chain), IgG3 (γ3 heavy chain), IgG4 (y4 heavy chain), IgA1 (α1 heavy chain), or IgA2 (α2 heavy chain).

The term "variable domain" as used herein with respect to an antibody refers to an antibody variable region or a fragment thereof comprising one or more CDRs. Although a variable domain may comprise an intact variable region (e.g., HCVR or LCVR), it may also comprise less than an intact variable region yet still retains the ability to bind to an antigen or form an antigen-binding site.

The term "antigen-binding portion" as used herein refers to an antibody fragment formed from a portion of an antibody comprising one or more CDRs or any other antibody fragment that binds to an antigen but does not comprise an intact antibody structure. Examples of antigen-binding portions include, but are not limited to, variable domains, variable regions, diabodies, Fabs, Fab', F(ab')₂, Fv fragments, disulfide-stabilized Fv fragments (dsFv), (dsFv)₂, bispecific dsFv (dsFv-dsFv'), disulfide-stabilized diabodies (ds diabodies), multispecific antibodies, camelized single domain antibodies, nanobodies, domain antibodies, and bivalent domain antibodies. The antigen-binding portion can bind to the same antigen as the parent antibody. In certain embodiments, the antigen-binding portion can comprise one or more CDRs from a particular human antibody grafted to a framework region from one or more different human antibodies. More detailed formats of antigen-binding portions are described in Spiess et al., 2015 (supra) and Brinkman et al., mAbs, 9(2), pp.182-212 (2017). "Fab" of an antibody refers to the portion of the antibody consisting of a light chain (comprising a variable region and a constant region) joined to the variable region and first constant region of a heavy chain by a disulfide bond. In certain embodiments, the constant regions of both the light chain and heavy chain are replaced with TCR constant regions.

"Fab'" refers to a Fab fragment that comprises a portion of the hinge region.

"F(ab')₂" refers to a dimer of Fab.

The "fragment difficult (Fd)" of an antibody refers to the amino-terminal half of a heavy chain fragment that can be combined with a light chain to form a Fab.

The "Fc" of an antibody refers to the portion of an antibody consisting of the second constant region (CH2) and third constant region (CH3) of a first heavy chain joined to the second constant region and third constant region of a second heavy chain by a disulfide bond. The Fc portion of an antibody is responsible for a variety of different effector functions, such as ADCC and CDC, but is not involved in antigen-binding.

The "hinge region" in terms of an antibody comprises the portion of a heavy chain molecule that joins the CH1 domain to the CH2 domain. This hinge region comprises about 25 amino acid residues and is flexible, thereby allowing the two N-terminal antigen-binding regions to move independently.

The term "CH2 domain" as used herein refers to a portion of a heavy chain molecule that extends, e.g., from about amino acid 244 to amino acid 360 of an IgG antibody, wherein the amino acids are numbered using conventional numbering schemes (amino acids 244 to 360, the Kabat numbering system; and amino acids 231-340, the EU numbering system; see Kabat, E. et al., U.S. Department of Health and Human Services (1983)).

The "CH3 domain" extends from the CH2 domain to the C-terminus of an IgG molecule and comprises about 108 amino acids. Certain immunoglobulin classes, such as IgM, further comprise a CH4 region.

"Fv" of an antibody refers to the smallest fragment of an antibody that contains an intact antigen-binding site. The Fv fragment consists of the variable region of one light chain joined to the variable region of one heavy chain. Several Fv designs are provided, including dsFvs, in which the linkage between two domains is enhanced by an introduced disulfide bond; and scFvs can be formed by forming a single polypeptide by joining two domains together with a peptide linker. Fv constructs comprising a variable domain of a heavy or light immunoglobulin chain linked to the variable domain and constant domain of the corresponding immunoglobulin heavy chain or light chain have been produced. Fvs have also been multimerized to form diabodies and triabodies (Maynard et al., Annu Rev Biomed Eng 2 339-376 (2000)).

"ScFab" refers to a fusion polypeptide having a Fd linked to a light chain by a polypeptide linker, resulting in a single-chain Fab fragment (scFab).

"TriFab" refers to a trivalent, bispecific fusion protein consisting of 3 units with Fab functions. TriFabs harbor two common Fabs fused to an asymmetric Fab-like portion.

"Fab-Fab" refers to a fusion protein formed by fusing the Fd chain of a first Fab arm to the N-terminus of the Fd chain of a second Fab arm.

"Fab-Fv" refers to a fusion protein formed by fusing an HCVR to the C-terminus of the Fd chain and an LCVR to the C-terminus of the light chain. "Fab-dsFv" molecules can be formed by introducing an interdomain disulfide bond between the HCVR domain and the LCVR domain.

"MAb-Fv" or "IgG-Fv" refers to fusion proteins formed by fusing an HCVR domain to the C-terminus of one Fc chain and separately expressing an LCVR domain or fusing it to the C-terminus of another Fc chain, and thus a bispecific, trivalent IgG-Fv (mAb-Fv) fusion protein whose Fv is stabilized by an interdomain disulfide bond is produced.

"ScFab-Fc-scFv₂" and "ScFab-Fc-scFv" refer to fusion proteins formed by fusing a single-chain Fab with a Fc and a disulfide-stabilized Fv domain.

"Appended IgG" refers to a fusion protein in which a Fab arm is fused to an IgG to form a format of bispecific (Fab)₂-Fc. It can form a "IgG-Fab" or "Fab-IgG" in which a Fab is fused to the C-terminus or N-terminus of an IgG molecule, whether or not a linker is present. In certain embodiments, the appended IgG can be further modified to a format of IgG-Fab₄ (see Brinkman et al., 2017, supra).

"DVD-Ig" refers to a dual-variable-domain antibody, which is formed by fusing an additional HCVR domain and LCVR domain of a second specificity to an IgG heavy chain and light chain. "CODV-Ig" refers to a related format in which two HCVR domains and two LCVR domains are linked in a way that allows crossover pairing of the variable HCVR-LCVR domains, which are arranged (from N-terminus to C-terminus) in the order HCVRA-HCVRB and LCVRB-LCVRA, or in the order HCVRB-HCVRA and LCVRA-LCVRB.

"CrossMab" refers to a technique of pairing an unmodified light chain with a corresponding unmodified heavy chain and pairing a modified light chain with a corresponding modified heavy chain to produce an antibody with reduced mispairing in the light chain.

"BiTE" is a bispecific T-cell engager molecule comprising a first scFv with a first antigen specificity in the LCVR-HCVR orientation linked to a second scFv with a second antigen specificity in the HCVR-LCVR orientation.

"WuXiBody" is a bispecific antibody comprising a soluble chimeric protein with the variable domains of an antibody and the constant domains of TCR, wherein the subunits (e.g., the α domain and β domain) of the constant domains of TCR are linked by an engineered disulfide bond.

"Percent sequence identity" with respect to an amino acid sequence (or nucleic acid sequence) refers to the percentage of amino acid (or nucleic acid) residues in a candidate sequence that are identical to the amino acid (or nucleic acid) residues in a reference sequence, in a sequence alignment in which gaps, if necessary, are introduced to achieve the maximum number of amino acids (or nucleic acids). Conservative substitutions of the amino acid residues may or may not be considered identical residues. The percent sequence identity of amino acid (or nucleic acid) sequences can be determined by producing a sequence alignment using tools disclosed in the art, such as BLASTN, BLASTp (U.S. National Center for Biotechnology Information (NCBI), see also Altschul S.F. et al., J. Mol. Biol., 215:403-410 (1990); Stephen F. et al., Nucleic Acids Res., 25:3389-3402 (1997)), ClustalW2 (available on the website of European Bioinformatics Institute, see also Higgins D.G. et al., Methods in Enzymology, 266:383-402 (1996); Larkin M.A. et al., Bioinformatics (Oxford, England), 23(21):2947-8 (2007)) and ALIGN or Megalign (DNASTAR) software. Those skilled in the art can use default parameters provided by the tools or can adjust the parameters appropriately as needed for alignment, e.g., by selecting a suitable algorithm.

"Antigen" or "Ag" as used herein refers to a compound, composition, peptide, polypeptide, protein or substance that can stimulate the production of antibodies or T cell responses in cell culture or in an animal, including compositions (such as one comprising cancer-specific proteins) that are added to cell culture (such as hybridoma), or injected or absorbed into an animal. An antigen reacts with the products of specific humoral or cellular immunity (e.g., an antibody, including products induced by heterologous antigens).

"Epitope" or "antigenic determinant" refers to the region of an antigen to which a binding agent (e.g., an antibody) binds. Epitopes can be formed from contiguous amino acids (also known as linear or sequential epitopes) or non-contiguous amino acids juxtaposed by tertiary folding of a protein (also known as configurational or conformational epitopes). Epitopes formed from contiguous amino acids are typically arranged linearly along the primary amino acid residues on the protein, and small segments of contiguous amino acids can be digested by antigens binding to major histocompatibility complex (MHC) molecules or retained upon exposure to denaturing solvents, whereas epitopes formed by tertiary folding are typically lost upon treatment with denaturing solvents. Epitopes typically comprise at least 3, more typically at least 5, about 7, or about 8-10 amino acids in a unique spatial conformation.

"Specific binding" or "specifically bind to" as used herein refers to a non-random binding reaction between two molecules, such as a reaction between an antibody and an antigen. In certain embodiments, the bispecific anti-CD3/CD20 polypeptide complex provided herein specifically binds to an antigen with a binding affinity (K_{D}) of ≤10⁻⁶ M (e.g., ≤5 × 10⁻⁷ M, ≤2 × 10⁻⁷ M, ≤10⁻⁷ M, ≤5 × 10⁻⁸ M, ≤2 × 10⁻⁸ M, ≤10⁻³ M, ≤5 × 10⁻⁹ M, <2 × 10⁻⁹ M, ≤10⁻⁹ M or ≤10⁻¹⁰ M). K_{D} as used herein refers to the ratio of the dissociation rate to the association rate (k_{off}/kₒₙ), which can be determined by using a surface plasmon resonance method, for example, using an instrument such as Biacore.

The term "operably link" or "operably linked" refers to the juxtaposition of two or more biological sequences of interest in such a way that they are in a relationship permitting them to function in their intended manner, whether or not a spacer or linker is present. When used with respect to polypeptides, the term is intended to indicate that the polypeptide sequences are linked in such a way that the product of the linkage has the intended biological function. For example, an antibody variable region can be operably linked to a constant region to form a stable product with antigen-binding activity. The term can also be used with respect to polynucleotides. For example, when a polynucleotide encoding a polypeptide is operably linked to a regulatory sequence (e.g., a promoter, enhancer or silencer sequence), the term is intended to mean that the polynucleotide sequence is linked in a way that allows for regulated expression of the polypeptide from the polynucleotide.

The term "fusion" or "fused" when used with respect to amino acid sequences (e.g., peptides, polypeptides or proteins) refers to the combination of two or more amino acid sequences into a non-naturally-occurring single amino acid sequence, e.g., by chemical bonding or recombinant means. The fusion amino acid sequence can be produced by the recombination of two genes encoding polynucleotide sequences, and can be expressed by introducing a construct containing the recombinant polynucleotide into a host cell.

The term "spacer" as used herein refers to an artificial amino acid sequence of 1, 2, 3, 4 or 5 amino acid residues, or 5 to 15, 20, 30, 50 or more amino acid residues that are linked by peptide bonds and used to link one or more polypeptides. The spacer may or may not have a secondary structure. Spacer sequences are well known in the art, see, e.g., Holliger et al., Proc. Natl. Acad. Sci. USA 90:6444-6448 (1993); and Poljak et al., Structure 2:1121-1123

(1994). Any suitable spacer known in the art can be used.

The term "antigen specificity" refers to a particular antigen or an epitope thereof that is selectively recognized by an antigen-binding molecule.

The term "substitution" as used herein with respect to amino acid residues refers to the substitution of one or more naturally occurring or introduced amino acids with another in a peptide, polypeptide or protein. Substitutions in a polypeptide can result in diminishment, enhancement or elimination of the function of the polypeptide.

A substitution can also be a "conservative substitution", which when used with respect to an amino acid sequence refers to the substitution of one amino acid residue with another different one that has a side chain with similar physicochemical properties, or the substitution of those amino acids which are not critical to the activity of the polypeptide. For example, conservative substitutions can be made between non-polar side chain amino acid residues (e.g., Met, Ala, Val, Leu and Ile, Pro, Phe and Trp), between uncharged polar side chain residues (e.g., Cys, Ser, Thr, Asn, Gly and Gln), between acidic side chain residues (e.g., Asp and Glu), between basic side chain amino acids (e.g., His, Lys and Arg), between β-branched side chain amino acids (e.g., Thr, Val and Ile), between sulfur-containing side chain amino acids (e.g., Cys and Met), or between aromatic side chain residues (e.g., Trp, Tyr, His and Phe). In certain embodiments, a substitution, a deletion or an addition can also be considered a "conservative substitution". The number of amino acids inserted or deleted can range from about 1 to 5. Conservative substitutions generally do not cause significant changes in the conformational structure of a protein, and therefore the biological activity of the protein can be retained.

The term "mutation" or "mutated" as used herein for an amino acid residue refers to a substitution, insertion or addition of an amino acid residue.

The term "T cell" as used herein refers to a class of lymphocytes that play a critical role in cell-mediated immunity, including helper T cells (e.g., CD4⁺ T cells, T helper type 1 T cells, T helper type 2 T cells, T helper type 3 T cells and T helper type 17 T cells), cytotoxic T cells (e.g., CD8⁺ T cells), memory T cells (e.g., central memory T cells (TCM cells), effector memory T cells (TEM cells and TEMRA cells) and resident memory T cells (TRM), which are CD8⁺ or CD4⁺), natural killer T (NKT) cells, and suppressor T cells.

A native "T cell receptor" or native "TCR" is a heterodimeric T cell surface protein that binds to an invariant CD3 chain to form a complex capable of mediating signal transduction. TCR is a member of the immunoglobulin superfamily and is similar to a half antibody with a single heavy chain and a single light chain. A native TCR has an extracellular portion, a transmembrane portion and an intracellular portion. The extracellular domain of TCR has a membrane-proximal constant region and a membrane-distal variable region.

The term "subject" or "individual" or "animal" or "patient" as used herein refers to a human or non-human animal, including mammals or primates, in need of diagnosis, prognosis, amelioration, prevention and/or treatment of a disease or disorder. Mammalian subjects include humans, domestic animals, farm animals, and zoo, sports or pet animals, such as dogs, cats, guinea pigs, rabbits, rats, mice, horses, pigs, cattle and bears.

"Treatment" or "therapy" includes preventing or alleviating a certain condition, reducing the rate at which a certain condition arises or develops, reducing the risk of developing a certain condition, preventing or delaying the development of symptoms related to a certain condition, reducing or terminating symptoms related to a certain condition, producing a complete or partial reversal of a certain condition, curing a certain condition, or a combination thereof. With respect to cancer, "treatment" or "therapy" can refer to inhibiting or slowing the growth, proliferation or metastasis of neoplastic or malignant cells, preventing or delaying the development of neoplastic or malignant cell growth, proliferation or metastasis, or some combinations thereof. With respect to a tumor, "treatment" or "therapy" includes removing all or part of the tumor, inhibiting or slowing tumor growth and metastasis, preventing or delaying the development of the tumor, or some combinations thereof.

### Bispecific Anti-CD3/CD20 Polypeptide Complex

The term "bispecific" as used herein means that there are two antigen-binding portions, each of which is capable of specifically binding to a different antigen or to a different epitope on the same antigen. The bispecific anti-CD3/CD20 polypeptide complex provided herein comprises a first antigen-binding portion associated with a second antigen-binding portion, and one of them specifically binds to CD3 and the other one specifically binds to CD20. In other words, the first antigen-binding portion can specifically bind to CD3, and the second antigen-binding portion can specifically bind to CD20. Alternatively, the first antigen-binding portion can specifically bind to CD20, and the second antigen-binding portion can specifically bind to CD3. In the present disclosure, the terms "bispecific anti-CD3xCD20 polypeptide complex" is used interchangeably with "polypeptide complex targeting CD3 and CD20" or "anti-CD3 and CD20 polypeptide complex" or "bispecific anti-CD3/CD20 polypeptide complex".

In certain embodiments, the present disclosure provides a bispecific anti-CD3/CD20 polypeptide complex comprising a first antigen-binding portion associated with a second antigen-binding portion, wherein:
the first antigen-binding portion comprises:
a first polypeptide, comprising, from N-terminus to C-terminus, a first heavy chain variable domain (VH) of a first antibody operably linked to a first T Cell Receptor (TCR) constant region (C1), and
a second polypeptide, comprising, from N-terminus to C-terminus, a first light chain variable domain (VL) of the first antibody operably linked to a second TCR constant region (C2),
wherein C1 and C2 can form a dimer comprising at least one non-native interchain bond between C1 and C2, and
the non-native interchain bond can stabilize the dimer, and
the second antigen-binding portion comprises:
   a second heavy chain variable domain (VH2) of a second antibody operably linked to an antibody heavy chain CH1 domain, and
   a second light chain variable domain (VL2) of the second antibody operably linked to an antibody light chain constant (CL) domain,
   wherein one of the first antigen-binding portion and the second antigen-binding portion is an anti-CD3 binding portion, and the other one is an anti-CD20 binding portion;
   the anti-CD3 binding portion is derived from an anti-CD3 antibody, comprising:
      a) heavy chain CDR1 comprising an amino acid sequence selected from the group consisting of SEQ ID NOs: 1 and 13,
      b) heavy chain CDR2 comprising an amino acid sequence selected from the group consisting of SEQ ID NOs: 2 and 14,
      c) heavy chain CDR3 comprising an amino acid sequence selected from the group consisting of SEQ ID NOs: 3 and 15,
      d) κ light chain CDR1 comprising an amino acid sequence selected from the group consisting of SEQ ID NOs: 4 and 16,
      e) κ light chain CDR2 comprising an amino acid sequence selected from the group consisting of SEQ ID NOs: 5 and 17, and
      f) κ light chain CDR3 comprising an amino acid sequence selected from the group consisting of SEQ ID NOs: 6 and 18,
   the anti-CD20 binding portion is derived from an anti-CD20 antibody, comprising:
      a) heavy chain CDR1 comprising an amino acid sequence selected from the group consisting of SEQ ID NOs: 7 and 19,
      b) heavy chain CDR2 comprising an amino acid sequence selected from the group consisting of SEQ ID NOs: 8 and 20,
      c) heavy chain CDR3 comprising an amino acid sequence selected from the group consisting of SEQ ID NOs: 9 and 21,
      d) κ light chain CDR1 comprising an amino acid sequence selected from the group consisting of SEQ ID NOs: 10 and 22,
      e) κ light chain CDR2 comprising an amino acid sequence selected from the group consisting of SEQ ID NOs: 11 and 23, and
      f) κ light chain CDR3 comprising an amino acid sequence selected from the group consisting of SEQ ID NOs: 12 and 24.

In certain embodiments, the bispecific anti-CD3/CD20 polypeptide complex provided herein comprises a first antigen-binding portion comprising a sequence derived from the constant region of TCR, but the second antigen-binding portion does not comprise a sequence derived from the constant region of TCR.

The bispecific anti-CD3/CD20 polypeptide complex provided herein is significantly less likely to have mispaired heavy chain and light chain variable domains. Without wishing to be bound by any theory, it is believed that the stable TCR constant regions in the first antigen-binding portion can be specifically linked to each other and thus contribute to the highly specific pairing of the target VH1 and VL1, while discouraging unwanted mispairing of VH1 or VL1 with other variable regions that do not provide a target antigen-binding site.

In certain embodiments, the second antigen-binding portion further comprises an antibody constant CH1 domain operably linked to VH2, and an antibody light chain constant domain operably linked to VL2. Thus, the second antigen-binding portion comprises a Fab.

When the first, second, third and fourth variable domains (e.g., VH1, VH2, VL1 and VL2) are expressed in one cell, it is highly desired that VH1 is specifically paired with VL1 and VH2 with VL2, such that the resulting bispecific protein product would have the correct antigen-binding specificity. However, in the prior art, such as hybrid-hybridoma (or quadroma), random pairing of VH1, VH2, VL1 and VL2 occurs, resulting in the generation of up to 10 different molecules, only one of which is a functional bispecific antigen-binding molecule. This not only reduces the yield, but also complicates the purification of the target product.

The bispecific anti-CD3/CD20 polypeptide complex provided herein is exceptional in that the variable domains are less likely to be mispaired, compared to the case where the first antigen-binding portion and the second antigen-binding portion are both counterparts of native Fabs. In an illustrative example, the first antigen-binding domain comprises VH1-C1 paired with VL1-C2, and the second antigen-binding domain comprises VH2-CH1 paired with VL2-CL. It has been surprisingly found that C1 and C2 preferentially associate with each other, and are less likely to be associated with CL or CH1, thereby the formation of unwanted pairings such as C1-CH, C1-CL, C2-CH and C2-CL is discouraged and significantly reduced. As a result of specific association of C1-C2, VH1 specifically pairs with VL1 and thereby forms the first antigen-binding site, and CH1 specifically pairs with CL, thereby allowing specific pairing of VH2-VL2 that provides the second antigen-binding site. Accordingly, the first antigen-binding portion and the second antigen-binding portion are less likely to be mispaired, and mispairing between, for example, VH1-VL2, VH2-VL1, VH1-VH2 and VL1-VL2 is significantly reduced compared to the case where the first antigen-binding portion and the second antigen-binding portion are both counterparts of native Fabs (e.g. in the form of VH1-CH1, VL1-CL, VH2-CH1 and VL2-CL).

In certain embodiments, the bispecific anti-CD3/CD20 polypeptide complex provided herein has significantly fewer mispairing products (e.g., a decrease of at least 1, 2, 3, 4, 5 or more mispairing products) and/or a significantly higher yield (e.g., an increase of at least 10%, 20%, 30%, 40%, 50%, 60% or more in yield) when expressed from a cell compared to a reference molecule expressed under comparable conditions, wherein the reference molecule is identical to the bispecific anti-CD3/CD20 polypeptide complex except that C1 is substituted with native CH1 and C2 with native CL.

### Antigen-Binding Portion Comprising Engineered Cα and Cβ

The first antigen-binding portion provided herein comprises a first antibody heavy chain variable domain operably linked to a first T cell receptor (TCR) constant region, and a first antibody light chain variable domain operably linked to a second TCR constant region, wherein the first TCR constant region and the second TCR constant region are associated via at least one non-native interchain disulfide bond. The first antigen-binding portion comprises at least two polypeptide chains, each of which comprises a variable domain derived from an antibody and a constant region derived from TCR. Thus, the first antigen-binding portion comprises a heavy chain variable domain and a light chain variable domain, each of which is operably linked to a pair of TCR constant regions. In certain embodiments, the pair of TCR constant regions in the first antigen-binding portion is α/β TCR constant regions. The TCR constant regions of the bispecific anti-CD3/CD20 polypeptide complex provided herein can associate with each other to form a dimer through at least one non-native disulfide bond.

It has been surprisingly found that the first antigen-binding portion with at least one non-native disulfide bond provided herein can be recombinantly expressed and assembled into a desired conformation that stabilizes the TCR constant region dimer and provides good antigen-binding activity of antibody variable regions. In addition, the first antigen-binding portion is found to well tolerate routine antibody engineering, e.g., modification of glycosylation sites and removal of some natural sequences. In addition, the bispecific anti-CD3/CD20 polypeptide complex provided herein can be incorporated into a bispecific format, which can be readily expressed and assembled with minimal or substantially no mispairing of antigen-binding sequences due to the presence of the TCR constant regions in the first antigen-binding portion. Other advantages of the first antigen-binding portion and the construct provided herein will be more apparent in the disclosure below.

In summary, the present disclosure provides a first antigen-binding portion comprising a first polypeptide and a second polypeptide, wherein the first polypeptide comprises, from N-terminus to C-terminus, a first heavy chain variable domain (VH) of a first antibody operably linked to a first T cell receptor (TCR) constant region (C1), and the second polypeptide comprises from N-terminus to C-terminus a first light chain variable domain (VL) of the first antibody operably linked to a second TCR constant region (C2), wherein C1 and C2 are capable of forming a dimer, and the non-native interchain disulfide bond between C1 and C2 is capable of stabilizing the dimer.

### TCR Constant Region

The first antigen-binding portion described herein comprises an α or β constant region derived from TCR.

The human TCR α chain constant region is known as TRAC, under NCBI accession No. P01848.

There are two different variants of the human TCR β chain constant region, known as TRBC1 and TRBC2 (IMGT nomenclature) (Toyonaga B et al., PNAs, Vol. 82, pp.8624-8628, Immunology (1985)).

In the present disclosure, the first TCR constant region and the second TCR constant region of the first antigen-binding portion provided herein are capable of forming a dimer, which comprises, between the TCR constant regions, at least one non-native interchain disulfide bond capable of stabilizing the dimer.

The term "dimer" as used herein refers to a structure of two molecules (e.g., polypeptides or proteins) that are associated via covalent or non-covalent interactions. A homodimer or homodimerization is formed from two identical molecules, whereas a heterodimer or heterodimerization is formed from two different molecules. The dimer formed from the first TCR constant region and the second TCR constant region is a heterodimer.

A "mutated" amino acid residue refers to one that is substituted, inserted or added and that is different from the corresponding native residue in its corresponding native TCR constant region. For example, if an amino acid residue at a particular position in a wild-type TCR constant region is referred to as a "native" residue, its corresponding mutated residue is any residue that is different from the native residue but resides at the same position on the TCR constant region. The mutated residue can be a different residue that substitutes for the native residue at the same position, or that is inserted before the native residue and therefore takes the native residue's original place.

In the bispecific anti-CD3/CD20 polypeptide complex provided herein, the first TCR constant region and/or the second TCR constant region have been engineered to comprise one or more mutated amino acid residues responsible for forming the non-native interchain disulfide bond. To introduce such a mutated residue into the TCR constant region, a coding sequence of a TCR region can be manipulated, for example, to replace a codon encoding a native residue with a codon encoding the mutated residue, or to insert a codon encoding the mutated residue before the codon of the native residue.

In the bispecific anti-CD3/CD20 polypeptide complex provided herein, the first TCR constant region and/or the second TCR constant region have been engineered to comprise one or more mutated cysteine residues such that, upon substitution of the cysteine residues, a non-nativenative interchain disulfide bond can be formed between the two TCR constant regions.

The non-native interchain disulfide bond is capable of stabilizing the first antigen-binding portion. Such effects in stabilization can be embodied in various ways. For example, the presence of the mutated amino acid residues or the non-native interchain disulfide bond can enable the bispecific anti-CD3/CD20 polypeptide complex to be stably expressed, and/or to be expressed at high levels, and/or to be associated into a stable complex with desirable biologic activity (e.g., antigen-binding activity), and/or to be expressed and assembled into a desired high-level stable complex with the desired biological activity. The ability of the interchain disulfide bond to stabilize the first TCR constant region and the second TCR constant region can be assessed using suitable methods known in the art (such as displaying molecular weight on SDS-PAGE, or determining thermostability by differential scanning calorimetry (DSC) or differential scanning fluorimetry (DSF)). In an illustrative example, the formation of the stable first antigen-binding portion provided herein can be confirmed by SDS-PAGE if the product shows a molecular weight comparable to the combined molecular weight of the first polypeptide and the second polypeptide. In certain embodiments, the first antigen-binding portion disclosed herein is stable in that its thermostability is no less than 50%, 60%, 70%, 80% or 90% of that of a native Fab. In certain embodiments, the first antigen-binding portion disclosed herein is stable in that its thermostability can compare to that of a native Fab.

Without wishing to be bound by any theory, it is believed that the unnative interchain disulfide bond formed between the first TCR constant region and the second TCR constant region in the first antigen-binding portion is capable of stabilizing a heterodimer of the TCR constant regions, thereby enhancing the level of correct folding, the structural stability, and/or the expression level of the heterodimer and of the first antigen-binding portion. Unlike the native TCR anchored on the cell membrane of the surface of a T cell, heterodimers of the extracellular domain of native TCR are found to be less stable, although they are similar in 3D structure to antibody Fab. As a matter of fact, the instability of dissolved native TCR used to be a significant impediment that made its crystal structure difficult to elucidate (see Wang, Protein Cell, 5(9), pp.649-652 (2014)). By introducing a pair of cysteine (Cys) mutations into the TCR constant regions and thereby enabling the formation of an interchain non-native disulfide bond, the first antigen-binding portion can be stably expressed with the antigen-binding ability of the antibody variable region retained.

TCR constant regions comprising mutated residues are also referred to herein as "engineered" TCR constant regions. In certain embodiments, the first TCR constant region (C1) of the bispecific anti-CD3/CD20 polypeptide complex comprises an engineered TCR α chain (Cα), and the second TCR constant region (C2) comprises an engineered TCR β chain (Cβ). In the bispecific anti-CD3/CD20 polypeptide complex provided herein, C1 comprises an engineered Cβ, and C2 comprises an engineered Cα.

In the bispecific anti-CD3/CD20 polypeptide complex provided herein, the engineered TCR constant regions comprise one or more mutated cysteine residues comprised within a contact interface of the first engineered constant region and/or the second engineered constant region.

The term "contact interface" as used herein refers to a particular region on the polypeptides where the polypeptides interact/associate with each other. A contact interface comprises one or more amino acid residues that are capable of interacting with the corresponding amino acid residues in contact or association when interaction occurs. The amino acid residues in a contact interface may or may not be in a continuous sequence. For example, when the interface is three-dimensional, the amino acid residues within the interface can be separated from each other at different positions on the linear sequence.

In certain embodiments, one or more disulfide bonds can be formed between the engineered Cα and the engineered Cβ. In certain embodiments, the pairing of cysteine residues can form a non-native interchain disulfide bond.

As used throughout the present disclosure, "XnY" with respect to a TCR constant region is intended to mean that the n^{th} amino acid residue X on the TCR constant region is substituted with amino acid residue Y, wherein X and Y are single-letter abbreviations for particular amino acid residues.

In the bispecific anti-CD3/CD20 polypeptide complex provided herein, the engineered Cβ comprises or is SEQ ID NO: 61, and the engineered Cα comprises or is SEQ ID NO: 62.

The sequences of SEQ ID NO: 61 and SEQ ID NO: 62 are provided below:

LEDLKNVFPPEVAVFEPSEAEISHTQKATLVCLATGFYPDHVELSWWVNGKEVHSGVCTDPQPLKEQPAL
QDSRYALSSRLRVSATFWQNPRNHFRCQVQFYGLSENDEWTQDRAKPVTQIVSAEA (SEQ ID NO: 61)
PDIQNPDPAVYQLRDSKSSDKSVCLFTDFDSQTQVSQSKDSDVYITDKCVLDMRSMDFKSNSAVAWSQKS
DFACANAFQNSIIPEDTFFPSPESS (SEQ ID NO: 62)

In the bispecific anti-CD3/CD20 polypeptide complex provided herein, one or more native glycosylation sites present in native TCR constant regions can be modified (e.g., removed) in the first antigen-binding portion provided herein. The term "glycosylation site" as used herein with respect to a polypeptide sequence refers to an amino acid residue with a side chain to which a carbohydrate portion (e.g., an oligosaccharide structure) can be attached. Glycosylation of polypeptides (such as antibodies) is typically N-linked or O-linked. N-linked refers to the attachment of a carbohydrate portion to the side chain of an asparagine residue, for example, an asparagine residue in a tripeptide sequence such as asparagine-X-serine and asparagine-X-threonine, wherein X is any amino acid except proline. O-linked glycosylation refers to the attachment of one of N-acetylgalactosamine, galactose or xylose to a hydroxyamino acid, most commonly to serine or threonine. Removal of native glycosylation sites can be conveniently accomplished by altering the amino acid sequence such that one or more of the tripeptide sequences (for N-linked glycosylation sites) described above or one or more of serine or threonine residues (for O-linked glycosylation sites) present in the sequence are substituted.

In the first antigen-binding portion provided herein, at least one native glycosylation site is absent from the engineered TCR constant regions (e.g., in the first TCR constant region and/or the second TCR constant region). Without wishing to be bound by any theory, it is believed that the first antigen-binding portion disclosed herein can tolerate removal of all or some of the glycosylation sites without affecting protein expression and stability, in contrast to the existing teaching that the presence of an N-linked glycosylation site on a TCR constant region such as Cα (i.e., N34, N68 and N79) and Cβ (i.e., N69) is necessary for protein expression and stability (see Wu et al., Mabs, 7:2, 364-376, 2015).

In the first antigen-binding portion provided herein, a constant region derived from TCR is operably linked to a variable region derived from an antibody.

In certain embodiments, the first antibody variable domain (VH) is fused to the first TCR constant region (C1) at a first junction domain, and the first antibody variable domain (VL) is fused to the second TCR constant region (C2) at a second junction domain.

The term "junction domain" as used herein refers to a boundary or border region where two amino acid sequences are fused or combined. In certain embodiments, the first junction domain comprises at least a portion of a C-terminal fragment of an antibody V/C junction, and the second junction domain comprises at least a portion of an N-terminal fragment of a TCR V/C junction.

The term "antibody V/C junction" as used herein refers to a junction of an antibody variable domain and a constant domain, for example, a junction of a heavy chain variable domain and a CH1 domain or a junction of a light chain variable domain and a light chain constant domain. Similarly, the term "TCR V/C junction" refers to a junction of a TCR variable domain and a constant domain, for example, a junction of a TCR α variable domain and a constant domain or a junction of a TCR β variable domain and a constant domain.

In certain embodiments, the first polypeptide comprises a sequence comprising a domain operably linked as in formula (I): VH-HCJ-C1, and the second polypeptide comprises a sequence comprising a domain operably linked as in formula (II): VL-LCJ-C2, wherein:
VH is a heavy chain variable domain of an antibody; HCJ is a first binding domain as defined above; C1 is a first TCR constant domain as defined above; VL is a light chain variable domain of an antibody; LCJ is a second junction domain as defined above; C2 is a second TCR constant domain as defined above.

### Antibody Variable Region

The bispecific anti-CD3/CD20 polypeptide complex provided herein comprises a first antigen-binding portion associated with a second antigen-binding portion, and one of them specifically binds to CD3 and the other to CD20. In the bispecific anti-CD3/CD20 polypeptide complex provided herein, the first antigen-binding portion comprises a first heavy chain variable domain (VH1) and a first light chain variable domain (VL1) of a first antibody, and the second antigen-binding portion comprises a second heavy chain variable domain (VH2) and a second light chain variable domain (VL2) of a second antibody, wherein the first antibody and the second antibody are different and are selected from the group consisting of an anti-CD3 antibody and an anti-CD20 antibody. In certain embodiments, the first antibody is an anti-CD3 antibody and the second antibody is an anti-CD20 antibody. In certain other embodiments, the first antibody is an anti-CD20 antibody and the second antibody is an anti-CD3 antibody.

In a conventional native antibody, a variable region comprises three CDRs separated by flanking framework regions (FRs), for example, as set forth in the following formula: FR1-CDR1-FR2-CDR2-FR3-CDR3-FR4, from N-terminus to C-terminus.

### a) Anti-CD3 binding portion

In the bispecific anti-CD3/CD20 polypeptide complex provided herein, the first antigen-binding portion or the second antigen-binding portion is an anti-CD3 binding portion.

In certain embodiments, the anti-CD3 binding portion is derived from the antibodies W3278-T2U3.E17R-1.uIgG4.SP and W3278-U3T2.F18R-1.ulgG4.SP shown in Table A below. The CDR sequences of the anti-CD3 binding portion of the W3278-T2U3.E17R-1.uIgG4.SP and W3278-U3T2.F18R-1.uIgG4.SP antibodies are provided below.

**Table A**

| Antibody ID: | | CDR1 | CDR2 | CDR3 |
|---|---|---|---|---|
| W3278-T2U3.E17R-1. uIgG4.SP, W3278-U3T2.F18R-1. uIgG4.SP | VH | SEQ ID NO: 1 | SEQ ID NO: 2 | SEQ ID NO: 3 |
| | | GYSFTTYYIH | WIFPGNDNIKYSEK FKG | DSVSIYYFDY |
| | | SEQ ID NO: 4 | SEQ ID NO: 5 | SEQ ID NO: 6 |
| | Vκ | KSSQSLLNSRTRKNY LA | WASTRKS | TQSFILRT |

The heavy chain and κ light chain variable region sequences of the anti-CD3 binding portions of the W3278-T2U3.E17R-1.uIgG4.SP and W3278-U3T2.F18R-1.uIgG4.SP antibodies are provided below.
VH-amino acid sequence (SEQ ID NO: 25): 
QVQLVQSGAEVKKPGSSVKVSCKASGYSFTTYYIHWVRQAPGQGLEWMGWIFPGNDNIKYSEKFKGRV
TITADKSTSTAYMELSSLRSEDTAVYYCAIDSVSIYYFDYWGQGTLVTVSS
VH-nucleic acid sequence (SEQ ID NO: 53): 
caggtgcaactcgtgcagtctggagctgaagtgaagaagcctgggtcttcagtcaaggtcagttgcaaggccagtgggtattccttcactacctactacatccactggg
tgcggcaggcaccaggacaggggcttgagtggatgggctggatctttcccggcaacgataatattaagtacagcgagaagttcaaagggagggtcaccattaccgc
cgacaaatccacttccacagcctacatggagttgagcagcctgagatccgaggatacagccgtgtactactgtgccattgacagcgtgtccatctactactttgactact
ggggccagggcacactggtcacagtgagcagc
Vκ-amino acid sequence (SEQ ID NO: 26): 
DIVMTQSPDSLAVSLGERATINCKSSQSLLNSRTRKNYLAWYQQKPGQPPKLLIYWASTRKSGVPDRFSGS
GSGTDFTLTISSLQAEDVAVYYCTQSFILRTFGGGTKVEIK
Vκ-nucleic acid sequence (SEQ ID NO: 54):

gacatcgtcatgacccagtccccagactctttggcagtgtctctcggggaaagagctaccatcaactgcaagagcagccagtcccttctgaacagcaggaccaggaa
gaattacctcgcctggtaccaacagaagcccggacagcctcctaagctcctgatctactgggcctcaacccggaagagtggagtgcccgatcgctttagcgggagc
ggctccgggacagatttcacactgacaatttcctccctgcaggccgaggacgtcgccgtgtattactgtactcagagcttcattctgcggacatttggcggcgggacta
aagtggagattaag

In some embodiments, the heavy chain variable region sequences of the anti-CD3 binding portions of the W3278-T2U3.E17R-1.uIgG4.SP and W3278-U3T2.F18R-1.uIgG4.SP antibodies are variants of the amino acid sequence of SEQ ID NO: 25, e.g., an amino acid sequence of SEQ ID NO: 25 with C-terminal deletions of amino acids SS.

In certain embodiments, the anti-CD3 binding portion is derived from the antibodies W3278-T3U2.F16-1.uIgG4.SP, W3278-U2T3.F18R-1.uIgG4.SP and W3278-T3U2.F17R-1.uIgG4.SP shown in Table B below. The CDR sequences of the anti-CD3 binding portions of the W3278-T3U2.F16-1.uIgG4.SP, W3278-U2T3.F18R-1.uIgG4.SP and W3278-T3U2.F17R-1.uIgG4.SP antibodies are provided below.

**Table B**

| Antibody ID: | | CDR1 | CDR2 | CDR3 |
|---|---|---|---|---|
| W3278-T3U2.F16-1.uI gG4.SP, W3278-U2T3.F18R-1. uIgG4.SP, W3278-T3U2.F17R-1. uIgG4.SP | VH | SEQ ID NO: 13 | SEQ ID NO: 14 | SEQ ID NO: 15 |
| | | GFAFTDYYIH | WISPGNVNTKYNE NFKG | DGYSLYYFDY |
| | Vκ | SEQ ID NO: 16 | SEQ ID NO: 17 | SEQ ID NO: 18 |
| | | KSSQSLLNSRTRKNYLA | WASTRQS | TQSHTLRT |

The heavy chain and κ light chain variable region sequences of the anti-CD3 binding portions of the W3278-T3U2.F16-1.uIgG4.SP, W3278-U2T3.F18R-1.uIgG4.SP and W3278-T3U2.F17R-1.uIgG4.SP antibodies are provided below.
VH-amino acid sequence (SEQ ID NO: 27): 
QVQLVQSGAEVKKPGSSVKVSCKASGFAFTDYYIHWVRQAPGQGLEWMGWISPGNVNTKYNENFKGR
VTITADKSTSTAYMELSSLRSEDTAVYYCARDGYSLYYFDYWGQGTLVTVSS
VH-nucleic acid sequence (SEQ ID NO: 55): 
caggtgcagcttgtgcagtctggggcagaagtgaagaagcctgggtctagtgtcaaggtgtcatgcaaggctagcgggttcgcctttactgactactacatccactgg
gtgcggcaggctcccggacaagggttggagtggatgggatggatctccccaggcaatgtcaacacaaagtacaacgagaacttcaaaggccgcgtcaccattacc
gccgacaagagcacctccacagcctacatggagctgtccagcctcagaagcgaggacactgccgtctactactgtgccagggatgggtactccctgtattactttgat
tactggggccagggcacactggtgacagtgagctcc
Vκ-amino acid sequence (SEQ ID NO: 28): 
DIVMTQSPDSLAVSLGERATINCKSSQSLLNSRTRKNYLAWYQQKPGQPPKLLIYWASTRQSGVPDRFSGS
GSGTDFTLTISSLQAEDVAVYYCTQSHTLRTFGGGTKVEIK
Vκ-nucleic acid sequence (SEQ ID NO: 56):

gatatcgtgatgacccagagcccagactcccttgctgtctccctcggcgaaagagcaaccatcaactgcaagagctcccaaagcctgctgaactccaggaccagga
agaattacctggcctggtatcagcagaagcccggccagcctcctaagctgctcatctactgggcctccacccggcagtctggggtgcccgatcggtttagtggatctg
ggagcgggacagacttcacattgacaattagctcactgcaggccgaggacgtggccgtctactactgtactcagagccacactctccgcacattcggcggagggact
aaagtggagattaag

In some embodiments, the heavy chain variable region sequences of the anti-CD3 binding portions of the W3278-T3U2.F16-1.uIgG4.SP, W3278-U2T3.F18R-1.uIgG4.SP and W3278-T3U2.F17R-1.uIgG4.SP antibodies are variants of the amino acid sequence of SEQ ID NO: 27, e.g., an amino acid sequence of SEQ ID NO: 27 with C-terminal deletions of amino acids SS.

The anti-CD3 binding portions provided herein also comprise suitable framework region (FR) sequences, so long as the anti-CD3 binding portions can specifically bind to CD3.

The anti-CD3 antibodies of the present disclosure have specific binding affinity for cells expressing CD3 (e.g., CD4⁺ T cells) and can activate human T cells and trigger the release of cytokines TNFα and IFNγ.

The binding affinity of the anti-CD3 binding portions provided herein can be expressed in terms of K_{D} value, which represents a ratio of dissociation rate to association rate (k_{off}/kₒₙ) when the binding between antigen and antigen-binding molecule is in equilibrium. Antigen-binding affinity (e.g., K_{D}) can be suitably determined using suitable methods known in the art, including, for example, flow cytometry assays. In some embodiments, the binding of an antibody to an antigen at different concentrations can be determined by flow cytometry. The determined mean fluorescence intensity (MFI) can be first plotted against antibody concentration, and then a one-site saturation equation Y = Bₘₐₓ × X/(K_{D} + X), where Bₘₐₓ refers to the maximum specific binding of the test antibody to the antigen, can be fit to the dependence of specific binding fluorescence intensity (Y) on antibody concentration (X) by using Prism version 5 (GraphPad Software, San Diego, California), and thereby the K_{D} value is calculated.

In certain embodiments, the anti-CD3 binding portions provided herein are capable of specifically binding to human CD3 or recombinant human CD3 expressed on the surface of a cell. CD3 is a receptor expressed on cells. Recombinant CD3 is soluble CD3 that is recombinantly expressed and is not associated with a cell membrane. Recombinant CD3 can be prepared using a variety of recombinant techniques. In one example, a CD3 DNA sequence encoding the extracellular domain of human CD3 (NP_000724.1) (Met1-Asp126) can be fused to a polyhistidine tag at the C-terminus in an expression vector. Then 293E cells are transfected with the expression vector and allowed to express a product, which is then purified by nickel affinity chromatography.

In some embodiments, the anti-CD3 binding portions provided herein are capable of specifically binding to human CD3 expressed on the surface of a cell with a binding affinity (K_{D}) of no more than 5 × 10⁻⁹ M, no more than 4 × 10⁻⁹ M, no more than 3 × 10⁻⁹ M, no more than 2 × 10⁻⁹ M, no more than 10⁻⁹ M, no more than 5 × 10⁻¹⁰ M, no more than 4×10⁻¹⁰ M, no more than 3 × 10⁻¹⁰ M, no more than 2 × 10⁻¹⁰ M, no more than 10⁻¹⁰ M, no more than 5 × 10⁻¹¹ M or no more than 4 × 10⁻¹¹ M, no more than 3 × 10⁻¹¹ M or no more than 2 × 10⁻¹¹ M or no more than 10⁻¹¹ M; the K_{D} value is determined by flow cytometry.

In certain embodiments, the anti-CD3 binding portions provided herein cross-react with cynomolgus monkey CD3 (e.g., cynomolgus monkey CD3 expressed on the surface of a cell, or soluble recombinant cynomolgus monkey CD3).

The binding of the anti-CD3 binding portions to recombinant CD3 or CD3 expressed on the surface of a cell can be measured using methods well known in the art, such as sandwich methods (e.g., ELISA), Western blots, flow cytometry, and other binding assays. In certain embodiments, the anti-CD3 binding portions provided herein specifically bind to recombinant human CD3 with an EC₅₀ (i.e., 50% binding concentration) of no more than 0.01 nM, no more than 0.02 nM, no more than 0.03 nM, no more than 0.04 nM, no more than 0.05 nM, no more than 0.06 nM, no more than 0.07 nM or no more than 0.08 nM; the EC₅₀ value is determined by ELISA. In certain embodiments, the anti-CD3 binding portions provided herein specifically bind to human CD3 expressed on the surface of a cell with an EC₅₀ of no more than 0.5 nM, no more than 0.6 nM, no more than 0.7 nM, no more than 0.8 nM, no more than 0.9 nM, no more than 1 nM, no more than 2 nM, no more than 3 nM, no more than 4 nM, no more than 5 nM, no more than 6 nM, no more than 7 nM, no more than 8 nM, no more than 9 nM or no more than 10 nM; the EC₅₀ value is determined by flow cytometry.

In certain embodiments, the anti-CD3 binding portions bind to cynomolgus monkey CD3 with a binding affinity similar to that of binding to human CD3.

In certain embodiments, the anti-CD3 binding portions provided herein specifically bind to recombinant cynomolgus monkey CD3 with an EC₅₀ of no more than 0.001 nM, no more than 0.005 nM, no more than 0.01 nM, no more than 0.02 nM, no more than 0.03 nM, no more than 0.04 nM or no more than 0.05 nM; the EC₅₀ value is determined by ELISA.

In certain embodiments, the anti-CD3 binding portions provided herein have specific binding affinity for human CD3 sufficient for diagnostic and/or therapeutic applications. Many therapeutic regimens modulate T cell immunity by targeting TCR signaling, particularly by clinical use of anti-human CD3 monoclonal antibodies.

### b) Anti-CD20 antibody

In the bispecific anti-CD3/CD20 polypeptide complex provided herein, the first antigen-binding portion or the second antigen-binding portion is an anti-CD20 binding portion.

In certain embodiments, the anti-CD20 binding portion is derived from the antibodies W3278-T2U3.E17R-1.uIgG4.SP and W3278-U3T2.F18R-1.uIgG4.SP shown in Table A' below. The CDR sequences of the anti-CD20 binding portions of the W3278-T2U3.E17R-1.uIgG4.SP and W3278-U3T2.F18R-1.uIgG4.SP antibodies are provided below.

**Table A'**

| Antibody ID: | | CDR1 | CDR2 | CDR3 |
|---|---|---|---|---|
| W3278-T2U3.E17R-1. | | SEQ ID NO: 7 | SEQ ID NO: 8 | SEQ ID NO: 9 |
| uIgG4.SP, W3278-U3T2.F18R-1. uIgG4.SP | VH | GFTFNDYAMH | TISWNSGSIGYADS VKG | DIQYGNYYYGMD V |
| | Vκ | SEQ ID NO: 10 | SEQ ID NO: 11 | SEQ ID NO: 12 |
| | | RASQSVSSYLA | DASNRAT | QQRSNWPIT |

The heavy chain and κ light chain variable region sequences of the anti-CD20 binding portions of the W3278-T2U3.E17R-1.uIgG4.SP and W3278-U3T2.F18R-1.ulgG4.SP antibodies are provided below.
VH-amino acid sequence (SEQ ID NO: 29): 
EVQLVESGGGLVQPGRSLRLSCAASGFTFNDYAMHWVRQAPGKGLEWVSTISWNSGSIGYADSVKGRFT
ISRDNAKKSLYLQMNSLRAEDTALYYCAKDIQYGNYYYGMDVWGQGTTVTVSS
VH-nucleic acid sequence (SEQ ID NO: 57): 
gaggtgcaattggtggagagcggaggagggctcgtgcagcctggaagatctcttaggctgagttgcgctgcatctgggttcacattcaacgactacgccatgcactg
ggtgaggcaggctcccggcaaagggctggaatgggtgtcaactatctcctggaactccggcagcatcggctacgccgatagcgtcaagggccggtttacaatttcc
cgcgataacgccaagaagtccctgtacctgcagatgaacagcctgcgggccgaggatactgccctctactactgtgccaaggacattcagtacgggaattactattac
gggatggacgtctggggccaggggaccaccgtgacagtcagctcc
Vκ-amino acid sequence (SEQ ID NO: 30): 
EIVLTQSPATLSLSPGERATLSCRASQSVSSYLAWYQQKPGQAPRLLIYDASNRATGIPARFSGSGSGTDFTL
TISSLEPEDFAVYYCQQRSNWPITFGQGTRLEIK
Vκ-nucleic acid sequence (SEQ ID NO: 58):

gaaatcgtgctgacccagtccccagcaaccctctccctttctcctggagagagagctaccctcagctgtagggcctcacagtctgtctccagttacctggcttggtacca
gcagaaacccgggcaggcccctaggttgctgatctacgacgccagcaatagggccactggcatcccagcccggttttccggaagcggcagcgggacagatttcac
actcactattagcagcctggagcccgaggacttcgccgtgtactattgccagcagcggtccaactggcccattacatttggccaagggacacgcctggagattaag

In certain embodiments, the anti-CD20 binding portion is derived from the antibodies W3278-T3U2.F16-1.uIgG4.SP, W3278-U2T3.F18R-1.uIgG4.SP and W3278-T3U2.F17R-1.uIgG4.SP shown in Table B' below. The CDR sequences of the anti-CD20 binding portions of the W3278-T3U2.F16-1.uIgG4.SP, W3278-U2T3.F18R-1.uIgG4.SP and W3278-T3U2.F17R-1.uIgG4.SP antibodies are provided below.

**Table B'**

| Antibody ID: | | CDR1 | CDR2 | CDR3 |
|---|---|---|---|---|
| W3278-T3U2.F16-1.uI gG4.SP, W3278-U2T3.F18R-1. uIgG4.SP, W3278-T3U2.F17R-1. uIgG4.SP | VH | SEQ ID NO: 19 | SEQ ID NO: 20 | SEQ ID NO: 21 |
| | | GYTFTSYNMH | AIYPGNGDTSYNQ KFKG | STYYGGDWYFNV |
| | Vκ | SEQ ID NO: 22 | SEQ ID NO: 23 | SEQ ID NO: 24 |
| | | RASSSVSYIH | ATSNLAS | QQWTSNPPT |

The heavy chain and κ light chain variable region sequences of the anti-CD20 binding portions of the W3278-T3U2.F16-1.uIgG4.SP, W3278-U2T3.F18R-1.uIgG4.SP and W3278-T3U2.F17R-1.uIgG4.SP antibodies are provided below.
VH-amino acid sequence (SEQ ID NO: 31):
VH-nucleic acid sequence (SEQ ID NO: 59): 
caggtccagctgcagcagcccggagccgaactggtcaaacccggggctagcgtgaaaatgtcttgcaaagcaagtggttacacattcacttcctataacatgcactg
ggtgaagcagacacctgggcgaggtctggaatggatcggcgccatctacccaggcaacggagacactagctataatcagaagtttaaaggaaaggccaccctgac
agctgataagtccagctctaccgcttacatgcagctgagttcactgacaagtgaggactcagcagtgtactattgcgcccgttctacctactatggcggagattggtattt
caatgtgtggggcgccggtaccacagtcaccgtgtccgcc
Vκ-amino acid sequence (SEQ ID NO: 32):
Vκ-nucleic acid sequence (SEQ ID NO: 60):

cagattgtcctgagccagagccctgccatcctgtctgctagtcccggcgagaaggtgaccatgacatgcagggcatccagctctgtctcctacatccactggttccag
cagaagcccgggagttcacctaaaccatggatctacgctacatccaacctggcaagcggtgtgcctgtcaggttttcaggttccggcagcggaacatcttacagtctg
actatttctcgggtggaggccgaagacgccgctacctactattgccagcagtggacctccaatccccctacattcggcggagggactaagctggagatcaaa

The anti-CD20 binding portions provided herein also comprise suitable framework region (FR) sequences, so long as the anti-CD20 binding portions can specifically bind to CD20.

In some embodiments, the anti-CD20 binding portions provided herein are capable of specifically binding to human CD20 expressed on the surface of a cell with a binding affinity (K_{D}) of no more than 5 × 10⁻⁹ M, no more than 1 × 10⁻⁹ M, no more than 9 × 10⁻¹⁰ M, no more than 8 × 10⁻¹⁰ M, no more than 7 × 10⁻¹⁰ M, no more than 6 × 10⁻¹⁰ M, no more than 5 × 10⁻¹⁰ M, no more than 4 × 10⁻¹⁰ M, no more than 3 × 10⁻¹⁰ M, no more than 2 × 10⁻¹⁰ M or no more than 1 × 10⁻¹⁰ M; the K_{D} value is determined by flow cytometry.

In certain embodiments, the anti-CD20 binding portions provided herein cross-react with cynomolgus monkey CD20 (e.g., cynomolgus monkey CD20 or soluble recombinant cynomolgus monkey CD20 expressed on the surface of a cell).

The binding of the anti-CD20 binding portions to CD20 expressed on a cell can be measured using methods well known in the art, such as sandwich methods (e.g., ELISA), Western blots, flow cytometry assays, and other binding assays. In certain embodiments, the anti-CD20 binding portions provided herein specifically bind to human CD20 expressed on a cell with an EC₅₀ of no more than 0.01 nM, no more than 0.02 nM, no more than 0.03 nM, no more than 0.04 nM, no more than 0.05 nM, no more than 0.1 nM, no more than 0.2 nM, no more than 0.3 nM, no more than 0.4 nM, no more than 0.5 nM, no more than 0.6 nM, no more than 0.7 nM, no more than 0.8 nM, no more than 0.9 nM or no more than 1 nM; the EC₅₀ value is determined by flow cytometry.

In certain embodiments, the anti-CD20 binding portions bind to cynomolgus monkey CD20 with a binding affinity similar to that of binding to human CD20. In certain embodiments, the anti-CD20 binding portions provided herein specifically bind to cynomolgus monkey CD20 expressed on a cell with an EC₅₀ of no more than 0.2 nM, no more than 0.5 nM, no more than 0.8 nM, no more than 1 nM, no more than 2 nM or no more than 3 nM; the EC₅₀ value is determined by flow cytometry.

In certain embodiments, the anti-CD20 binding portions disclosed herein are internalized by CD20-expressing cells with an EC₅₀ of no more than 1 pM, no more than 2 pM, no more than 3 pM, no more than 4 pM, no more than 5 pM, no more than 6 pM, no more than 7 pM, no more than 8 pM, no more than 9 pM, no more than 10 pM, no more than 11 pM, no more than 12 pM, no more than 13 pM, no more than 14 pM, no more than 15 pM, no more than 16 pM, no more than 17 pM, no more than 18 pM, no more than 19 pM, no more than 20 pM, no more than 21 pM, no more than 22 pM, no more than 23 pM, no more than 24 pM, no more than 25 pM, no more than 30 pM, no more than 35 pM, no more than 40 pM, no more than 45 pM or no more than 50 pM; the EC₅₀ value is determined by Fab-Zap assay.

### Bispecific Anti-CD3/CD20 Polypeptide Complex

In certain embodiments, the first antigen-binding portion and/or the second antigen-binding portion are polyvalent, such as divalent, trivalent or tetravalent. The term "valent" as used herein refers to the presence of a specified number of antigen-binding sites in a given molecule. Thus, the terms "divalent", "tetravalent" and "hexavalent" indicate the presence of two binding sites, four binding sites and six binding sites, respectively, in an antigen-binding molecule. If both binding sites are intended to specifically bind to the same antigen or the same epitope, the bivalent molecule can be monospecific. Likewise, a trivalent molecule can be bispecific, for example, when two binding sites are monospecific for a first antigen (or epitope) and the third binding site is specific for a second antigen (or epitope). In certain embodiments, the first antigen-binding portion and/or the second antigen-binding portion in the bispecific anti-CD3/CD20 polypeptide complex disclosed herein can be divalent, trivalent or tetravalent and have at least two binding sites for the same antigen or epitope. In certain embodiments, this provides stronger binding to the antigen or epitope than a corresponding monovalent antibody. In certain embodiments, in a divalent antigen-binding portion, the first valence of the binding site and the second valence of the binding site are structurally identical (i.e., have the same sequence) or structurally different (i.e., have different sequences but the same specificity).

In certain embodiments, the first antigen-binding portion and/or the second antigen-binding portion are polyvalent, and comprise two or more antigen-binding sites that are operably associated with each other (with or without a spacer).

In certain embodiments, the second antigen-binding portion comprises two or more Fabs of the second antibody. The two Fabs can be operably associated with each other; for example, a first Fab can be covalently attached to a second Fab via a heavy chain, with or without a spacer in between.

In certain embodiments, the first antigen-binding portion is linked to a first dimerization domain, and the second antigen-binding portion is linked to a second dimerization domain. The term "dimerization domain" as used herein refers to peptide domains that are capable of associating with each other to form a dimer, or in some examples, peptide domains that enables spontaneous dimerization of two peptides.

In certain embodiments, the first dimerization domain can be associated with the second dimerization domain. The association can be achieved via any suitable interaction or linkage or bonding (e.g., via a linker, a disulfide bond, a hydrogen bond, an electrostatic interaction, a salt bridge, or a hydrophobic-hydrophilic interaction, or a combination thereof). Exemplary dimerization domains include, but are not limited to, an antibody hinge region, an antibody CH2 domain, an antibody CH3 domain, and other suitable protein monomers that are capable of dimerizing and associating with each other. The hinge region, CH2 and/or CH3 domain can be derived from any antibody isotype, such as IgG1, IgG2 and IgG4.

"Disulfide bond" refers to a covalent bond having the structure R-S-S-R'. The amino acid cysteine contains a sulfydryl group, which can form a disulfide bond with a second sulfydryl group, for example from another cysteine residue. The disulfide bond can be formed between the sulfydryl groups of two cysteine residues present on two polypeptide chains, respectively, thereby forming an interchain bridge or an interchain bond.

A hydrogen bond is formed by an electrostatic interaction between two polar groups when a hydrogen atom is covalently bonded to a highly electronegative atom (such as nitrogen, oxygen or fluorine). A hydrogen bond can be formed between the framework oxygen (e.g., a chalcogen group) and the amide hydrogen (nitrogen group) of two residues in a polypeptide, respectively, for example, between the nitrogen group in Asn and the oxygen group in His, or between the oxygen group in Asn and the nitrogen group in Lys. Hydrogen bonds are stronger than van der Waals interactions but weaker than covalent or ionic bonds, and are crucial for maintaining secondary structures and tertiary structures. For example, an α-helix is formed when the spacing of amino acid residues regularly occurs between positions i and i+4, and a β-sheet is a peptide chain segment of 3-10 amino acids formed when two peptide segments are linked by at least two or three framework hydrogen bonds, forming a twisted, pleated sheet.

Electrostatic interactions are non-covalent interactions and are important to protein folding, stability, flexibility and functionality; they include ionic interactions, hydrogen bonding and halogen bonding. Electrostatic interactions can form in polypeptides, for example, between Lys and Asp, between Lys and Glu, between Glu and Arg, or between Glu or Trp on the first chain and Arg, Val or Thr on the second chain.

A salt bridge is a close-range electrostatic interaction arising mainly from the carboxyl anion of Asp or Glu and the ammonium cation from Lys or guanidinium from Arg, which are a pair of oppositely charged residues in close spatial proximity in a native protein structure. Charged and polar residues in a largely hydrophobic interface can serve as hot spots for binding. Among others, residues with ionizable side chains (such as His, Tyr and Ser) can also be involved in the formation of a salt bridge.

Hydrophobic interactions can be formed between one or more Val, Tyr and Ala on the first chain and one or more Val, Leu and Trp on the second chain, or between His and Ala on the first chain and Thr and Phe on the second chain (see Brinkmann et al., 2017, supra).

In certain embodiments, the first dimerization domain and/or the second dimerization domain comprise at least a portion of an antibody hinge region. In certain embodiments, the first dimerization domain and/or the second dimerization domain can further comprise an antibody CH2 domain and/or an antibody CH3 domain. In certain embodiments, the first dimerization domain and/or the second dimerization domain comprise at least a portion of a hinge-Fc region, i.e., a hinge-CH2-CH3 domain. In certain embodiments, the first dimerization domain can be operably linked to the C-terminus of the first TCR constant region. In certain embodiments, the second dimerization domain can be operably linked to the C-terminus of the antibody CH1 constant region of the second antigen-binding portion.

In the bispecific anti-CD3/CD20 polypeptide complex provided herein, the first dimerization domain is operably linked to the C-terminus of an engineered TCR constant region, forming a chimeric constant region. In other words, the chimeric constant region comprises the first dimerization domain operably linked to the engineered TCR constant region.

In certain embodiments, the chimeric constant region comprises an engineered Cβ attached to a first hinge-Fc region derived from IgG1, IgG2 or IgG4.

In certain embodiments, the chimeric constant region further comprises a first antibody CH2 domain and/or a first antibody CH3 domain. For example, the chimeric constant region further comprises a first antibody CH2-CH3 domain attached to the C-terminus of the third junction domain.

These pairs of chimeric constant regions and second TCR constant regions are useful in that they can be manipulated to fuse to a desirable antibody variable region and thus to provide the bispecific anti-CD3/CD20 polypeptide complex disclosed herein. For example, an antibody heavy chain variable region can be fused to the chimeric constant region (including C1) to provide the first polypeptide chain of the bispecific anti-CD3/CD20 polypeptide complex disclosed herein, and similarly, an antibody light chain variable region can be fused to the second TCR constant region (including C2) to provide the second polypeptide chain of the bispecific anti-CD3/CD20 polypeptide complex disclosed herein.

In certain embodiments, the second dimerization domain comprises a hinge region. The hinge region can be derived from an antibody, such as IgG1, IgG2 or IgG4. In certain embodiments, the second dimerization domain optionally can further comprise an antibody CH2 domain and/or an antibody CH3 domain, such as a hinge-Fc region. The hinge region can be attached to the antibody heavy chain of the second antigen-binding site (e.g., Fab).

In the bispecific anti-CD3/CD20 polypeptide complex, the first dimerization domain and the second dimerization domain can be associated to form a dimer. In certain embodiments, the first dimerization domain and the second dimerization domain are different and are associated in a way that discourages homodimerization and/or favors heterodimerization. For example, the first dimerization domain and the second dimerization domain can be selected such that they are not identical and they preferentially form heterodimers with each other rather than homodimers with themselves. In certain embodiments, the first dimerization domain and the second dimerization domain can be associated to form a heterodimer by forming knobs-into-holes, a hydrophobic interaction, an electrostatic interaction, a hydrophilic interaction or increased flexibility. For the "knobs-into-holes", see Ridgway et al., Protein Engineering, 9(7), pp.617-21(1996); Merchant et al., Nature Biotechnology, 16(7), pp.677-681(1998); etc.

In certain embodiments, the first dimerization domain and the second dimerization domain comprise a CH2 domain and/or a CH3 domain, each of which is mutated so that it can form knobs-into-holes. A knob can be obtained by substituting a large amino acid residue for a small amino acid residue in the first CH2/CH3 polypeptide, and a hole can be obtained by substituting a small amino acid residue for a large amino acid residue. For details of mutation sites for knobs-into-holes, see Ridgway et al., 1996, supra; Spiess et al., 2015, supra; and Brinkmann et al., 2017, supra.

### Bispecific Format

In the bispecific anti-CD3/CD20 polypeptide complex described herein, the first antigen-binding portion and the second binding portion can be associated to form an Ig-like structure. Similar to a native antibody, an Ig-like structure has a Y-shaped configuration with two arms for antigen-binding and one stem for association and stabilization. Similarity to native antibodies can provide many advantages, such as good *in vivo* pharmacokinetics, desirable immune responses, stability, etc. Ig-like structures comprising the first antigen-binding portion provided herein in association with the second antigen-binding portion provided herein have been found to have thermal stability comparable to that of an Ig (e.g., IgG). In certain embodiments, the Ig-like structure provided herein is at least 70%, 80%, 90%, 95% or 100% of a native IgG.

In certain embodiments, the bispecific anti-CD3/CD20 polypeptide complex comprises 4 polypeptide chains: i) VH1-C1-hinge-CH2-CH3; ii) VL1-C2; iii) VH2-CH1-hinge-CH2-CH3, and iv) VL2-CL, wherein C1 and C2 are capable of forming a dimer comprising at least one non-native interchain bond, and two hinge regions and/or two CH3 domains are capable of forming one or more interchain bonds capable of aiding in dimerization.

When compared to bispecific polypeptide complexes in other formats, the bispecific anti-CD3/CD20 polypeptide complex provided herein has a longer *in vivo* half-life and is relatively easy to manufacture.

### Sequence of Bispecific Anti-CD3/CD20 Polypeptide Complex

In some embodiments, the first antigen-binding portion of the bispecific anti-CD3/CD20 polypeptide complex is capable of specifically binding to CD3 and the second antigen-binding portion is capable of specifically binding to CD20. In other embodiments, the first antigen-binding portion of the bispecific anti-CD3/CD20 polypeptide complex is capable of specifically binding to CD20 and the second antigen-binding portion is capable of specifically binding to CD3.

In certain embodiments, the bispecific anti-CD3/CD20 polypeptide complex comprises a combination of four polypeptide sequences: SEQ ID NO: 33, SEQ ID NO: 34, SEQ ID NO: 35 and SEQ ID NO: 36 (W3278-T2U3.E17R-1.uIgG4.SP antibody), as shown in Example 2. In certain embodiments, the bispecific anti-CD3/CD20 polypeptide complex comprises a combination of four polypeptide sequences: SEQ ID NO: 37, SEQ ID NO: 38, SEQ ID NO: 39 and SEQ ID NO: 40 (W3278-T3U2.F16-1.uIgG4.SP antibody), as shown in Example 2. In certain embodiments, the bispecific anti-CD3/CD20 polypeptide complex comprises a combination of four polypeptide sequences: SEQ ID NO: 41, SEQ ID NO: 42, SEQ ID NO: 43 and SEQ ID NO: 44, as shown in Example 2. In a certain embodiment, the bispecific anti-CD3/CD20 polypeptide complex comprises five polypeptide chains: a) a first polypeptide chain having a sequence as set forth in SEQ ID NO: 41; b) a second polypeptide chain having a sequence as set forth in SEQ ID NO: 42; c) a third polypeptide chain having a sequence as set forth in SEQ ID NO: 43; d) a fourth polypeptide chain having a sequence as set forth in SEQ ID NO: 43; and e) a fifth polypeptide chain having a sequence as set forth in SEQ ID NO: 44. For example, in a specific embodiment, the bispecific anti-CD3/CD20 polypeptide complex is a W3278-U2T3.F18R-1.ulgG4.SP antibody, which comprises two anti-CD20 binding portions, the heavy chain VH-CH1 domain of one of which is operably linked to the heavy chain VH domain of the anti-CD3 binding portion, as shown in Example 2. In certain embodiments, the bispecific anti-CD3/CD20 polypeptide complex comprises a combination of four polypeptide sequences: SEQ ID NO: 45, SEQ ID NO: 46, SEQ ID NO: 47 and SEQ ID NO: 48 (W3278-U3T2.F18R-1.uIgG4.SP antibody), as shown in Example 2. In certain embodiments, the bispecific anti-CD3/CD20 polypeptide complex comprises a combination of four polypeptide sequences: SEQ ID NO: 49, SEQ ID NO: 50, SEQ ID NO: 51 and SEQ ID NO: 52 (W3278-T3U2.F17R-1.uIgG4.SP antibody), as shown in Example 2. In such embodiments, the first antigen-binding portion binds to CD3 and the second antigen-binding portion binds to CD20.

In certain embodiments, the bispecific anti-CD3/CD20 polypeptide complex comprises 4 polypeptide chains, including: i) VH1 operably linked to a first chimeric constant region; ii) VL1 operably linked to a second chimeric constant region; iii) VH2 operably linked to a conventional antibody heavy chain constant region, and iv) VL2 operably linked to a conventional antibody light chain constant region. In certain embodiments, the first chimeric constant region can comprise C1-hinge-CH2-CH3, each portion of which is as defined above. In certain embodiments, the second chimeric constant region can comprise C2, which is as defined above. In certain embodiments, the conventional antibody heavy chain constant region can comprise CH1-hinge-CH2-CH3, each portion of which is as defined above. In certain embodiments, the conventional antibody light chain constant region can comprise CL, which is as defined above.

In certain embodiments of the present disclosure, one or more of the amino acids in the native glycosylation sites at positions 193, 182, 203, 206 and 207 of the polypeptide chain set forth in SEQ ID NO: 44 are modified; preferably, the amino acid at position 193 is modified. In certain embodiments of the present disclosure, the modifications include one or more of S193X, S182X, S203X, S206X and S207X, wherein X is any amino acid other than serine (Ser) and/or threonine (Thr). In certain preferred embodiments of the present disclosure, the modification is S193X, wherein X is alanine (Ala), glycine (Gly), proline (Pro) or valine (Val). In certain embodiments of the present disclosure, the mutation eliminates an O-glycosylation site; the type of O-glycosylation is an O-saccharide in a Core1 configuration, with a structural formula of NeuAc-Gal-GalNAc or NeuAc-Gal-(NeuAc)GalNAc.

As described above, the modified bispecific anti-CD3/CD20 polypeptide complex which was produced by modifying the native glycosylation sites in corresponding polypeptide chains of the present disclosure is more similar to a native antibody, and has significantly reduced immunogenicity, increased half-life, and improved druggability.

### Preparation Method (not claimed)

The present disclosure provides an isolated nucleic acid or polynucleotide encoding the bispecific anti-CD3 x CD20 polypeptide complex described herein.

The term "nucleic acid" or "polynucleotide" as used herein refers to deoxyribonucleic acid (DNA) or ribonucleic acid (RNA) and polymers thereof in single or double stranded form. Unless specifically limited, the term encompasses polynucleotides containing known native nucleotide analogs that have binding properties similar to those of the reference nucleic acid and are metabolized similarly to naturally occurring nucleotides. Unless otherwise indicated, a particular polynucleotide sequence also implicitly encompasses conservatively modified variants thereof (e.g., degenerate codon substitutions), alleles, homologous genes, SNPs and complementary sequences, as well as sequences explicitly indicated. Specifically, degenerate codon substitutions may be achieved by generating sequences in which the third position of one or more selected (or all) codons are substituted with mixed bases and/or deoxyinosine residues (see Batzer et al., Nucleic Acid Res. 19:5081 (1991); Ohtsuka et al., J. Biol. Chem. 260:2605-2608 (1985); and Rossolini et al., Mol. Cell. Probes 8:91-98(1994)).

The nucleic acid or polynucleotide encoding the bispecific anti-CD3 x CD20 polypeptide complex disclosed herein can be constructed using recombinant techniques. To this end, DNA encoding the antigen-binding portion (e.g., a CDR or variable region) of a parent antibody can be isolated and sequenced using conventional procedures (e.g., by using oligonucleotide probes that are capable of specifically binding to genes encoding the heavy chain and light chain of the antibody). Likewise, DNA encoding the TCR constant region can also be obtained. By way of example, a polynucleotide sequence encoding the variable domain (VH) and a polynucleotide sequence encoding the first TCR constant region (C1) are obtained and operably linked to allow transcription and expression in a host cell to produce the first polypeptide. Similarly, a polynucleotide sequence encoding VL is operably linked to the polynucleotide sequence encoding C1 to allow the expression of the second polynucleotide in a host cell. If needed, coding polynucleotide sequences for one or more spacers can also be operably linked to other coding sequences to allow the expression of the desired product.

The coding polynucleotide sequences can further be operably linked to one or more control sequences, optionally in an expression vector, such that the expression or production of the first polypeptide and the second polypeptide is feasible and under appropriate control.

The encoding polynucleotide sequences can be inserted, using recombinant techniques well known in the art, into vectors for further cloning (DNA amplification) or for expression. In another embodiment, the polypeptide complex and bispecific anti-CD3 x CD20 polypeptide complex disclosed herein can be produced by homologous recombination well known in the art. Many vectors are available. Vector components typically include, but are not limited to, one or more of the following: a signal sequence, an origin of replication, one or more marker genes, an enhancer element, a promoter (e.g., SV40, CMV or EF-1α) and a transcription termination sequence.

Vectors comprising the polynucleotide sequences described in the present disclosure can be introduced into host cells for cloning or gene expression. The term "host cell" as used herein refers to a cell into which an exogenous polynucleotide and/or vector has been introduced. The host cells are transformed with vectors for the expression or cloning described above and cultured in a medium.

In one aspect, the present disclosure provides a method for expressing the bispecific anti-CD3 x CD20 polypeptide complex disclosed herein, which comprises culturing the host cell disclosed herein under such conditions that the bispecific anti-CD3 x CD20 polypeptide complex is expressed.

In certain embodiments, the present disclosure provides a method for producing the bispecific anti-CD3 x CD20 polypeptide complex disclosed herein, which comprises: a) introducing into a host cell one or more polynucleotides encoding a first antigen-binding portion comprising a first polynucleotide encoding a first polypeptide (the first polypeptide comprises, from N-terminus to C-terminus, a first heavy chain variable domain (VH) of a first antibody, which is operably linked to a first T-cell receptor (TCR) constant region (C1)), a second polynucleotide encoding a second polypeptide (the second polypeptide comprises, from N-terminus to C-terminus, a first light chain variable domain (VL) of the first antibody, which is operably linked to a second TCR constant region (C2)) and one or more additional polynucleotides encoding a second antigen-binding portion, wherein: C1 and C2 are capable of forming a dimer, and the non-native interchain bond is capable of stabilizing the dimer of C1 and C2; the first antigen-binding portion and the second antigen-binding portion have reduced mispairing compared to the case where the first antigen-binding portion and the second antigen-binding portion are both natural counterparts of native Fabs, and the first antibody has a first antigen specificity and the second antibody has a second antigen specificity; and b) causing the host cell to express the bispecific anti-CD3 x CD20 polypeptide complex. In certain embodiments, the method further comprises isolating the bispecific anti-CD3 x CD20 polypeptide complex.

The bispecific anti-CD3 x CD20 polypeptide complex disclosed herein prepared from the host cell can be purified using methods such as hydroxyapatite chromatography, gel electrophoresis, dialysis, DEAE-cellulose ion exchange chromatography, ammonium sulfate precipitation, salting out and affinity chromatography, wherein affinity chromatography is the preferred purification technique.

When the bispecific anti-CD3 x CD20 polypeptide complex disclosed herein comprises an immunoglobulin Fc domain, protein A can be used as an affinity ligand, depending on the species and isotype of the Fc domain that is present in the polypeptide complex. Protein A can be used for the purification of polypeptide complexes based on the human γ1, γ2 or γ4 heavy chain (Lindmark et al., J. Immunol. Meth. 62:1-13 (1983)). Protein G is applicable to all mouse isotypes and human y3 (Guss et al., EMBO J. 5:1567 1575 (1986)). Agarose is the most commonly used affinity ligand attachment matrix, but other matrices can also be used. Mechanically stable matrices such as controlled pore glass or poly(styrene)benzene can achieve faster flow rates and shorter processing times compared to agarose.

When the bispecific anti-CD3 x CD20 polypeptide complex disclosed herein comprises a CH3 domain, purification can be carried out using Bakerbond ABX.TM resin (J. T. Baker, Phillipsburg, New Jersey). Other techniques for protein purification, such as fractionation in an ion exchange column, ethanol precipitation, reversed-phase HPLC, silica gel chromatography, heparin-agarose gel chromatography on an anion or cation exchange resin (e.g., a polyaspartic acid column), chromatofocusing, SDS-PAGE and ammonium sulfate precipitation, can also be used, depending on the antibody to be recovered.

After any preliminary purification steps, the mixture containing the polypeptide complex of interest and impurities can be treated by low pH hydrophobic interaction chromatography using an elution buffer having a pH of about 2.5-4.5, preferably at a low salt concentration (e.g., at a salt concentration from about 0 to 0.25 M).

In certain embodiments, the bispecific anti-CD3 x CD20 polypeptide complex disclosed herein can be readily purified in a high yield using conventional methods. One advantage of the bispecific anti-CD3 x CD20 polypeptide complex is that mispairing between heavy chain variable domains and the light chain variable domains is significantly reduced. This reduces the production of unwanted by-products and makes it possible to obtain high-purity products in high yields using relatively simple purification processes.

The following examples are provided to better illustrate the present disclosure and they should not be construed as limiting the scope of the present disclosure. All of the specific compositions, materials and methods described below, in their entirety or in part, fall within the scope of the present disclosure. These specific compositions, materials and methods are not intended to limit the present disclosure, but are merely intended to be illustrative of specific embodiments that fall within the scope of the present disclosure.

### Examples

### Example 1

### Preparation of Materials and Benchmark Antibodies

### 1. Preparation of materials

Information on the commercially available materials used in the examples is provided in Table 1.

**Table 1. Commercially available materials**

| Material | | Vector Cat. |
|---|---|---|
| CD4⁺ T cell isolation kit (human) | Stemcell | 19052 |
| CD8⁺ T cell isolation kit (human) | Stemcell | 19053 |
| Calcein-AM | Invitrogen | C3099 |
| CellTracker^{™} FarRed | Invitrogen | C34572 |
| Propidium iodide (PI) | Invitrogen | P3566 |
| Alexa Fluor647-conjugated goat anti-human IgG Fc | Jackson | 109-605-098 |
| FITC-labeled anti-human CD4 | BD Pharmingen | 550628 |
| PerCP-Cy5.5-labeled anti-human CD8 | BD Pharmingen | 565310 |
| PE-labeled anti-human CD69 | BD Pharmingen | 555531 |
| APC-labeled anti-human CD25 | BD Pharmingen | 555434 |
| Capture antibody purified anti-human TNF | BD Pharmingen | 555212 (51-26371E) |
| Detection antibody biotinylated anti-human TNF monoclonal antibody | BD Pharmingen | 555212 (51-26372E) |
| Enzyme reagent streptavidin-HRP | BD Pharmingen | 555212 (51-9002813) |
| Recombinant human TNF standard | BD Pharmingen | 555212 (51-26376E) |
| Human/primate IL-2 antibody mAb mouse IgG2A | R&D | MAB602 |
| Human/primate IL-2 biotinylated antibody | R&D | BAF202 |
| Recombinant human IL-2 | R&D | 202-IL |
| Jurkat | ATCC | TIB-152 |
| Raji | ATCC | CCL-86 |
| NAMALWA | ATCC | CRL-1432 |
| Ramos | ATCC | CRL-1596 |
| SU-DHL-1 | ATCC | CRL-2955 |

### 2. Generationof benchmark antibodies

Two benchmark antibodies W327-BMK1 and W327-BMK4 were used as reference antibodies in the examples.

The anti-human CD20 benchmark antibody BMK1 (Rituximab) was generated based on the sequence of clone C2B8 from US patent application US 20140004037 A1. The gene for the anti-CD3×CD20 reference bispecific antibody BMK4 (REGN1979) was synthesized according to the sequence in US patent application US 20150266966 A1. The BMK antibodies were expressed by Expi293 cells and then purified by protein A chromatography.

### Example 2

### Preparation of bispecific antibodies of the present disclosure

### 1. Design and engineering of antibodies and TCR chimeric proteins

### TCR sequence

TCR is a heterodimeric protein consisting of two chains. About 95% of human T cells have TCR consisting of an α chain and a β chain. Considering that more crystal structures are available for β chain TRBC1, TRBC1 sequences were selected as the major backbone for use in designing the polypeptide complex ("WuXiBody") disclosed herein. A typical amino acid sequence of TRBC1 can be found in Protein Data Bank (PDB) structure 4L4T.

### Interchain disulfide bond of TCR

The TCR crystal structure was used to guide our WuXiBody design. Unlike the native TCR anchored on the membrane of T cell surface, soluble TCR molecules are less stable, although its 3D structure is very similar to that of antibody Fab. As a matter of fact, the instability of dissolved TCR used to be a significant impediment that made its crystal structure difficult to elucidate (see Wang, Protein Cell, 5(9), pp.649-652 (2014)). We adopted a strategy of introducing a pair of Cys mutations into the TCR constant region and found that it can significantly improve chain assembly and enhance expression.

The junction regions linking the antibody variable domains and the TCR constant domain, their relative fusion orientations and the junction regions linking Fc were all carefully fine-tuned. As the structure of TCR is very similar to that of antibody Fab, we superimposed the antibody Fv homology model on the TCR variable region (PDB 4L4T). The superimposed structure indicates that the antibody Fv is structurally compatible with the TCR constant domain. All the relevant engineering parameters were designed based on this structural alignment and the corresponding sequences.

### 2. Preparation of bispecific antibodies of the present disclosure

The W3278 BsAbs were produced as human IgG4 in a knobs-into-holes format (S. Atwell, J. B. Ridgway, J. A. Wells, P. Carter, Stable heterodimers from remodeling the domain interface of a homodimer using a phage display library. J. Mol. Biol. 270, 26-35 (1997); and C. Spiess, M. Merchant, A. Huang, et al. D. G. Yansura, J. M. Scheer, Bispecific antibodies with natural architecture produced by co-culture of bacteria expressing two distinct half-antibodies. Nat. Biotechnol. 31, 753-758 (2013)). The human IgG4 Fc region was designed with the S228P mutation. Anti-CD3 monoclonal antibodies were produced by immunizing mice with human CD3ε and CD3δ ECD protein using hybridoma technology according to an in-house protocol. The anti-CD20 arm variable region sequences were based on the sequence of Ofatumumab (clone 2F2 of PCT publication No. WO 2010083365A1) or Rituximab (clone C2B8 of US patent application US 20140004037 A1). The sequences of the W3278 BsAb candidates are listed in Table 2. Their DNA sequences were synthesized at Genewiz (Shanghai) and were cloned into modified pcDNA3.3 expression vectors. The expression vectors of the anti-CD20 arm and anti-CD3 arm were co-transfected into Expi293 (Invitrogen-A14527) by using an ExpiFectamine293 transfection kit (Invitrogen-A14524). The cells were cultured in Expi293 expression medium (Invitrogen-A1435101) on an orbital shaker spinning at 135 rpm in an incubator at 37 °C containing a humidified atmosphere with 8% CO₂. The culture supernatant was collected, and the protein was purified using a protein A column (GE Healthcare,17543802). The protein concentration was measured using a UV-Vis spectrophotometer (NanoDrop 2000, Thermo Scientific). The protein purity was assessed by SDS-PAGE and analytical HPLC-SEC. FIG. 1 shows a schematic of the W3278-BsAb candidates.

**Table 2. The sequences of the heavy chain and/or light chain of each BsAb**

| Clone ID | | SEQ ID NO | Amino acid sequence |
|---|---|---|---|
| W3278-T2U3.E 17R-1.uIgG4.SP | H1 | 33 | QVQLVQSGAEVKKPGSSVKVSCKASGYSFTTYYIHWVRQAPGQ GLEWMGWIFPGNDNIKYSEKFKGRVTITADKSTSTAYMELSSLR SEDTAVYYCAIDSVSIYYFDYWGQGTLVTVSSASTKGPSVFPLAP CSRSTSESTAALGCLVKDYFPEPVTVSWNSGALTSGVHTFPAVL QSSGLYSLSSVVTVPSSSLGTKTYTCNVDHKPSNTKVDKRVESK YGPPCPPCPAPEFLGGPSVFLFPPKPKDTLMISRTPEVTCVVVDV SQEDPEVQFNWYVDGVEVHNAKTKPREEQFNSTYRVVSVLTVL HQDWLNGKEYKCKVSNKGLPSSIEKTISKAKGQPREPQVYTLPP CQEEMTKNQVSLWCLVKGFYPSDIAVEWESNGQPENNYKTTPP VLDSDGSFFLYSRLTVDKSRWQEGNVFSCSVMHEALHNHYTQK SLSLSLG |
| | H2 | 34 | EVQLVESGGGLVQPGRSLRLSCAASGFTFNDYAMHWVRQAPG KGLEWVSTISWNSGSIGYADSVKGRFTISRDNAKKSLYLQMNSL RAEDTALYYCAKDIQYGNYYYGMDVWGQGTTVTVLEDLKNVF PPEVAVFEPSEAEISHTQKATLVCLATGFYPDHVELSWWVNGKEVH |
| | | | SGVCTDPQPLKEQPALQDSRYALSSRLRVSATFWQNPRNHFRCQVQ FYGLSENDEWTQDRAKPVTQIVSAEAWGRYGPPCPPCPAPEFLGG PSVFLFPPKPKDTLMISRTPEVTCVVVDVSQEDPEVQFNWYVDG VEVHNAKTKPREEQFNSTYRVVSVLTVLHQDWLNGKEYKCKV SNKGLPSSIEKTISKAKGQPREPQVCTLPPSQEEMTKNQVSLSCA VKGFYPSDIAVEWESNGQPENNYKTTPPVLDSDGSFFLVSRLTV DKSRWQEGNVFSCSVMHEALHNHYTQKSLSLSLG |
| | L1 | 35 | DIVMTQSPDSLAVSLGERATINCKSSQSLLNSRTRKNYLAWYQQ KPGQPPKLLIYWASTRKSGVPDRFSGSGSGTDFTLTISSLQAEDV AVYYCTQSFILRTFGGGTKVEIKRTVAAPSVFIFPPSDEQLKSGTA SVVCLLNNFYPREAKVQWKVDNALQSGNSQESVTEQDSKDST YSLSSTLTLSKADYEKHKVYACEVTHQGLSSPVTKSFNRGEC |
| | L2 | 36 | EIVLTQSPATLSLSPGERATLSCRASQSVSSYLAWYQQKPGQAPR LLIYDASNRATGIPARFSGSGSGTDFTLTISSLEPEDFAVYYCQQR SNWPITFGQGTRLEIKPDIQNPDPAVYQLRDSKSSDKSVCLFTDFD SQTQVSQSKDSDVYITDKCVLDMRSMDFKSNSAVAWSQKSDFACAN AFQNSIIPEDTFFPSPESS |
| W3278-T3U2.F1 6-1.uIgG4.SP | H1 | 37 | QVQLVQSGAEVKKPGSSVKVSCKASGFAFTDYYIHWVRQAPGQ GLEWMGWISPGNVNTKYNENFKGRVTITADKSTSTAYMELSSL RSEDTAVYYCARDGYSLYYFDYWGQGTLVTVLEDLKNVFPPEVA VFEPSEAEISHTQKATLVCLATGFYPDHVELSWWVNGKEVHSGVCT DPQPLKEQPALQDSRYALSSRLRVSATFWQNPRNHFRCQVQFYGLS ENDEWTQDRAKPVTQIVSAEAWGRYGPPCPPCPAPEFLGGPSVFL FPPKPKDTLMISRTPEVTCVVVDVSQEDPEVQFNWYVDGVEVH NAKTKPREEQFNSTYRVVSVLTVLHQDWLNGKEYKCKVSNKG LPSSIEKTISKAKGQPREPQVYTLPPCQEEMTKNQVSLWCLVKG FYPSDIAVEWESNGQPENNYKTTPPVLDSDGSFFLYSRLTVDKSR WQEGNVFSCSVMHEALHNHYTQKSLSLSLG |
| | H2 | 38 | QVQLQQPGAELVKPGASVKMSCKASGYTFTSYNMHWVKQTPG RGLEWIGAIYPGNGDTSYNQKFKGKATLTADKSSSTAYMQLSSL TSEDSAVYYCARSTYYGGDWYFNVWGAGTTVTVSAASTKGPS VFPLAPCSRSTSESTAALGCLVKDYFPEPVTVSWNSGALTSGVH TFPAVLQSSGLYSLSSVVTVPSSSLGTKTYTCNVDHKPSNTKVD KRVGGGGSGGGGSQVQLQQPGAELVKPGASVKMSCKASGYTF TSYNMHWVKQTPGRGLEWIGAIYPGNGDTSYNQKFKGKATLT ADKSSSTAYMQLSSLTSEDSAVYYCARSTYYGGDWYFNVWGA GTTVTVSAASTKGPSVFPLAPCSRSTSESTAALGCLVKDYFPEPV TVSWNSGALTSGVHTFPAVLQSSGLYSLSSVVTVPSSSLGTKTYT CNVDHKPSNTKVDKRVESKYGPPCPPCPAPEFLGGPSVFLFPPKP KDTLMISRTPEVTCVVVDVSQEDPEVQFNWYVDGVEVHNAKT |
| | | | KPREEQFNSTYRVVSVLTVLHQDWLNGKEYKCKVSNKGLPSSI EKTISKAKGQPREPQVCTLPPSQEEMTKNQVSLSCAVKGFYPSDI AVEWESNGQPENNYKTTPPVLDSDGSFFLVSRLTVDKSRWQEG NVFSCSVMHEALHNHYTQKSLSLSLG |
| | L1 | 39 | DIVMTQSPDSLAVSLGERATINCKSSQSLLNSRTRKNYLAWYQQ KPGQPPKLLIYWASTRQSGVPDRFSGSGSGTDFTLTISSLQAEDV AVYYCTQSHTLRTFGGGTKVEIKPDIQNPDPAVYQLRDSKSSDKSV CLFTDFDSQTQVSQSKDSDVYITDKCVLDMRSMDFKSNSAVAWSQK SDFACANAFQNSIIPEDTFFPSPESS |
| | L2 | 40 | QIVLSQSPAILSASPGEKVTMTCRASSSVSYIHWFQQKPGSSPKP WIYATSNLASGVPVRFSGSGSGTSYSLTISRVEAEDAATYYCQQ WTSNPPTFGGGTKLEIKRTVAAPSVFIFPPSDEQLKSGTASVVCL LNNFYPREAKVQWKVDNALQSGNSQESVTEQDSKDSTYSLSST LTLSKADYEKHKVYACEVTHQGLSSPVTKSFNRGEC |
| W3278-U2T3.F1 8R-1.uIgG4.SP | H1 | 41 | QVQLQQPGAELVKPGASVKMSCKASGYTFTSYNMHWVKQTPG RGLEWIGAIYPGNGDTSYNQKFKGKATLTADKSSSTAYMQLSSL TSEDSAVYYCARSTYYGGDWYFNVWGAGTTVTVSAASTKGPS VFPLAPCSRSTSESTAALGCLVKDYFPEPVTVSWNSGALTSGVH TFPAVLQSSGLYSLSSVVTVPSSSLGTKTYTCNVDHKPSNTKVD KRVGGGGSGGGGSQVQLVQSGAEVKKPGSSVKVSCKASGFAFT DYYIHWVRQAPGQGLEWMGWISPGNVNTKYNENFKGRVTITA DKSTSTAYMELSSLRSEDTAVYYCARDGYSLYYFDYWGQGTLV TVLEDLKNVFPPEVAVFEPSEAEISHTQKATLVCLATGFYPDHVELS WWVNGKEVHSGVCTDPQPLKEQPALQDSRYALSSRLRVSATFWQN PRNHFRCQVQFYGLSENDEWTQDRAKPVTQIVSAEAWGRYGPPCP PCPAPEFLGGPSVFLFPPKPKDTLMISRTPEVTCVVVDVSQEDPE VQFNWYVDGVEVHNAKTKPREEQFNSTYRVVSVLTVLHQDWL NGKEYKCKVSNKGLPSSIEKTISKAKGQPREPQVYTLPPCQEEM TKNQVSLWCLVKGFYPSDIAVEWESNGQPENNYKTTPPVLDSD GSFFLYSRLTVDKSRWQEGNVFSCSVMHEALHNHYTQKSLSLSL G |
| | H2 | 42 | QVQLQQPGAELVKPGASVKMSCKASGYTFTSYNMHWVKQTPG RGLEWIGAIYPGNGDTSYNQKFKGKATLTADKSSSTAYMQLSSL TSEDSAVYYCARSTYYGGDWYFNVWGAGTTVTVSAASTKGPS VFPLAPCSRSTSESTAALGCLVKDYFPEPVTVSWNSGALTSGVH TFPAVLQSSGLYSLSSVVTVPSSSLGTKTYTCNVDHKPSNTKVD KRVESKYGPPCPPCPAPEFLGGPSVFLFPPKPKDTLMISRTPEVTC VVVDVSQEDPEVQFNWYVDGVEVHNAKTKPREEQFNSTYRVV SVLTVLHQDWLNGKEYKCKVSNKGLPSSIEKTISKAKGQPREPQ VCTLPPSQEEMTKNQVSLSCAVKGFYPSDIAVEWESNGQPENNY |
| | | | KTTPPVLDSDGSFFLVSRLTVDKSRWQEGNVFSCSVMHEALHN HYTQKSLSLSLG |
| | L1 | 43 | QIVLSQSPAILSASPGEKVTMTCRASSSVSYIHWFQQKPGSSPKP WIYATSNLASGVPVRFSGSGSGTSYSLTISRVEAEDAATYYCQQ WTSNPPTFGGGTKLEIKRTVAAPSVFIFPPSDEQLKSGTASVVCL LNNFYPREAKVQWKVDNALQSGNSQESVTEQDSKDSTYSLSST LTLSKADYEKHKVYACEVTHQGLSSPVTKSFNRGEC |
| | L2 | 44 | DIVMTQSPDSLAVSLGERATINCKSSQSLLNSRTRKNYLAWYQQ KPGQPPKLLIYWASTRQSGVPDRFSGSGSGTDFTLTISSLQAEDV AVYYCTQSHTLRTFGGGTKVEIKPDIQNPDPAVYQLRDSKSSDKSV CLFTDFDSQTQVSQSKDSDVYITDKCVLDMRSMDFKSNSAVAWSQK SDFACANAFQNSIIPEDTFFPSPESS |
| W3278-U3T2.F1 8R-1.uIgG4.SP | H1 | 45 | EVQLVESGGGLVQPGRSLRLSCAASGFTFNDYAMHWVRQAPG KGLEWVSTISWNSGSIGYADSVKGRFTISRDNAKKSLYLQMNSL RAEDTALYYCAKDIQYGNYYYGMDVWGQGTTVTVSSASTKGP SVFPLAPCSRSTSESTAALGCLVKDYFPEPVTVSWNSGALTSGV HTFPAVLQSSGLYSLSSVVTVPSSSLGTKTYTCNVDHKPSNTKV DKRVGGGGSGGGGSQVQLVQSGAEVKKPGSSVKVSCKASGYS FTTYYIHWVRQAPGQGLEWMGWIFPGNDNIKYSEKFKGRVTIT ADKSTSTAYMELSSLRSEDTAVYYCAIDSVSIYYFDYWGQGTLV TVLEDLKNVFPPEVAVFEPSEAEISHTQKATLVCLATGFYPDHVELS WWVNGKEVHSGVCTDPQPLKEQPALQDSRYALSSRLRVSATFWQN PRNHFRCQVQFYGLSENDEWTQDRAKPVTQIVSAEAWGRYGPPCP PCPAPEFLGGPSVFLFPPKPKDTLMISRTPEVTCVVVDVSQEDPE VQFNWYVDGVEVHNAKTKPREEQFNSTYRVVSVLTVLHQDWL NGKEYKCKVSNKGLPSSIEKTISKAKGQPREPQVYTLPPCQEEM TKNQVSLWCLVKGFYPSDIAVEWESNGQPENNYKTTPPVLDSD GSFFLYSRLTVDKSRWQEGNVFSCSVMHEALHNHYTQKSLSLSL G |
| | H2 | 46 | EVQLVESGGGLVQPGRSLRLSCAASGFTFNDYAMHWVRQAPG KGLEWVSTISWNSGSIGYADSVKGRFTISRDNAKKSLYLQMNSL RAEDTALYYCAKDIQYGNYYYGMDVWGQGTTVTVSSASTKGP SVFPLAPCSRSTSESTAALGCLVKDYFPEPVTVSWNSGALTSGV HTFPAVLQSSGLYSLSSVVTVPSSSLGTKTYTCNVDHKPSNTKV DKRVESKYGPPCPPCPAPEFLGGPSVFLFPPKPKDTLMISRTPEVT CVVVDVSQEDPEVQFNWYVDGVEVHNAKTKPREEQFNSTYRV VSVLTVLHQDWLNGKEYKCKVSNKGLPSSIEKTISKAKGQPREP QVCTLPPSQEEMTKNQVSLSCAVKGFYPSDIAVEWESNGQPENN YKTTPPVLDSDGSFFLVSRLTVDKSRWQEGNVFSCSVMHEALH NHYTQKSLSLSLG |
| | L1 | 47 | EIVLTQSPATLSLSPGERATLSCRASQSVSSYLAWYQQKPGQAPR LLIYDASNRATGIPARFSGSGSGTDFTLTISSLEPEDFAVYYCQQR SNWPITFGQGTRLEIKRTVAAPSVFIFPPSDEQLKSGTASVVCLLN NFYPREAKVQWKVDNALQSGNSQESVTEQDSKDSTYSLSSTLT LSKADYEKHKVYACEVTHQGLSSPVTKSFNRGEC |
| | L2 | 48 | DIVMTQSPDSLAVSLGERATINCKSSQSLLNSRTRKNYLAWYQQ KPGQPPKLLIYWASTRKSGVPDRFSGSGSGTDFTLTISSLQAEDV AVYYCTQSFILRTFGGGTKVEIKPDIQNPDPAVYQLRDSKSSDKSV CLFTDFDSQTQVSQSKDSDVYITDKCVLDMRSMDFKSNSAVAWSQK SDFACANAFQNSIIPEDTFFPSPESS |
| W3278-T3U2.F1 7R-1.uIgG4.SP | H1 | 49 | QVQLVQSGAEVKKPGSSVKVSCKASGFAFTDYYIHWVRQAPGQ GLEWMGWISPGNVNTKYNENFKGRVTITADKSTSTAYMELSSL RSEDTAVYYCARDGYSLYYFDYWGQGTLVTVLEDLKNVFPPEVA VFEPSEAEISHTQKATLVCLATGFYPDHVELSWWVNGKEVHSGVCT DPQPLKEQPALQDSRYALSSRLRVSATFWQNPRNHFRCQVQFYGLS ENDEWTQDRAKPVTQIVSAEAWGRGGGGSGGGGSQVQLQQPGA ELVKPGASVKMSCKASGYTFTSYNMHWVKQTPGRGLEWIGAI YPGNGDTSYNQKFKGKATLTADKSSSTAYMQLSSLTSEDSAVYY CARSTYYGGDWYFNVWGAGTTVTVSAASTKGPSVFPLAPCSRS TSESTAALGCLVKDYFPEPVTVSWNSGALTSGVHTFPAVLQSSGL YSLSSVVTVPSSSLGTKTYTCNVDHKPSNTKVDKRVESKYGPPC PPCPAPEFLGGPSVFLFPPKPKDTLMISRTPEVTCVVVDVSQEDP EVQFNWYVDGVEVHNAKTKPREEQFNSTYRVVSVLTVLHQDW LNGKEYKCKVSNKGLPSSIEKTISKAKGQPREPQVYTLPPCQEE MTKNQVSLWCLVKGFYPSDIAVEWESNGQPENNYKTTPPVLDS DGSFFLYSRLTVDKSRWQEGNVFSCSVMHEALHNHYTQKSLSL SLG |
| | H2 | 50 | QVQLQQPGAELVKPGASVKMSCKASGYTFTSYNMHWVKQTPG RGLEWIGAIYPGNGDTSYNQKFKGKATLTADKSSSTAYMQLSSL TSEDSAVYYCARSTYYGGDWYFNVWGAGTTVTVSAASTKGPS VFPLAPCSRSTSESTAALGCLVKDYFPEPVTVSWNSGALTSGVH TFPAVLQSSGLYSLSSVVTVPSSSLGTKTYTCNVDHKPSNTKVD KRVESKYGPPCPPCPAPEFLGGPSVFLFPPKPKDTLMISRTPEVTC VVVDVSQEDPEVQFNWYVDGVEVHNAKTKPREEQFNSTYRVV SVLTVLHQDWLNGKEYKCKVSNKGLPSSIEKTISKAKGQPREPQ VCTLPPSQEEMTKNQVSLSCAVKGFYPSDIAVEWESNGQPENNY KTTPPVLDSDGSFFLVSRLTVDKSRWQEGNVFSCSVMHEALHN HYTQKSLSLSLG |
| | L1 | 51 | DIVMTQSPDSLAVSLGERATINCKSSQSLLNSRTRKNYLAWYQQ KPGQPPKLLIYWASTRQSGVPDRFSGSGSGTDFTLTISSLQAEDV AVYYCTQSHTLRTFGGGTKVEIKPDIQNPDPAVYQLRDSKSSDKSV CLFTDFDSQTQVSQSKDSDVYITDKCVLDMRSMDFKSNSAVAWSQK SDFACANAFQNSIIPEDTFFPSPESS |
| | L2 | 52 | QIVLSQSPAILSASPGEKVTMTCRASSSVSYIHWFQQKPGSSPKP WIYATSNLASGVPVRFSGSGSGTSYSLTISRVEAEDAATYYCQQ WTSNPPTFGGGTKLEIKRTVAAPSVFIFPPSDEQLKSGTASVVCL LNNFYPREAKVQWKVDNALQSGNSQESVTEQDSKDSTYSLSST LTLSKADYEKHKVYACEVTHQGLSSPVTKSFNRGEC |

| | | | |
|---|---|---|---|
| Note: TCR sequences are shown in italic. | | | |

Further, for the bispecific antibody W3278-U2T3.F18R-1.uIgG4.SP, one or more of the amino acids in the native glycosylation sites at positions 193, 182, 203, 206 and 207 of the light chain set forth in SEQ ID NO: 44 were modified by the inventors, preferably, the amino acid at position 193 is modified. In certain embodiments, the modifications include one or more of S193X, S182X, S203X, S206X and S207X, wherein X represented any amino acid other than serine (Ser) and/or threonine (Thr). In certain preferred embodiments, the modification is S193X, wherein X is alanine (Ala), glycine (Gly), proline (Pro) or valine (Val). The mutation described above eliminated an O-glycosylation site; the type of O-glycosylation was an O-saccharide in a Core1 configuration, with a structural formula of NeuAc-Gal-GalNAc or NeuAc-Gal-(NeuAc)GalNAc.

As described above, the modified bispecific antibody which was produced by modifying the native glycosylation sites in corresponding polypeptide chains of the bispecific antibody disclosured hereinwas more similar to a native antibody, and had significantly reduced immunogenicity, increased half-life, and improved druggability.

### Example 3

### In Vitro Characterization

### 1. Cell lines and primary cell isolation

The following cell lines cultured in complete media (RPMI1640 supplemented with 10% FBS, 100 U/mL penicillin and 100 µg/mL streptomycin) were used: Jurkat (CD3+/CD20- cells), Raji, Ramos and NAMALWA (CD20+/CD3- cells), and SU-DHL-1 (CD20-/CD3- cells).

Human peripheral blood mononuclear cells (PBMCs) were freshly isolated from heparinized venous blood from healthy normal donors by density centrifugation using Ficoll-Paque PLUS (GE Healthcare-17-1440-03). Primary human CD8⁺ T cells were isolated from fresh human PBMCs using an EasySep kit (Stemcell-19053), and CD4⁺ T cells were purified using an EasySep (Stemcell-19052) column.

### 2. Binding of W3278 BsAb to target cells

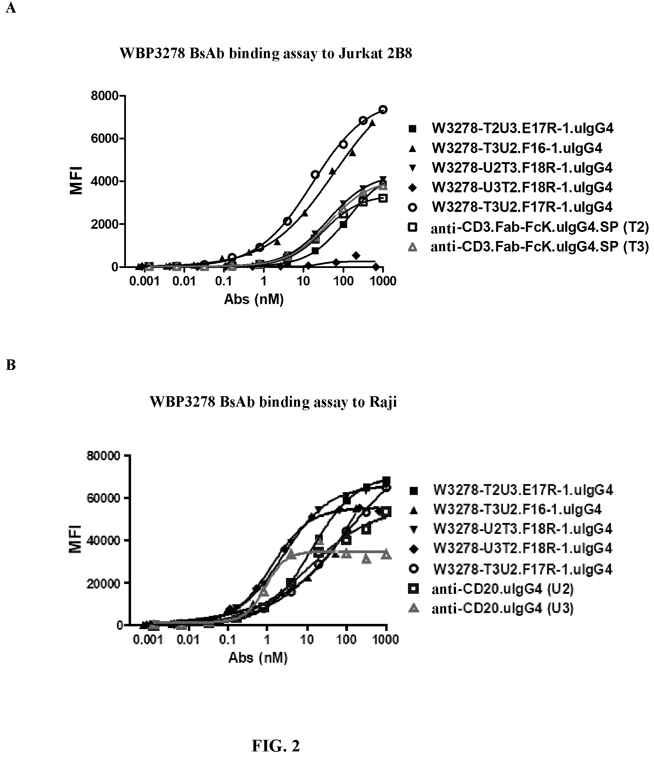

The binding of W3278 BsAb to target cells was determined by flow cytometry. Briefly, 1 × 10⁵/well of target cells (CD3+/CD20- cells or CD20+/CD3- cells) were incubated with serial dilutions of W3278 BsAb or human IgG4 isotype control antibody at 4 °C for 60 min. After incubation, the cells were washed twice with cold 1% BSA/1×PBS. Then Alexa Fluor647-conjugated goat anti-human IgG Fc (Jackson-109-605-098) was added, and the cells were incubated at 4 °C for 30 min. After being washed twice, the geometric mean fluorescence (MFI) of the stained cells was measured using a FACS Canto II hemocytometer (BD Biosciences). Wells containing no antibody or containing only fluorescent secondary antibody were used to establish background fluorescence. The EC₅₀ values for cell binding were determined using GraphPad Prism 5 software (GraphPad Software, La Jolla, CA); each value was calculated by four-parameter non-linear regression analysis. The FACS binding of W3278 BsAb to target cells are shown in FIG. 2, and the binding EC₅₀ are shown in Table 3 below.

**Table 3. The FACS binding EC₅₀ of W3278 BsAbs and the parental antibodies to cell surface targets**

| Abs | Jurkat 2B8 | | Raji | |
|---|---|---|---|---|
| | EC50 (nM) | TOP MFI | EC50 (nM) | TOP MFI |
| W3278-T2U3.E17R-1.uIgG4.SP | 128.7 | 3876 | 13.2 | 68500 |
| W3278-T3U2.F16-1.uIgG4.SP | 58.9 | 6753 | >1000 | 55200 |
| W3278-U2T3.F18R-1.uIgG4.SP | 41.7 | 4077 | 2.5 | 66700 |
| W3278-U3T2.F18R-1.uIgG4.SP | 26.6 | 536 | 1.3 | 55400 |
| W3278-T3U2.F17R-1.uIgG4.SP | 17.3 | 7355 | 85.3 | 64900 |
| Anti-CD3 Fab (T2) | 33.0 | 3214 | | |
| Anti-CD3 Fab (T3) | 43.7 | 3803 | | |
| Anti-CD20. IgG4 (U2) | | | 11.4 | 53500 |
| Anti-CD20. IgG4 (U3) | | | 0.9 | 33300 |

To determine the simultaneous binding of W3278 BsAb to CD3 and CD20-expressing cells, 1 × 10⁶/mL of Raji cells and 1 × 10⁶/mL of Jurkat cells were labeled with 50 nM Calcein-AM (Invitrogen-C3099) and 20 nM FarRed (Invitrogen-C34572), respectively. After being washed with cold 1% BSA/1XPBS, the labeled Raji cells and Jurkat cells were resuspended and mixed at a 1:1 ratio to a final concentration of 1 × 10⁶ cells/mL. The mixed cells were plated at 1 × 10⁵/well, and serial dilutions of W3278 BsAb were then added. After incubation at 4 °C for 60 min, the percentage of Calcein-AM and FarRed double positive cells was analyzed by FACS.

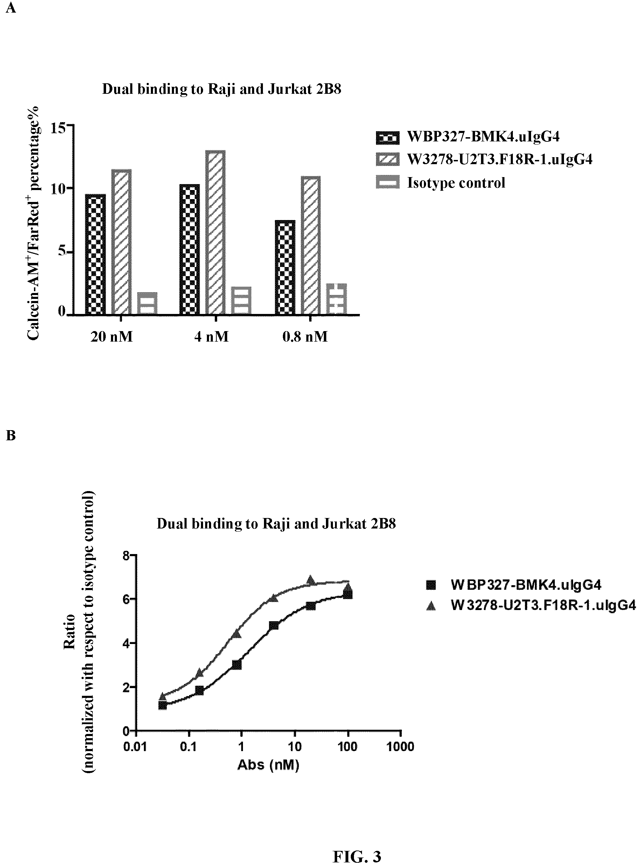

The results in FIG. 3 indicate that W3278 lead BsAb showed dose-dependent simultaneous dual-target binding, which was more effective than that ofBMK4.

### 3. In vitro cytotoxicity assays

The efficacy of BsAb to regulate tumor cell lysis via CD8⁺ T lymphocytes was determined through FACS-based cytotoxicity assays. Briefly, freshly isolated human CD8⁺ T cells were cultured for 3 to 5 days in a complete medium containing 50 IU/mL recombinant human IL-2 and 10 ng/mL OKT-3. At the following day, target cells, Raji, Ramos, NAMALWA and SU-DHL-1 (1 × 10⁶ cells/mL) were labeled with 20 nM Far-Red in DPBS (Invitrogen-C34572) at 37 °C for 30 min, and then washed twice with assay buffer (phenol red-free RPMI 1640 medium + 10% FBS). The Far-Red-labeled target cells (2 × 10⁴/well) were plated in 110 µL/well of complete media containing effector CD8⁺ T cells (effector/target cell ratio of 5:1) and serial dilutions of BsAbs or hIgG4 isotype control and incubated overnight at 37 °C. Finally, propidium Iodide (PI) (Invitrogen-P3566) was added, and the cells were incubated at room temperature for 15 min and then analyzed by flow cytometry. The percent cytotoxicity was calculated using the following equation: cytotoxicity% = 100 × Far Red⁺PI⁺ / (Far Red⁺PI⁺+ Far Red⁺PI⁻) × 100%. EC₅₀ values of *in vitro* cytotoxicity were determined by Prism four-parameter non-linear regression analysis.

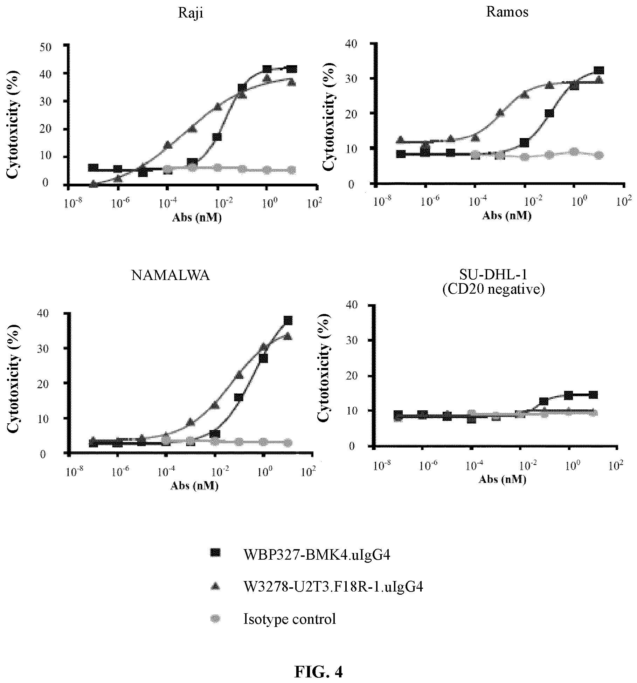

The results in FIG. 4 show that W3278 lead Ab "W3278-U2T3.F18R-1.uIgG4" did not kill CD20 negative SU-DHL-1 cells. W3278 lead Ab "W3278-U2T3.F18R-1.uIgG4" induced cell killing of CD20 positive cells more effectively than BMK4, and the killing efficiency EC₅₀ increased in proportion to the CD20 expression level on the cell surface. The BsAb-mediated cytotoxicity EC₅₀ and maximum cytotoxicity (Max Cyto)% are shown in Table 4.

**Table 4. The cytotoxicity EC₅₀ and maximum cytotoxicity% of W3278 lead BsAb and BMK4 BsAb on different B cell lines**

| Abs | Raji | | Ramos | | NAMALWA | | SU-DHL-1 | |
|---|---|---|---|---|---|---|---|---|
| | EC50 (pM) | Max Cyto% | EC50 (pM) | Max Cyto% | EC50 (pM) | Max Cyto% | EC50 (pM) | Max Cyto% |
| WBP327-BMK4.uIgG4 | 21.49 | 36.4 | 119.2 | 24.6 | 411.7 | 40.3 | 53.59 | 6.1 |
| W3278-U2T3.F18R-1.uIgG4 | 0.52 | 41.7 | 1.25 | 17.1 | 46.8 | 33.0 | NA | 1.6 |

### 4. Cell activation and cytokine release assays

BsAb-mediated T cell activation was assessed by measuring effector cells' expression of CD69 or CD25 by flow cytometry. Freshly isolated purified CD4⁺ T cells and CD8⁺ T cells were separately tested as effector cells. Briefly, 5 × 10⁴ CD4⁺ or CD8⁺ T cells were plated in 110 µL/well of a complete media containing serial dilutions of BsAbs or hIgG4 isotype control antibody in the presence of 1 × 10⁴ Raji or SU-DHL-1 cells/well at 37 °C for 24 h. After incubation, the cells were washed twice with 1% BSA/1XDPBS, and then stained with an anti-human Ab group (FITC-labeled anti-human CD4 (BD Pharmingen-550628); PerCP-Cy5.5-labeled anti-human CD8 (BD Pharmingen-565310); PE-labeled anti-human CD69 (BD Pharmingen-555531) and APC-labeled anti-human CD25 (BD Pharmingen-555434)) at 4 °C for 30 min. The T cell activation assessed via CD69 or CD25 expression was analyzed by FACS. EC₅₀ of T cell activation was determined by four-parameter non-linear regression analysis.

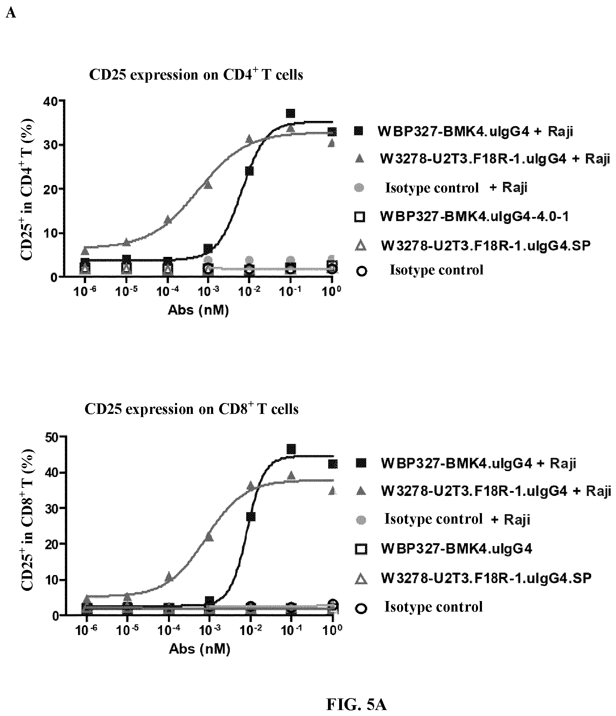

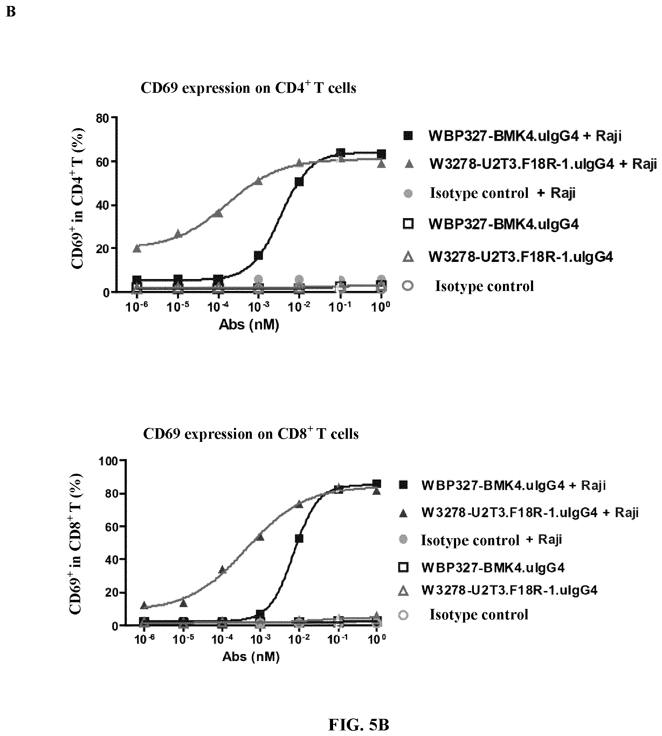

In the absence of target cells, W3278 lead Ab did not induce T cell activation. W3278 lead Ab induced CD4⁺ and CD8⁺ T cell activation only in the presence of target cells, as shown by CD25 expression (FIG. 5A) and CD69 expression (FIG. 5B), and that is more effectively than BMK4. The activation EC₅₀ is shown in Tables 5A and 5B.

**Table 5A. EC₅₀ of T cells' CD25 expression mediated by W3278 BsAb and BMK4 BsAb in the presence of Raji cells**

| Abs | CD4⁺/ Raji | | CD8⁺/ Raji | |
|---|---|---|---|---|
| | EC50 (pM) | Max % | EC50 (pM) | Max % |
| W3278-U2T3.F18R-1.uIgG4 | 0.6 | 34.0 | 0.8 | 39.3 |
| WBP327-BMK4.uIgG4 | 6.5 | 37.1 | 8.2 | 46.5 |

**Table 5B. EC₅₀ of T cells' CD69 expression mediated by W3278 BsAb and BMK4 BsAb in the presence of Raji cells**

| Abs | CD4⁺ / Raji | | CD8⁺/ Raji | |
|---|---|---|---|---|
| | EC50 (pM) | Max % | EC50 (pM) | Max % |
| W3278-U2T3.F18R-1.uIgG4.SP | 0.2 | 61.8 | 0.4 | 83.8 |
| WBP327-BMK4.uIgG4 | 3.5 | 64.2 | 7.3 | 86.0 |

For cytokine release (TNF-α and IL-2) assays, 5 × 10⁴ freshly isolated CD4⁺ T cells were plated in 110 µL/well of complete media containing serial dilutions of BsAbs or hIgG4 isotype control antibody in the presence of 1 × 10⁴ Raji or SU-DHL-1 cells/well at 37 °C for 24 h. After 24 h of incubation, the plate was centrifuged, and the supernatant was collected and stored at -80 °C for cytokine concentration measurement by ELISA.

For TNF-α detection by ELISA, a 96-well ELISA plate (Nunc MaxiSorp, ThermoFisher) was coated with 50 µL of capture antibody-purified anti-human TNF (BD Pharmingen-51-26371E) in carbonate-bicarbonate buffer (20 mM Na₂CO₃, 180 mM NaHCO₃, pH 9.2) overnight at 4 °C. At the following day, the plate was washed with wash buffer (1X PBST buffer, 0.05% tween-20) and then blocked with 200 µL of assay diluent (PBS + 10% FBS). After blocking, 50 µL of a test sample and recombinant human TNF standard (BD Pharmingen-51-26376E) were added, and the plate was incubated at room temperature for 2 h. The binding of TNF-α to the plate was detected by the detection antibody biotinylated anti-human TNF (BD Pharmingen-51-26372E). Streptavidin-HRP reagent (BD Pharmingen-51-9002813)) and tetramethylbenzidine (TMB) substrate (Sigma-860336-5G) were used for color reaction. Washing was performed with wash buffer between steps. After about 30 min, the color reaction was stopped with 2 M HCl. The absorbance of each well was measured at 450 nm using a multifunctional plate reader (SpectraMax^{®} M5e).

Similarly, the IL-2 concentration in the culture supernatant was measured by ELISA. An anti-human IL-2 antibody mAb (R&D-MAB602) was used as the capture antibody and a biotinylated anti-human IL-2 antibody (R&D-BAF202) was used as the detection antibody.

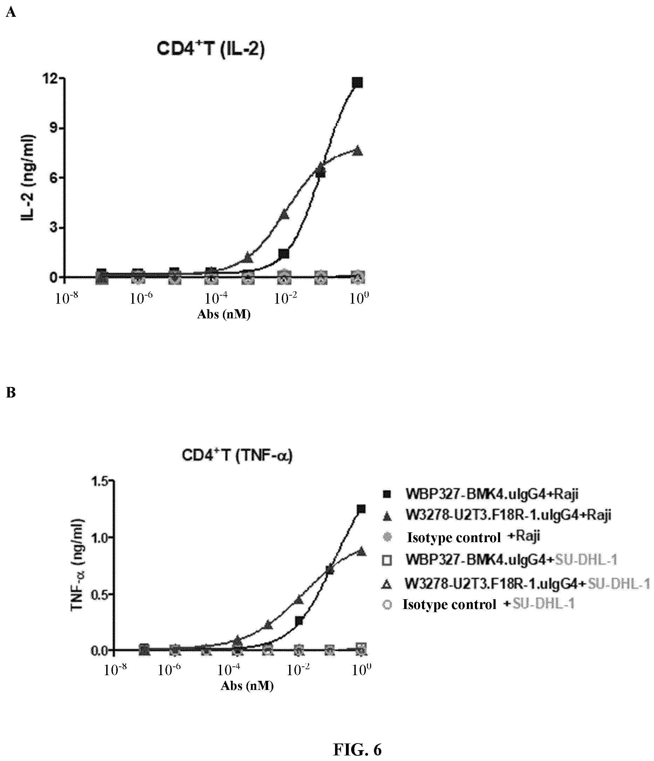

As shown in FIG. 6, W3278 lead Ab did not induce T cells' cytokine release in the presence of CD20 negative SU-DHL-1 cells. Only in the presence of target Raji cells was W3278 lead Ab able to induce lower levels of cytokine release than BMK4. In addition, W3278 Ab elicited a larger EC₅₀ window (shown as an EC₅₀ ratio of cytokine release to cell killing) than BMK4 (Table 6).

**Table 6. The EC₅₀ of BsAb-mediated CD4+ T cells' cytokine release and the maximum levels, as well as the EC₅₀ ratios of cytokine release to Raji cell killing**

| Ab | TNF-α | | IL-2 | | EC₅₀ ratio TNFα release/ Raji cell killing | EC₅₀ ratio IL-2 release/ Raji cell killing |
|---|---|---|---|---|---|---|
| | EC50 (pM) | Maximum level (ng/mL) | EC50 (pM) | Maximum level (ng/mL) | | |
| WBP327-BMK4.uIgG4 | 131.4 | 1.3 | 113.6 | 11.8 | 6 (131.4pM / 21.49pM) | 5 (113.6pM / 21.49pM) |
| W3278-U2T3.F18R-1.uIgG4.SP | 13.5 | 0.9 | 11.4 | 7.7 | 26 (13.5pM / 0.52pM) | 22 (11.4pM / 0.52pM) |

### 5. Serum stability testing

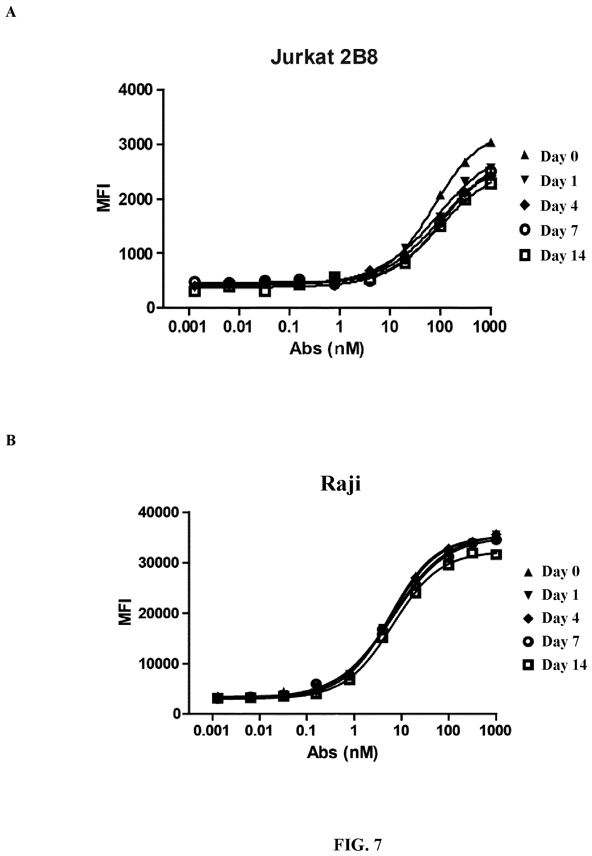

The antibody was mixed with freshly collected human serum and the proportion of serum in the mixture sample was ensured to be >95%. The mixture sample was aliquoted and incubated at 37 °C for 0-14 days. At each time point as shown in FIG. 7, the samples were snap-frozen in liquid nitrogen and stored at -80 °C before analysis. The bindings of each sample to Raji or Jurkat cells was analyzed by FACS.

As shown in FIG. 7, the human serum treated W3278 Ab bound to both Jurkat (FIG. 7A) and Raji (FIG. 7B) cells similarly to the freshly thawed Ab (day 0). These results indicate that W3278 Ab was stable in human serum for at least 14 days (FIG. 7).

### 6. DSF assay and thermal stability testing

A DSF assay was carried out using real-time fluorescent quantitative PCR (QuantStudio 7 Flex, Thermo Fisher Scientific). Briefly, 19 µL of antibody solution was mixed with 1 µL of 62.5 X SYPRO Orange solution (Invitrogen) and the mixture was added to a 96-well plate (Biosystems). The plate was heated from 26 °C to 95 °C at a rate of 2 °C/min and the resulting fluorescence data was collected. The negative derivatives of changes in fluorescence with respect to different temperatures were calculated and the maximum value was defined as melting temperature Tₕ. If a protein has multiple unfolding transitions, the first two Tₕ were reported and designated Tₘ₁ and Tₘ₂. Tₘ₁ was always interpreted as the formal melting temperature Tₘ, for ease of comparison between different proteins. Data acquisition and Tₕ calculation were conducted automatically by the operating software (QuantStudio Real-Time PCR PCR Software v1.3). The Tₘ₁ and Tₘ₂ values for W3278 Ab in different buffers are shown in Table 7. The Tₘ for W3278 was about 61 °C, which suggests the good thermal stability of W3278 Ab.

**Table 7. Thermal stability measured by DSF (Tₘ values are indicated by Tₘ; in the Table)**

| Name of protein | pI | Buffer | Tₘ₁ (°C) | Tₘ₂ (°C) |
|---|---|---|---|---|
| | | PBS | 61.1 | 72.6 |
| W3278-U2T3.F18R-1.uIgG4.SP | 7.42 | 20 mM histidine + 7% sucrose pH 6.5 | 60.3 | 73.6 |
| | | 50 mM NaAC + 7% sucrose pH 5.6 | 61.0 | 74.2 |

### Example 4

### In Vivo Anti-Tumor Efficacy

The antibodies were tested for *in vivo* anti-tumor efficacy in a PBMC humanized NOG mouse model bearing Raji tumor. Female NOG mice (Beijing Vital River Laboratory Animal Technology Co., Ltd.) at 6-8 weeks of age were used in the study. Raji tumor cells (ATCC^{®} CCL-86^{™}) were monolayer cultured in 1640 medium containing 10% fetal bovine serum, 100 U/mL penicillin and 100 µg/mL streptomycin in an incubator at 37 °C with 5% CO₂. The tumor cells were routinely sub-cultured twice weekly. Cells growing in exponential growth phase were collected and counted for tumor inoculation. Human PBMCs were isolated from heparin whole blood of a healthy donor individual using Ficoll-Paque Plus according to the manufacturer's instructions.

As a treatment model, each mouse was given a subcutaneous co-injection of pre-mixed Raji tumor cells (2.0 × 10⁶) and PBMCs (3.0 × 10⁶) in the right upper flank. When the mean tumor volume reached about 60 mm³, the animals were randomized into groups and received their first antibody injections. For the efficacy study, mice received two intravenous injections of the indicated amount of antibody every week over a total of 3 weeks. All procedures involved in animal handling, care and treatment in the study were conducted in accordance with the instructions approved by the Institutional Animal Care and Use Committee (IACUC) of WuXi AppTec followed by the guidance of the Association for Assessment and Accreditation of Laboratory Animal Care (AAALAC). For all tumor studies, mice were weighed and tumor growth was measured twice weekly using calipers. Tumor volumes were estimated as ½ (length × width²).

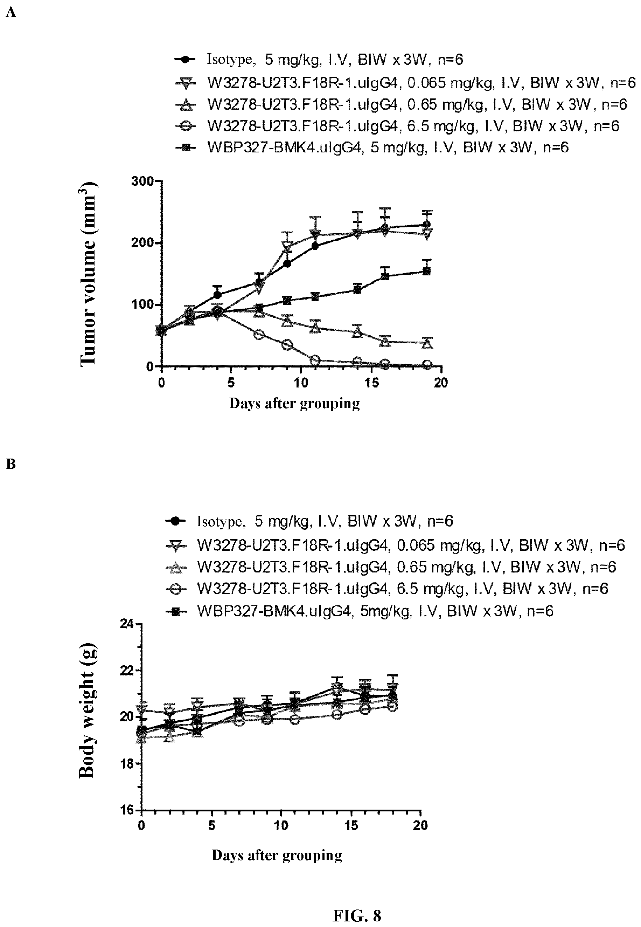

As shown in FIG. 8, W3278 lead Ab treatment demonstrated dose-dependent anti-tumor activity and was more effective than BMK4 treatment (FIG. 8A). The mice had a normal body weight during the experiment (FIG. 8B).

### Example 5

### Single Dose Study of WBP3278 Bispecific Antibody in Cynomolgus Monkey First Used for Immunization

To determine whether treatment with a WBP3278 bispecific antibody can deplete circulating B cells in primates and determine whether any unexpected toxicity will be caused, the inventors conducted an exploratory, non-GLP pharmacological study in cynomolgus monkeys (*Macaca Fascicularis*). Four male cynomolgus monkeys (at 3-4 years of age, weighed about 4 kg) first used for immunization were provided by Guangdong Zhao Qing Chuang Yao Biotechnology Co., Ltd. All procedures related to animal handling, care and treatment in the study were conducted in accordance with the guidelines approved by the Institutional Animal Care and Use Committee (IACUC) of the PharmaLegacy Laboratoryfollowed by the guidance of the Association for Assessment and Accreditation of Laboratory Animal Care (AAALAC).

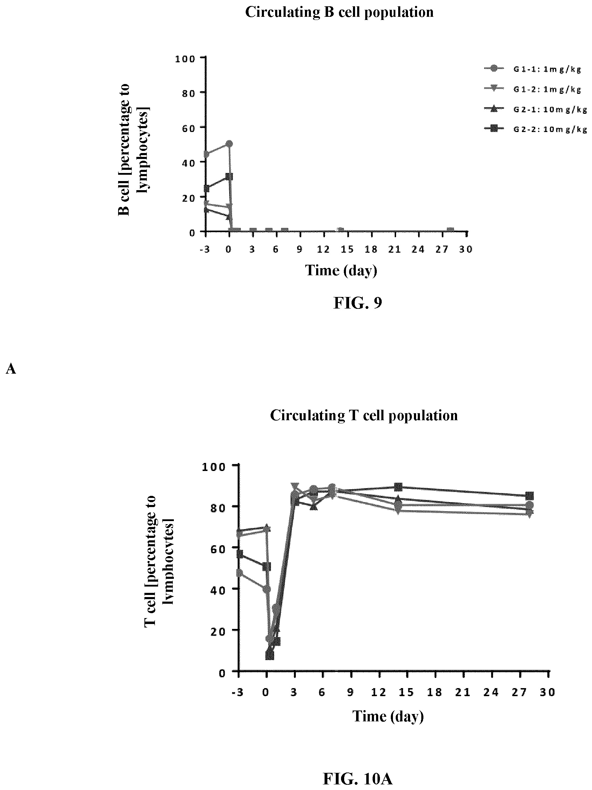

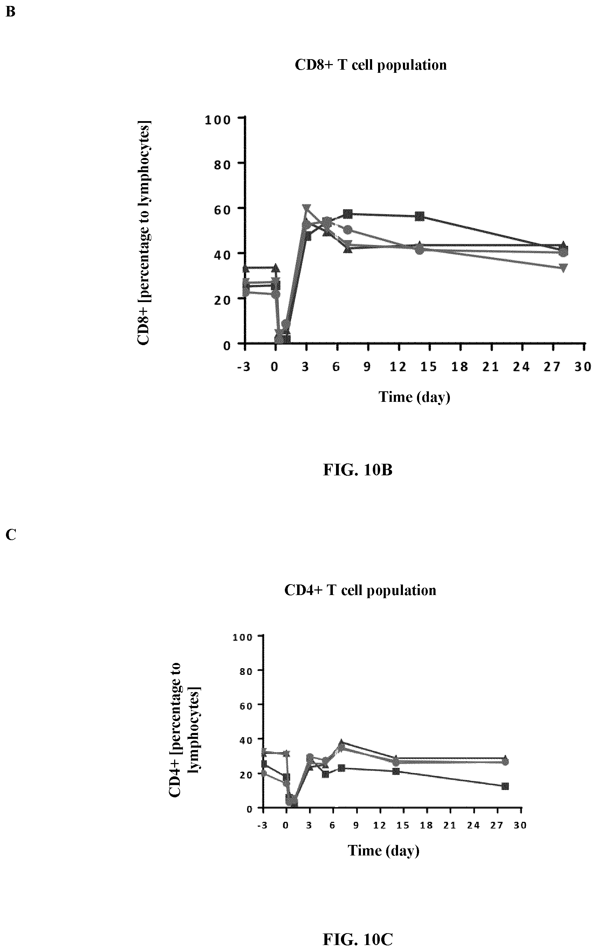

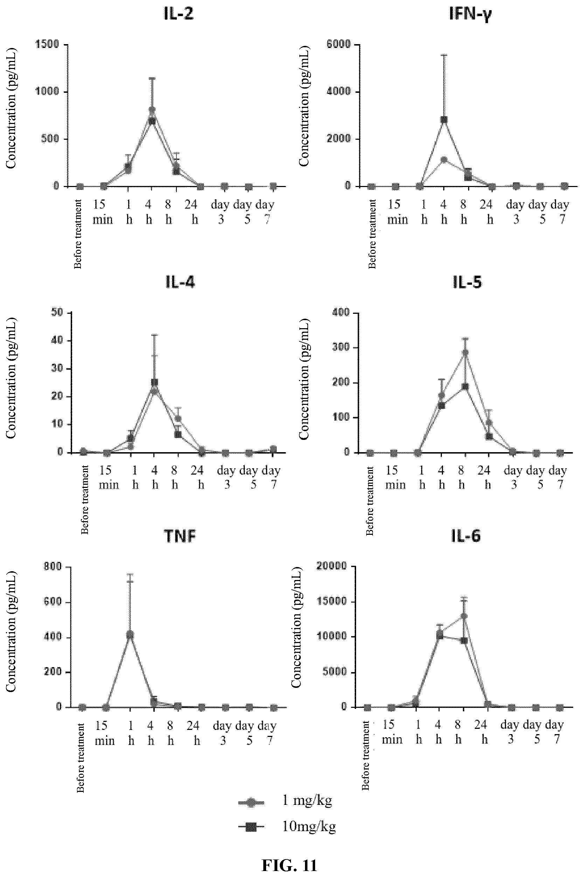

The four animals were divided into two groups (2 animals per group) and were each given one dose of WBP3278 lead antibody (i.e., W3278-U2T3.F18R-1.uIgG4, hereinafter referred to as WBP3278 lead Ab) at 1 mg/kg (group 1) and 10 mg/kg (group 2) by slow intravenous injection within 60 seconds. The levels of circulating B cells and T cells in peripheral blood were monitored by FACS for 4 weeks for the following lymphocytes: B lymphocytes (CD45+/CD20+), T lymphocytes (CD45+/CD3+), CD4⁺ T lymphocytes (CD4+/CD45+/CD3+), and CD8⁺ T lymphocytes (CD8+/CD45+/CD3+). The levels of circulating inflammatory cytokines were analyzed by using a BD^{™} cytometric bead array (CBA) non-human primate Th1/Th2 cytokine kit (BD Bioscience, Cat: 557800). The treatment with WBP3278 lead Ab resulted in immediate and complete depletion of circulating B cells, which lasted at least 4 weeks (FIG. 9). The level of circulating T cells was also reduced at first following the treatment with WBP3278 lead Ab, and returned to the baseline level or to a slightly higher level after 72 h and lasted at least 4 weeks (FIG. 10A). After 72 h, the level of CD8⁺ T cells increased (FIG. 10B) and the level of CD4⁺ T cells returned to the normal level (FIG. 10C), and both lasted at least 4 weeks. After the treatment with WBP3278 lead Ab, rapid increases in the levels of circulating cytokines were observed, and all the levels of cytokines returned to the normal levels after 24 h (FIG. 11).

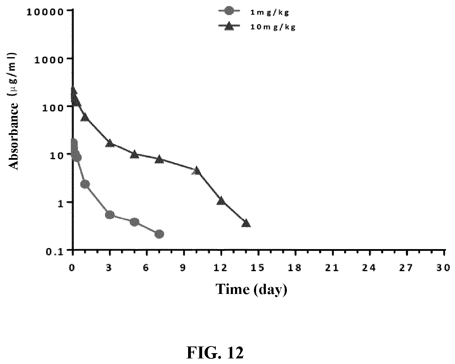

The concentration of WBP3278 lead Ab in serum was determined by ELISA. Briefly, an ELISA plate was coated with anti-human IgG (SouthernBiotech, #2049-01) and then serial dilutions of serum samples were added. The binding signals were detected with goat anti-human IgG biotin (SouthernBiotech, #2049-08) and streptavidin-HRP (Life, #SNN1004). The absorbance was measured at (450-540) nm using a multi-well plate reader (SpectraMax^{®} M5e). Non-compartmental pharmacokinetic analyses were performed on WBP3278 lead Ab concentrations in cynomolgus monkeys using Phoenix WinNonlin software (version 8.1, Pharsight, Mountain View, CA). The linear/log trapezoidal rule was applied in obtaining the PK parameters. Individual BLQ was excluded in the calculation of the mean concentrations. The normal dose levels and the number of nominal samplings were used in the calculation of all the pharmacokinetic parameters. A summary of PK parameters is listed in Table 8 and is shown in FIG. 12. It can be seen that when the dose was increased from 1 mg/kg to 10 mg/kg, the systemic exposure C₀ increased from 19.9 µg/mL to 282 µg/mL (about 14-fold) and AUC₀₋ₗₐₛₜ increased from 259 µg.h/mL to 5788 µg.h/mL (about 22-fold). The serum half-life (T_{1/2}) of the WBP3278 bispecific antibody was 43.3 h and 89.8 h at 1 mg/kg and 10 mg/kg, respectively.

**Table 8. A summary of PK parameters of WBP3278 lead Ab**

| PK parameters | 1 mg/kg (mean) | 10 mg/kg (mean) |
|---|---|---|
| C₀ (µg/mL) | 19.9 | 282 |
| T_{1/2} (h) | 43.3 | 89.8 |
| AUC₀₋ₗₐₛₜ (µg.h/mL) | 259 | 5788 |

In addition, cage-side observations during the experiment showed that no unexpected toxicity was observed for both the high and low dose levels of WBP3278 lead Ab (data not shown).

The results of the experiment show that WBP3278 lead Ab could effectively eliminate B cells *in vivo* without causing adverse events such as cytokine storms, and had sufficient half-life in cynomolgus monkeys. These experimental results support the advancement of the preclinical drug development of WBP3278 lead Ab.

### Examples for Formulations

Size exclusion chromatography-high performance liquid chromatography (SEC-HPLC): for determining the purity of an antibody in a sample. In Example 6, an Agilent 1260 high performance liquid chromatograph and a TSK G3000SWXL (TOSOH) gel chromatography column were adopted; elution was performed with 50 mM phosphate buffered saline + 300 mM sodium chloride (pH 6.8) buffer as the mobile phase, and the detection wavelength was 280 nm. In Examples 7, 8.1, 8.2 and 10, a Waters I-Class high performance liquid chromatograph and an Agilent AdvanceBio SEC 200Å gel chromatography column were adopted; elution was performed with 17.8 mM sodium dihydrogen phosphate + 32.2 mM disodium hydrogen phosphate + 200 mM L-arginine hydrochloride buffer as the mobile phase, and the detection wavelength was 280 nm. The contents of high polymers, main peaks and degradation fragments were calculated in percentage terms using an area normalization method.

Differential scanning calorimetry (DSC): In Examples 6-7, the onset temperature (Tmonset) and unfolding temperature (Tm) of a sample were analyzed using SYS12907 (Malvern). The sample was diluted to a concentration of 1 mg/mL, and the program was as follows: the start temperature of scanning was 10 °C, the end temperature of scanning was 95 °C, and the temperature was raised at a rate of 200 °C/h. In Example 10, the onset temperature (Tmonset) and melting temperature (Tm) of a sample were analyzed using a protein stability analyzer PrometheusNT.48 (NanoTemper). 10 µL of the sample was placed in the sample chamber, and the program was as follows: the start temperature of scanning was 25 °C, the end temperature of scanning was 95 °C, and the temperature was raised at a rate of 0.3 °C/h.

In Example 6, the aggregation temperature (Tagg) of a sample was determined using dynaproplateReaderII (Wyatt). 20 µL of the sample was added to a 384-well sample plate, and 20 µL of paraffin oil was added onto the sample. Then the 384-well sample plate was centrifuged to remove air bubbles. The program was as follows: the heating program was from 25 °C to 60 °C, the scan time was 5 seconds, and 5 scans were performed. In Example 10, the aggregation temperature (Tagg) of a sample was determined using DynaproplateReader III (Wyatt). 30 µL of the sample was added to a 384-well sample plate, and the plate was sealed with sealing film. Then the 384-well sample plate was centrifuged to remove air bubbles. The program was as follows: the heating program was from 25 °C to 85°C, the scan time was 5 seconds, and 5 scans were performed.

Modulated differential scanning calorimetry (mDSC): The glass transition temperature (Tg') of a sample during lyophilization was analyzed using DSC Q2000 (TA Instruments), and the program was as follows: the temperature was reduced to -60 °C, held for 5 min, and increased to 20 °C at a rate of 5 °C/min.

The number of insoluble particles was analyzed using a particle detector GWJ-8 (Tianjin Tianda Tianfa GWJ-8 Technology Co., Ltd.). The number of MFI particles was analyzed using a micro-flow-imaging-particle-analyzer MFI5100 (ProteinSimple), and the data were analyzed using the MVAS software provided with the system.

The water content of a sample was measured using a water content titrator C30 (METTLER TOLEDO). Appearance: The sample flask was wiped clean and then placed under a clarity detector. The illuminance was adjusted to 1500 lx. The solution was visually inspected for appearance and visible foreign matter against a black background and a white background.

### Example 6

### Buffer System and pH Value

WBP3278 lead Ab was exchanged into the target buffer by dialysis, with a protein concentration of 2 mg/mL. Filtration was performed using a 0.22 µm PVDF filter membrane in a biosafety cabinet, and the filtrate was aliquoted into 2 mL vials at a strength of 2 mg/mL/vial. The vials were stoppered and capped. Stability tests were performed at 35 °C, and the Tm, Tagg and SEC-HPLC measurements are shown in Table 9, Table 10 and Table 11, respectively.

**Table 9. Tm measurements**

| Sample | TmOnset (°C) | Tm1 (°C) | Tm2 (°C) | Tm3 (°C) |
|---|---|---|---|---|
| 20 mM citric acid-sodium citrate, pH 5.0 | 49.4 | 57.8 | 64.4 | 75.6 |
| 20 mM citric acid-sodium citrate, pH 5.5 | 53.1 | 62.3 | 75.5 | - |
| 20 mM citric acid-sodium citrate, pH 6.0 | 55.1 | 64.4 | 75.3 | - |
| 20 mM citric acid-sodium citrate, pH 6.5 | 56.0 | 65.1 | 75.2 | - |
| 10 mM citric acid-sodium citrate, pH 6.0 | 55.2 | 64.2 | 75.0 | - |
| 20 mM histidine-histidine hydrochloride, pH 5.5 | 49.9 | 58.9 | 65.1 | 77.1 |
| 20 mM histidine-histidine hydrochloride, pH 6.0 | 52.4 | 61.8 | 77.0 | - |
| 20 mM histidine-histidine hydrochloride, pH 6.5 | 53.0 | 62.2 | 75.4 | - |

| | | | | |
|---|---|---|---|---|
| Note: "-" indicates undetectable. | | | | |

**Table 10. Tagg measurements**

| Sample | SLS (°C) | DLS (°C) |
|---|---|---|
| 20 mM citric acid-sodium citrate, pH 5.0 | 40.87 | 40.30 |
| 20 mM citric acid-sodium citrate, pH 5.5 | 45.63 | 40.34 |
| 20 mM citric acid-sodium citrate, pH 6.0 | 38.48 | 39.93 |
| 20 mM citric acid-sodium citrate, pH 6.5 | 40.15 | 41.01 |
| 10 mM citric acid-sodium citrate, pH 6.0 | 49.55 | 47.81 |
| 20 mM histidine-histidine hydrochloride, pH 5.5 | 41.49 | 41.04 |
| 20 mM histidine-histidine hydrochloride, pH 6.0 | 37.63 | 37.69 |
| 20 mM histidine-histidine hydrochloride, pH 6.5 | 36.54 | 39.55 |

**Table 11. SEC-HPLC measurements**

| Sample | Sampling points | SEC-HPLC | | |
|---|---|---|---|---|
| | | High polymer% | Main peak% | Fragment% |
| 20 mM citric acid-sodium citrate, pH 5.0 | T0 | 0.8 | 99.2 | ND |
| | 35 °C for 1 week | 8.2 | 91.8 | ND |
| | 35 °C for 2 weeks | 10.4 | 89.6 | ND |
| | 35 °C for 4 weeks | 17.9 (+17.1) | 80.3 | 1.6 |
| 20 mM citric acid-sodium citrate, pH 5.5 | T0 | 2.1 | 97.9 | ND |
| | 35 °C for 1 week | 3.0 | 97.0 | ND |
| | 35 °C for 2 weeks | 2.8 | 97.2 | ND |
| | 35 °C for 4 weeks | 3.8 (+1.7) | 96.2 | ND |
| 20 mM citric acid-sodium citrate, pH 6.0 | T0 | 0.9 | 99.1 | ND |
| | 35 °C for 1 week | 1.3 | 98.7 | ND |
| | 35 °C for 2 weeks | 1.4 | 98.6 | ND |
| | 35 °C for 4 weeks | 1.5 (+0.6) | 98.5 | ND |
| 20 mM citric acid-sodium citrate, pH 6.5 | T0 | 0.9 | 99.1 | ND |
| | 35 °C for 1 week | 0.9 | 99.1 | ND |
| | 35 °C for 2 weeks | 1.0 | 99.0 | ND |
| | 35 °C for 4 weeks | 1.3 (+0.4) | 98.6 | 0.1 |
| 10 mM citric acid-sodium citrate, pH 6.0 | T0 | 0.5 | 99.5 | ND |
| | 35 °C for 1 week | 0.8 | 99.2 | ND |
| | 35 °C for 2 weeks | 0.8 | 99.2 | ND |
| | 35 °C for 4 weeks | 1.1 (+0.6) | 98.9 | ND |
| 20 mM histidine-histidine hydrochloride, pH 5.5 | T0 | 1.4 | 98.6 | ND |
| | 35 °C for 1 week | 3.2 | 96.8 | ND |
| | 35 °C for 2 weeks | 3.9 | 96.1 | ND |
| | 35 °C for 4 weeks | 4.9 (+3.5) | 95.1 | ND |
| 20 mM histidine-histidine | T0 | 0.4 | 99.6 | ND |
| hydrochloride, pH 6.0 | 35 °C for 1 week | 1.1 | 98.9 | ND |
| | 35 °C for 2 weeks | 1.5 | 98.5 | ND |
| | 35 °C for 4 weeks | 2.3 (+1.9) | 97.7 | ND |
| 20 mM histidine-histidine hydrochloride, pH 6.5 | T0 | 0.2 | 99.8 | ND |
| | 35 °C for 1 week | 0.9 | 99.1 | ND |
| | 35 °C for 2 weeks | 1.2 | 98.8 | ND |
| | 35 °C for 4 weeks | 1.6 (+1.4) | 98.4 | ND |

| | | | | |
|---|---|---|---|---|
| Note: "T0" represents time 0; "ND" represents undetectable; (+X) represents an increase of X relative to T0 and (-X) represents a decrease of X relative to T0; for example, high polymer = 17.9 (+17.1) indicates high polymer (17.9%) with an increase of 17.1% relative to T0. | | | | |

### Example 7

### Sugar and Surfactant

1 kg of 10 mM citric acid-sodium citrate buffer (pH 6.2) was prepared as follows: 0.282 g of citric acid monohydrate (C₆H₈O₇•H₂O), 2.547 g of sodium citrate dihydrate (C₆H₅Na₃O₇•2H₂O) were weighed out, and water was added until the buffer reaches 1 kg, and the buffer was filtered.

WBP3278 lead Ab was exchanged into the 10 mM citric acid-sodium citrate buffer prepared above by dialysis. A sucrose mother solution, a trehalose dihydrate mother solution or mannitol mother solution was added, and a Tween 80 mother solution was added to prepare the target formulation with a protein concentration of 2 mg/mL, which was then filtered through a 0.22 µm PVDF filter membrane in a biosafety cabinet and aliquoted into 4 mL vials at a strength of 2 mg/mL/vial. The vials were stoppered and capped. 8% (w/v) sucrose refers to 80 mg/mL sucrose; 8.8% (w/v) trehalose dihydrate refers to 88 mg/mL trehalose dihydrate; 0.02% (w/v) Tween 80 refers to 0.2 mg/mL Tween 80. The Tm measurements and the SEC-HPLC and pH measurements of the three formulations are shown in Table 12 and Table 13, respectively.

**Table12. Tm measurements**

| Sample | TmOnset(°C ) | Tm1 (°C) | Tm2 (°C) |
|---|---|---|---|
| 2 mg/mL WBP3278 lead Ab, 10 mM citric acid-sodium citrate pH 6.2, 8% (w/v) sucrose, 0.02% (w/v) Tween 80 | 56.7 | 65.5 | 76.5 |
| 2 mg/mL WBP3278 lead Ab, 10 mM citric acid-sodium citrate pH 6.2, 8.8% (w/v) trehalose dihydrate, 0.02% (w/v) Tween 80 | 56.8 | 65.7 | 76.7 |
| 2 mg/mL WBP3278 lead Ab, 10 mM citric acid-sodium citrate pH 6.2, 2% (w/v) sucrose, 4% (w/v) mannitol, 0.02% (w/v) Tween 80 | 57.5 | 65.6 | 76.6 |

**Table 13. SEC-HPLC and pH measurements**

| Sample | Sampling points SEC-HPLC | | | | pH |
|---|---|---|---|---|---|
| | High polymer% | | Main peak% | Fragment% | |
| 2 mg/mL WBP3278 lead Ab, 10 mM citric acid-sodium citrate pH 6.2, 8% (w/v) sucrose, 0.02% (w/v) Tween 80 | T0 | 1.9 | 97.5 | 0.6 | 6.2 |
| | 40 °C for 2 weeks | 3.1 | 95.9 | 1.0 | 6.2 |
| | 40 °C for 4 weeks | 4.8 (+2.9) | 93.9 (-3.6) | 1.3 | 6.2 |
| | 3 rounds of freezing and thawing | 2.2 | 97.3 | 0.4 | 6.2 |
| | 5 rounds of freezing and thawing | 1.9 | 97.7 | 0.4 | 6.2 |
| | 1 day of shaking | 1.9 | 97.6 | 0.5 | 6.2 |
| | 3 days of shaking | 1.8 | 97.7 | 0.5 | 6.2 |
| 2 mg/mL WBP3278 lead Ab, 10 mM citric acid-sodium citrate pH 6.2, 8.8% (w/v) trehalose dihydrate, 0.02% (w/v) Tween 80 | T0 | 1.9 | 97.5 | 0.6 | 6.2 |
| | 40 °C for 2 weeks | 3.1 | 96.0 | 0.9 | 6.2 |
| | 40 °C for 4 weeks | 4.8 (+2.9) | 93.8 (-3.7) | 1.3 | 6.2 |
| | 3 rounds of freezing and thawing | 2.1 | 97.4 | 0.4 | 6.2 |
| | 5 rounds of freezing and thawing | 1.9 | 97.6 | 0.5 | 6.2 |
| | 1 day of shaking | 1.9 | 97.5 | 0.6 | 6.2 |
| | 3 days of shaking | 1.7 | 97.7 | 0.5 | 6.2 |
| 2 mg/mL WBP3278 lead Ab, 10 mM citric acid-sodium citrate pH 6.2, 2% (w/v) sucrose, 4% (w/v) mannitol, 0.02% (w/v) Tween 80 | T0 | 1.8 | 97.6 | 0.6 | 6.2 |
| | 40 °C for 2 weeks | 3.1 | 96.0 | 0.9 | 6.2 |
| | 40 °C for 4 weeks | 4.9 (+3.1) | 93.8 (-3.8) | 1.3 | 6.2 |
| | 3 rounds of freezing and thawing | 2.0 | 97.6 | 0.4 | 6.2 |
| | 5 rounds of freezing and thawing | 1.9 | 97.7 | 0.5 | 6.2 |
| | 1 day of shaking | 1.8 | 97.7 | 0.5 | 6.2 |
| | 3 days of shaking | 1.7 | 97.7 | 0.5 | 6.2 |

| | | | | | |
|---|---|---|---|---|---|
| Note: "T0" represents time 0; the freezing and thawing were performed at -70 °C to room temperature. | | | | | |

### Example 8.1

### Sugar and Surfactant

WBP3278 lead Ab was exchanged into 10 mM citric acid-sodium citrate buffer (prepared according to the preparation method shown in Example 7) by dialysis. A sucrose mother solution, a trehalose dihydrate mother solution or mannitol mother solution was added, and a Tween 80 mother solution was added to prepare the target formulation with a protein concentration of 2 mg/mL, which was then filtered through a 0.22 µm PVDF filter membrane in a biosafety cabinet and aliquoted into 4 mL vials at a strength of 2 mg/mL/vial. The vials were half stoppered and then placed in a freeze-dryer for lyophilization. The lyophilization program was as follows: pre-freezing, primary drying and secondary drying. The procedure is, for example, shown in Table 14. The three formulations were reconstituted from water, and the appearance, reconstitution time and water content of the reconstituted formulations are shown in Table 15. The Tg' and SEC-HPLC measurements are shown in Table 16 and Table 17, respectively.

**Table 14. Lyophilization procedure**

| Program | | Shelf temperature (°C) | Rate (°C/min) | Holding time (min) | Cabinet pressure (mbar) |
|---|---|---|---|---|---|
| Pre-freezing | 1 | 5 | 1 | 30 | Atmospheric pressure |
| | 2 | -5 | 1 | 30 | Atmospheric pressure |
| | 3 | -45 | 1 | 60 | Atmospheric pressure |
| | 4 | -22 | 1 | 180 | Atmospheric pressure |
| | 5 | -45 | 1 | 90 | Atmospheric pressure |
| Primary drying | 6 | -33 | 1 | 80 | 0.1 |
| | 7 | -28 | 1 | 1410 | 0.1 |
| Secondary drying | 8 | 35 | 0.3 | 240 | 0.1 |

**Table 15. The appearance, reconstitution time and water content measurements of reconstituted lyophilized formulations**

| Sample | Sampling points | Appearance after reconstitution | Reconstitution time | Water content % |
|---|---|---|---|---|
| 2 mg/mL WBP3278 lead Ab, 10 mM citric acid-sodium citrate pH 6.2, 8% (w/v) sucrose, 0.02% (w/v) Tween 80 | T0 | Colorless, clear and free of visible foreign matter | 28 s | 1.1 |
| | 40 °C for 2 weeks | | 34 s | 1.1 |
| | 40 °C for 4 weeks | | 23 s | 1.0 |
| 2 mg/mL WBP3278 lead Ab, 10 mM citric acid-sodium citrate pH 6.2, 8.8% (w/v) trehalose dihydrate, 0.02% (w/v) Tween 80 | T0 | Colorless, clear and free of visible foreign matter | 31 s | 0.7 |
| | 40 °C for 2 weeks | | 22 s | 0.8 |
| | 40 °C for 4 weeks | | 36 s | 0.4 |
| 2 mg/mL WBP3278 lead Ab, 10 mM citric acid-sodium citrate pH 6.2, 2% (w/v) sucrose, 4% (w/v) mannitol, 0.02% (w/v) Tween 80 | T0 | Colorless, clear and free of visible foreign matter | 2 min 10 s | 1.2 |
| | 40 °C for 2 weeks | Colorless, slightly opalescent and free of visible foreign matter | 1 min 26 s | 1.2 |
| | 40 °C for 4 weeks | Colorless, slightly opalescent and free of visible foreign matter | 2 min 01 s | 1.1 |

| | | | | |
|---|---|---|---|---|
| Note: "T0" represents time 0. | | | | |

**Table 16. Tg' measurements**

| Sample | Tg'(°C) |
|---|---|
| 2 mg/mL WBP3278 lead Ab, 10 mM citric acid-sodium citrate pH 6.2, 8% (w/v) sucrose, 0.02% (w/v) Tween 80 | -34.4 |
| 2 mg/mL WBP3278 lead Ab, 10 mM citric acid-sodium citrate pH 6.2, 8.8% (w/v) trehalose dihydrate, 0.02% (w/v) Tween 80 | -31.8 |
| 2 mg/mL WBP3278 lead Ab, 10 mM citric acid-sodium citrate pH 6.2, 2% (w/v) sucrose, 4% (w/v) mannitol, 0.02% (w/v) Tween 80 | -36.5 |

**Table 17. SEC-HPLC and pH measurements**

| Sample | Sampling points | SEC-HPLC | | | pH |
|---|---|---|---|---|---|
| | | High polymer% | Main peak% | Fragment% | |
| 2 mg/mL WBP3278 lead Ab, 10 mM citric | T0 | 1.9 | 97.6 | 0.6 | 6.2 |
| acid-sodium citrate pH 6.2, 8% (w/v) sucrose, 0.02% (w/v) Tween 80 | 40 °C for 2 weeks | 2.1 | 97.5 | 0.4 | 6.2 |
| | 40 °C for 4 weeks | 2.1 | 97.4 | 0.4 | 6.2 |
| 2 mg/mL WBP3278 lead Ab, 10 mM citric acid-sodium citrate pH 6.2, 8.8% (w/v) trehalose dihydrate, 0.02% (w/v) Tween 80 | T0 | 1.9 | 97.6 | 0.6 | 6.2 |
| | 40 °C for 2 weeks | 2.1 | 97.5 | 0.4 | 6.2 |
| | 40 °C for 4 weeks | 2.3 | 97.2 | 0.4 | 6.2 |
| 2 mg/mL WBP3278 lead Ab, 10 mM citric acid-sodium citrate pH 6.2, 2% (w/v) sucrose, 4% (w/v) mannitol, 0.02% (w/v) Tween 80 | T0 | 2.2 | 97.2 | 0.6 | 6.2 |
| | 40 °C for 2 weeks | 2.9 | 96.7 | 0.4 | 6.2 |
| | 40 °C for 4 weeks | 3.1 | 96.5 | 0.4 | 6.2 |

| | | | | | |
|---|---|---|---|---|---|
| Note: "T0" represents time 0. | | | | | |

### Example 8.2

### Sugar and Surfactant

WBP3278 lead Ab was exchanged into 10 mM citric acid-sodium citrate buffer (prepared according to the preparation method shown in Example 7) by ultrafiltration. A sucrose mother solution or a trehalose dihydrate mother solution was added, and a Tween 80 mother solution was added to prepare the target formulation with a protein concentration of 2 mg/mL, which was then subjected to two-stage filtration through a 0.22 µm PVDF filter membrane and aliquoted into 6 mL vials at a strength of 2 mg/mL/vial. The vials were half stoppered and then placed in a freeze-dryer for lyophilization. The procedure is, for example, shown in Table 14. The SEC-HPLC measurements of the two formulations are shown in Table 18. Furthermore, MFI particles and insoluble particles in both formulations were in the normal range, but MFI particles (in a range of 2 ≤ X < 5 µm) in the sucrose-containing formulation far more than in the trehalose-containing formulation, and insoluble particles (in a range of 2 ≤ X < 5 µm) in the the sucrose-containing formulation also slightly more than in the trehalose-containing formulation. After being stored at 40 °C for 4 weeks, at 25 °C for 6 months and at 2-8 °C for 6 months, neither of the formulations showed significant changes in particles.

**Table 18. SEC-HPLC measurements**

| Sample | Sampling points | SEC-HPLC | | | pH |
|---|---|---|---|---|---|
| | | High polymer% | Main peak% | Fragment% | |
| 2 mg/mL WBP3278 lead Ab, 10 mM citric acid-sodium citrate pH 6.2, 8% (w/v) sucrose, 0.02% (w/v) Tween 80 | T0 | 1.9 | 95.1 | 2.9 | 6.2 |
| | 40 °C for 2 weeks | 2.0 | 95.1 | 2.8 | 6.2 |
| | 40 °C for 4 weeks | 2.1 | 94.8 | 3.1 | 6.2 |
| | 25 °C for 1 month | 2.2 | 94.7 | 3.2 | 6.2 |
| | 25 °C for 6 months | 2.1 | 94.8 | 3.1 | 6.2 |
| | 2-8 °C for 1 month | 2.1 | 94.7 | 3.2 | 6.2 |
| | 2-8 °C for 6 months | 1.9 | 95.0 | 3.0 | 6.2 |
| 2 mg/mL WBP3278 lead Ab, 10 mM citric acid-sodium citrate pH 6.2, 8.8% (w/v) trehalose dihydrate, 0.02% (w/v) Tween 80 | T0 | 2.1 | 95.1 | 2.8 | 6.2 |
| | 40 °C for 2 weeks | 2.4 | 94.6 | 3.0 | 6.2 |
| | 40 °C for 4 weeks | 2.4 | 94.4 | 3.2 | 6.2 |
| | 25 °C for 1 month | 2.2 | 94.6 | 3.2 | 6.2 |
| | 25 °C for 6 months | 2.2 | 94.7 | 3.1 | 6.2 |
| | 2-8 °C for 1 month | 2.1 | 94.6 | 3.3 | 6.2 |
| | 2-8 °C for 6 months | 2.0 | 95.0 | 3.0 | 6.2 |

| | | | | | |
|---|---|---|---|---|---|
| Note: "T0" represents time 0. | | | | | |

### Example 9

### Biological activity

Biological activity assays were carried out by *in vitro* T cell activation experiments. Raji cells (CD3- and CD20+ human B cell lines) were used as target cells and luciferase-expressing engineered Jurkat cells (CD3+ and CD20-human T cell lines) as effector cells. When the CD3 binding portion and CD20 binding portion of WBP3278 lead Ab bound to Raji cells and Jurkat cells, respectively, Jurkat cells would be activated and downstream signal transduction would be caused, leading to luciferase expression. The fluorescence signals were detected by a detection reagent Bio-Glo. A reference sample (a formulation sample from a batch representative of the process) and a test sample (a formulation sample to be tested) were diluted by gradient dilution (4-fold gradient dilution from an initial concentration of 9000 ng/mL, with a total of 11 dilution gradients), and then the dilutions were added to a 96-well cell culture plate at 25 µL/well. Raji cells and Jurkat cells growing at log phase were adjusted to 2 × 10⁶ cells/mL, mixed in equal volume, and added to the above 96-well cell culture plate at 50 µL/well. The plate was incubated in a 37 °C, 5% CO₂ carbon dioxide cell incubator for 4-5 h. A Bio-Lite Luciferase reagent was added at 75 µL/well, and the plate was incubated at room temperature in the dark for 3-5 min. The RLU values were read using the chemiluminescence detection module of a multifunctional plate reader. Biological activity of test sample (%) = (EC₅₀ value of reference sample/EC₅₀ value of test sample) × 100%

The experimental results show that WBP3278 lead Ab in the lyophilized formulation still retained good biological activity after being stored at 40 °C for 4 weeks, at 25 °C for 6 months and at 2-8 °C for 6 months.

**Table 19. Biological activity assay results**

| Sample | Sampling points | Biological activity (%) |
|---|---|---|
| A lyophilized formulation containing 2 mg/mL WBP3278 lead Ab, 10 mM citric acid-sodium citrate pH 6.2, 8.8% (w/v) trehalose dihydrate, and 0.02% (w/v) Tween 80 | T0 | 114 |
| | 40 °C for 2 weeks | 110 |
| | 40 °C for 4 weeks | 96 |
| | 25 °C for 1 month | 105 |
| | 25 °C for 6 months | 109 |
| | 2-8 °C for 1 month | 103 |
| | 2-8 °C for 6 months | 108 |

| | | |
|---|---|---|
| Note: "T0" represents time 0. | | |

### Example 10

WBP3278 lead Ab was exchanged into a citric acid-sodium citrate buffer (prepared according to the preparation method shown in Example 7) by ultrafiltration. An osmotic pressure regulator and/or a stabilizer mother solution were added, and a Tween 80 mother solution was added to prepare the target preparation (as shown in Table 20), which was then subjected to two-stage filtration through a 0.22 µm PVDF filter membrane and aliquoted into 6 mL neutral borosilicate glass tubular injection vials at a strength of 1 mL/vial. The vials were stoppered and capped. The Tm and Tagg measurements of the different formulations are shown in Table 21. The appearance and SEC-HPLC measurements are shown in Table 22. Furthermore, the biological activities of all the formulations are within the normal range (see Example 9 for the assay method).

**Table 20. Components of formulations**

| No. | Protein concentration | Buffer | Surfactant | Osmotic pressure regulator and/or stabilizer | Osmotic pressure regulator and/or stabilizer |
|---|---|---|---|---|---|
| F5-1 | 2mg/mL | 10 mM citric acid-sodium citrate pH 6.2 | 0.02% (w/v) Polysorbate 80 | 8.8% (w/v) Trehalose dihydrate | / |
| F5-2 | 5mg/mL | 10 mM citric acid-sodium citrate pH 6.0 | | | 6 mg/mL Sodium chloride |
| F5-3 | | 10 mM citric acid-sodium citrate pH6.0 | | | 100 mmol/L Arginine hydrochloride |
| F5-4 | | 20 mM citric acid-sodium citrate pH 6.0 | | | / |
| F5-5 | 10mg/mL | 10 mM citric acid-sodium citrate pH 6.0 | | | 6 mg/mL Sodium chloride |
| F5-6 | | 10 mM citric acid-sodium citrate pH 6.0 | | | 100 mmol/L Arginine hydrochloride |
| F5-7 | | 20 mM citric acid-sodium citrate pH 6.0 | | | / |

| | | | | | |
|---|---|---|---|---|---|
| Note: / represents none. | | | | | |

**Table 21. Tm and Tagg measurements**

| No. | Tm (°C) | | | Tagg (°C) | |
|---|---|---|---|---|---|
| | TmOnset | Tm1 | Tm2 | DLS | SLS |
| F5-1 | 53.2 | 59.3 | 71.3 | 51.6 | 51.5 |
| F5-2 | 52.9 | 59.4 | 71.7 | 51.6 | 51.6 |
| F5-3 | 51.8 | 58.7 | 71.9 | 51.0 | 51.2 |
| F5-4 | 53.4 | 59.6 | 71.4 | 51.3 | 51.5 |
| F5-5 | 53.0 | 59.4 | 71.4 | 51.2 | 50.3 |
| F5-6 | 52.1 | 58.6 | 71.5 | 51.0 | 50.8 |
| F5-7 | 53.5 | 59.4 | 71.5 | 51.0 | 51.4 |

**Table 22. Appearance and SEC-HPLC measurements**

| No. | Sampling points | Appearance | SEC-HPLC | |
|---|---|---|---|---|
| | | | High polymer (%) | Main peak (%) |
| F5-1 | Day 0 | Colorless, clear and free of visible foreign matter | 1.71 | 95.67 |
| | 40 °C for 2 weeks | | 1.88 | 95.99 |
| | 40 °C for 4 weeks | | 2.56 | 92.27 |
| | 2-8 °C for 1 month | | \ | \ |
| | 2-8 °C for 3 months | | \ | \ |
| | 2-8 °C for 6 months | | \ | \ |
| F5-2 | Day 0 | Colorless, clear and free of visible foreign matter | 2.49 | 95.16 |
| | 40 °C for 2 weeks | Colorless, clear and free of visible foreign matter | 2.21 | 95.90 |
| | 40 °C for 4 | Colorless, clear, 1 particle | 3.11 | 91.89 |

| | Sampling No. points | Appearance | SEC-HPLC | |
|---|---|---|---|---|
| | | | High polymer (%) | Main peak (%) |
| | weeks | | | |
| | 2-8 °C for 1 month | Colorless, clear and free of visible foreign matter | \ | \ |
| | 2-8 °C for 3 months | Colorless, clear and free of visible foreign matter | \ | \ |
| | 2-8 °C for 6 months | Colorless, clear and free of visible foreign matter | \ | \ |
| F5-3 | Day 0 | Colorless, clear and free of visible foreign matter | 2.36 | 95.31 |
| | 40 °C for 2 weeks | Colorless, clear and free of visible foreign matter | 2.26 | 95.81 |
| | 40 °C for 4 weeks | Colorless, clear, 1 particle | 3.57 | 91.39 |
| | 2-8 °C for 1 month | Colorless, clear and free of visible foreign matter | \ | \ |
| | 2-8 °C for 3 months | Colorless, clear and free of visible foreign matter | \ | \ |
| | 2-8 °C for 6 months | Colorless, clear and free of visible foreign matter | \ | \ |
| F5-4 | Day 0 | Colorless, clear and free of visible foreign matter | 2.36 | 95.28 |
| | 40 °C for 2 weeks | Colorless, clear and free of visible foreign matter | 2.22 | 95.74 |
| | 40 °C for 4 weeks | Colorless, clear, 1 particle | 3.12 | 91.77 |
| | 2-8 °C for 1 month | Colorless, clear and free of visible foreign matter | \ | \ |
| | 2-8 °C for 3 months | Colorless, clear and free of visible foreign matter | \ | \ |
| | 2-8 °C for 6 months | Colorless, slightly opalescent, with a few particles | \ | \ |

| | | | | |
|---|---|---|---|---|
| Note: "\" indicates no detection. | | | | |

## Claims

1. A liquid formulation comprising
(a) a bispecific anti-CD3/CD20 polypeptide complex wherein the bispecific anti-CD3/CD20 polypeptide complex is at a concentration of about 0.1 mg/mL to about 50 mg/mL
(b) a buffer, wherein the buffer comprises a histidine salt buffer, a succinate buffer or a citrate buffer wherein the buffer has a concentration of about 1 mM to about 30 mM;
(c) a saccharide, wherein the saccharide is at a concentration of 150 mM to about 320 mM; and
(d) a polysorbate 80 or a polysorbate 20, wherein the polysorbate 80 or polysorbate 20 is at a concentration of about 0.001% to about 0.1% (w/v);
wherein the term "about" means +/-5%,
wherein the bispecific anti-CD3/CD20 polypeptide complex comprises a first antigen-binding portion associated with a second antigen-binding portion, wherein:
the first antigen-binding portion comprises:
a first polypeptide, comprising from N-terminus to C-terminus a first heavy chain variable domain (VH) of a first antibody operably linked to a first T Cell Receptor (TCR) constant region (C1), and
a second polypeptide, comprising from N-terminus to C-terminus a first light chain variable domain (VL) of the first antibody operably linked to a second TCR constant region (C2),
wherein:
C1 and C2 can form a dimer comprising at least one non-native interchain bond between C1 and C2, and the non-native interchain bond can stabilize the dimer, wherein C1 comprise an engineered Cβ comprising SEQ ID NO: 61, and C2 comprise an engineered Cα comprising SEQ ID NO: 62,
and
the second antigen-binding portion comprises:
a second heavy chain variable domain (VH2) of a second antibody operably linked to an antibody heavy chain CH1 domain, and
a second light chain variable domain (VL2) of the second antibody operably linked to an antibody light chain constant (CL) domain,
wherein:
one of the first antigen-binding portion and the second antigen-binding portion is an anti-CD3 binding portion, and the other one is an anti-CD20 binding portion;
the anti-CD3 binding portion is derived from an anti-CD3 antibody, comprising:
a) heavy chain CDR1 comprising an amino acid sequence of SEQ ID NO: 13,
b) heavy chain CDR2 comprising an amino acid sequence of SEQ ID NO: 14,
c) heavy chain CDR3 comprising an amino acid sequence of SEQ ID NO: 15,
d) κ light chain CDR1 comprising an amino acid sequence of SEQ ID NO: 16,
e) κ light chain CDR2 comprising an amino acid sequence of SEQ ID NO: 17, and
f) κ light chain CDR3 comprising an amino acid sequence of SEQ ID NO: 18, the anti-CD20 binding portion is derived from an anti-CD20 antibody, comprising:
a) heavy chain CDR1 comprising an amino acid sequence of SEQ ID NO: 19,
b) heavy chain CDR2 comprising an amino acid sequence of SEQ ID NO: 20,
c) heavy chain CDR3 comprising an amino acid sequence of SEQ ID NO: 21,
d) κ light chain CDR1 comprising an amino acid sequence of SEQ ID NO: 22,
e) κ light chain CDR2 comprising an amino acid sequence of SEQ ID NO: 23, and
f) κ light chain CDR3 comprising an amino acid sequence of SEQ ID NO: 24.

2. The liquid formulation according to claim 1, wherein
the anti-CD3 binding portion comprises: a heavy chain variable domain sequence comprising a sequence of SEQ ID NO: 27, and a light chain variable domain sequence comprising a sequence of SEQ ID NO: 28; or a heavy chain variable domain sequence comprising a sequence of SEQ ID NO: 27 with C-terminal deletions of amino acids SS, and a light chain variable domain sequence comprising a sequence of SEQ ID NO: 28,
the anti-CD20 binding portion comprises: a heavy chain variable domain sequence comprising a sequence of SEQ ID NO: 31, and a light chain variable domain sequence comprising a sequence of SEQ ID NO: 32.

3. The liquid formulation according to claim 1 or 2, wherein the first antigen-binding portion is linked to a first dimerization domain, the second antigen-binding portion is linked to a second dimerization domain, and the first dimerization domain and the second dimerization domain are associated;
optionally, the association is via a linker, a disulfide bond, a hydrogen bond, an electrostatic interaction, a salt bridge or a hydrophobic-hydrophilic interaction or a combination thereof;
optionally, the first dimerization domain and/or the second dimerization domain comprise at least a portion of an antibody hinge region, which is optionally from IgG1, IgG2 or IgG4;
optionally, the first dimerization domain and/or the second dimerization domain comprise an antibody CH2 domain and/or an antibody CH3 domain;
optionally, the first dimerization domain is operably linked to the first TCR constant region (C1) at a third junction domain;
optionally, the second dimerization domain is operably linked to the C-terminus of the antibody CH1 constant region of the second antigen-binding portion.

4. The liquid formulation according to any one of claims 1-3, wherein the bispecific anti-CD3/CD20 polypeptide complex comprises a combination of four polypeptide sequences: SEQ ID NO: 41, SEQ ID NO: 42, SEQ ID NO: 43 and SEQ ID NO: 44.

5. The liquid formulation according to any one of claims 1-4, wherein the bispecific anti-CD3/CD20 polypeptide complex comprises five polypeptide chains: a) a first polypeptide chain having a sequence as set forth in SEQ ID NO: 41; b) a second polypeptide chain having a sequence as set forth in SEQ ID NO: 42; c) a third polypeptide chain having a sequence as set forth in SEQ ID NO: 43; d) a fourth polypeptide chain having a sequence as set forth in SEQ ID NO: 43; and e) a fifth polypeptide chain having a sequence as set forth in SEQ ID NO: 44.

6. The liquid formulation according to any one of claims 1 to 5, wherein the buffer comprises a histidine-histidine hydrochloride buffer, a succinic acid-sodium succinate buffer and a citric acid-sodium citrate buffer; preferably, the buffer is a citric acid-sodium citrate buffer;
optionally, the buffer has a concentration of about 5 mM to about 20 mM, and preferably about 10 mM.

7. The liquid formulation according to any one of claims 1-6, wherein the bispecific anti-CD3/CD20 polypeptide complex is at a concentration of about 0.5 mg/mL to 20 mg/mL, or about 1 mg/mL to about 20 mg/mL, more preferably about 2 mg/mL to about 10 mg/mL, or about 2 mg/mL to about 5 mg/mL.

8. The liquid formulation according to any one of claims 1-7, wherein the liquid formulation has a pH of about 5.0 to about 7.5, or about 5.0 to about 6.5, preferably about 6.0 to about 6.5, and more preferably about 6, about 6.1 or about 6.2.

9. The liquid formulation according to any one of claims 1-8, wherein the saccharide is trehalose or sucrose;
preferably, the saccharide is trehalose;
optionally, the saccharide is at a concentration of about 180 mM to about 265 mM, about 230 mM to about 240 mM, or about 230 mM to about 235 mM, more preferably about 233 mM or about 232.6 mM.

10. The liquid formulation according to any one of claims 1 to 9, wherein the liquid formulation further comprises one or more of sodium chloride, arginine-hydrochloride, glycine and proline;
preferably, the sodium chloride, arginine-hydrochloride, glycine or proline is at a concentration of about 10 mM to about 300 mM, preferably about 20 mM to about 200 mM, or about 50 mM to about 150 mM, more preferably about 100 mM to about 110 mM, or about 100 mM to about 105 mM.

11. The liquid formulation according to any one of claims 1-10, wherein polysorbate 80 or polysorbate 20 is at a concentration of about 0.005% to about 0.08% (w/v), or about 0.01% to about 0.04% (w/v), more preferably about 0.02% (w/v).

12. The liquid formulation according to any one of claims 1-11, wherein the liquid formulation comprises:
(a) about 0.1 mg/mL to about 50 mg/mL bispecific anti-CD3/CD20 polypeptide complex,
(b) about 1 mM to about 30 mM citrate buffer,
(c) about 150 mM to about 320 mM saccharide; optionally, one or more of the following can be comprised: about 10 mM to about 300 mM sodium chloride, about 10 mM to about 300 mM arginine-hydrochloride, about 10 mM to about 300 mM glycine and about 10 mM to about 300 mM proline; and
(d) about 0.001% to about 0.1% (w/v) polysorbate 80 or polysorbate 20, wherein the liquid formulation has a pH of about 5.0 to about 7.5, preferably about 6.0 to about 6.5;
optionally, the liquid formulation comprises:
(a) about 0.1 mg/mL to about 50 mg/mL bispecific anti-CD3/CD20 polypeptide complex,
(b) about 1 mM to about 30 mM citrate buffer,
(c) about 150 mM to about 320 mM trehalose; optionally, one or more of the following can be comprised: about 10 mM to about 300 mM sodium chloride, about 10 mM to about 300 mM arginine-hydrochloride, about 10 mM to about 300 mM glycine and about 10 mM to about 300 mM proline; and
(d) about 0.001% to about 0.1% (w/v) polysorbate 80 or polysorbate 20, wherein the liquid formulation has a pH of about 5.0 to about 7.5, preferably about 6.0 to about 6.5;
optionally, the liquid formulation comprises:
(a) about 0.5 mg/mL to about 20 mg/mL bispecific anti-CD3/CD20 polypeptide complex,
(b) about 5 mM to about 20 mM citrate buffer,
(c) about 180 mM to about 265 mM trehalose; optionally, one or more of the following can be comprised: about 20 mM to about 200 mM sodium chloride, about 20 mM to about 200 mM arginine-hydrochloride, about 20 mM to about 200 mM glycine and about 20 mM to about 200 mM proline; and
(d) about 0.005% to about 0.08% (w/v) polysorbate 80 or polysorbate 20,
wherein the liquid formulation has a pH of about 5.0 to about 7.5, preferably about 6.0 to about 6.5;
optionally, the liquid formulation comprises:
(a) about 0.5 mg/mL to about 20 mg/mL bispecific anti-CD3/CD20 polypeptide complex,
(b) about 5 mM to about 20 mM citric acid-sodium citrate buffer,
(c) about 180 mM to about 265 mM trehalose; optionally, one or more of the following can be comprised: about 20 mM to about 200 mM sodium chloride, about 20 mM to about 200 mM arginine-hydrochloride, about 20 mM to about 200 mM glycine and about 20 mM to about 200 mM proline; and
(d) about 0.005% to about 0.08% (w/v) polysorbate 80 or polysorbate 20, wherein the liquid formulation has a pH of about 5.0 to about 7.5, preferably about 6.0 to about 6.5;
optionally, the liquid formulation comprises:
(a) about 0.1 mg/mL to about 50 mg/mL bispecific anti-CD3/CD20 polypeptide complex,
(b) about 1 mM to about 30 mM histidine-histidine hydrochloride or citric acid-sodium citrate buffer,
(c) about 150 mM to about 320 mM saccharide, and one or more of about 10 mM to about 300 mM sodium chloride, about 10 mM to about 300 mM arginine-hydrochloride, about 10 mM to about 300 mM glycine and about 10 mM to about 300 mM proline, and
(d) about 0.001% to about 0.1% (w/v) polysorbate 80 or polysorbate 20, wherein the liquid formulation has a pH of about 5.0 to about 7.5, preferably about 6.0 to about 6.5;
optionally, the liquid formulation comprises:
(a) about 0.5 mg/mL to about 20 mg/mL bispecific anti-CD3/CD20 polypeptide complex,
(b) about 5 mM to about 20 mM histidine-histidine hydrochloride or citric acid-sodium citrate buffer,
(c) about 180 mM to about 265 mM trehalose or sucrose, and about 20 mM to about 200 mM sodium chloride and/or about 20 mM to about 200 mM arginine-hydrochloride, and
(d) about 0.005% to about 0.08% (w/v) polysorbate 20 or polysorbate 80,
wherein the liquid formulation has a pH of about 5.0 to about 7.5, preferably about 6.0 to about 6.5;
optionally, the liquid formulation comprises:
(a) about 0.1 mg/mL to about 50 mg/mL bispecific anti-CD3/CD20 polypeptide complex,
(b) about 1 mM to about 30 mM citrate buffer,
(c) about 150 mM to about 320 mM trehalose, and about 10 mM to about 300 mM sodium chloride, and
(d) about 0.001% to about 0.1% (w/v) polysorbate 80 or polysorbate 20, wherein the liquid formulation has a pH of about 5.0 to about 7.5, preferably about 6.0 to about 6.5;
optionally, the liquid formulation comprises:
(a) about 0.5 mg/mL to about 20 mg/mL bispecific anti-CD3/CD20 polypeptide complex,
(b) about 5 mM to about 20 mM citrate buffer,
(c) about 180 mM to about 265 mM trehalose, and about 20 mM to about 200 mM sodium chloride, and
(d) about 0.005% to about 0.08% (w/v) polysorbate 80 or polysorbate 20, wherein the liquid formulation has a pH of about 5.0 to about 7.5, preferably about 6.0 to about 6.5,
optionally, the liquid pharmaceutical formulation comprises:
(a) 5 mg/mL bispecific anti-CD3/CD20 polypeptide complex,
(b) 10 mM citrate buffer,
(c) 230 mM to 235 mM trehalose, and 100 mM to 105 mM sodium chloride, and
(d) 0.02% (w/v) polysorbate 80,
wherein the liquid pharmaceutical formulation has a pH of 5.0 to 7.5, preferably 6.0 to 6.5;
optionally, the liquid pharmaceutical formulation comprises:
(a) 5 mg/mL bispecific anti-CD3/CD20 polypeptide complex,
(b) 10 mM citrate buffer,
(c) 233 mM trehalose, and 103 mM sodium chloride, and
(d) 0.02% (w/v) polysorbate 80,
wherein the liquid pharmaceutical formulation has a pH of 5.0 to 7.5, preferably 6.0 to 6.5;
optionally, the liquid pharmaceutical formulation comprises:
(a) 5 mg/mL bispecific anti-CD3/CD20 polypeptide complex,
(b) 10 mM citric acid-sodium citrate buffer,
(c) 230 mM to 235 mM trehalose, and 100 mM to 105 mM sodium chloride, and
(d) 0.02% (w/v) polysorbate 80,
wherein the liquid pharmaceutical formulation has a pH of 5.0 to 7.5, preferably 6.0 to 6.5;
optionally, the liquid pharmaceutical formulation comprises:
(a) 5 mg/mL bispecific anti-CD3/CD20 polypeptide complex,
(b) 10 mM citric acid-sodium citrate buffer,
(c) 233 mM trehalose, and 103 mM sodium chloride, and
(d) 0.02% (w/v) polysorbate 80,
wherein the liquid pharmaceutical formulation has a pH of 5.0 to 7.5, preferably 6.0 to 6.5.

13. A lyophilizate comprising a bispecific anti-CD3/CD20 polypeptide complex, wherein the lyophilizate is obtained by lyophilizing the liquid formulation according to any one of claims 1-12; or
the lyophilizate can form the liquid formulation according to any one of claims 1-12 upon reconstitution.

14. A pharmaceutical composition comprising a bispecific anti-CD3/CD20 polypeptide complex, wherein the pharmaceutical composition is a solution obtained by reconstituting the lyophilizate according to claim 13.

15. The liquid formulation according to any one of claims 1-12, the lyophilizate according to claim 13, or the pharmaceutical composition according to claim 14 for use in treating a CD20-related disease or disorder; optionally, the CD20-related disease or disorder is cancer; preferably the cancer is selected from the group consisting of, but is not limited to, lymphoma, lung cancer, liver cancer, cervical cancer, colon cancer, breast cancer, ovarian cancer, pancreatic cancer, melanoma, glioblastoma, prostate cancer, esophageal cancer and gastric cancer.

## Patentansprüche

1. Flüssige Formulierung, umfassend
(a) einen bispezifischen Anti-CD3/CD20-Polypeptidkomplex, wobei der bispezifische Anti-CD3/CD20-Polypeptidkomplex in einer Konzentration von etwa 0,1 mg/ml bis etwa 50 mg/ml vorliegt,
(b) einen Puffer, wobei der Puffer einen Histidinsalzpuffer, einen Succinatpuffer oder einen Citratpuffer umfasst,
wobei der Puffer eine Konzentration von etwa 1 mM bis etwa 30 mM aufweist;
(c) ein Saccharid, wobei das Saccharid in einer Konzentration von 150 mM bis etwa 320 mM vorliegt; und
(d) ein Polysorbat 80 oder ein Polysorbat 20, wobei das Polysorbat 80 oder Polysorbat 20 in einer Konzentration von etwa 0,001% bis etwa 0,1% (Gew./Vol.) vorliegt; wobei der Begriff "etwa" +/- 5 % bedeutet
wobei der bispezifische Anti-CD3/CD20-Polypeptidkomplex einen ersten Antigenbindungsabschnitt umfasst, der mit einem zweiten Antigenbindungsabschnitt assoziiert ist, wobei:
der erste Antigenbindungsabschnitt umfasst:
ein erstes Polypeptid, das vom N-Terminus zum C-Terminus eine erste variable Domäne (VH) der schweren Kette eines ersten Antikörpers umfasst, die funktionsfähig mit einer ersten konstanten Region (C1) des T-Zell-Rezeptors (TCR) verbunden ist, und
ein zweites Polypeptid, das vom N-Terminus zum C-Terminus eine erste variable Domäne (VL) der leichten Kette des ersten Antikörpers umfasst, die funktionsfähig mit einer zweiten konstanten Region (C2) des TCR verbunden ist,
wobei:
C1 und C2 ein Dimer bilden können, das mindestens eine nicht-native Interkettenbindung zwischen C1 und C2 umfasst, und die nicht-native Interkettenbindung das Dimer stabilisieren kann, wobei C1 ein konstruiertes Cβ umfasst, das SEQ ID NO: 61 umfasst, und C2 ein konstruiertes Cα umfasst, das SEQ ID NO: 62 umfasst,
und
der zweite Antigenbindungsabschnitt umfasst:
eine zweite variable Domäne der schweren Kette (VH2) eines zweiten Antikörpers, die funktionsfähig mit einer CH1-Domäne der schweren Kette des Antikörpers verbunden ist, und
eine zweite variable Domäne der leichten Kette (VL2) des zweiten Antikörpers, die funktionsfähig mit einer konstanten Domäne (CL) der leichten Kette des Antikörpers verbunden ist,
wobei:
einer der ersten Antigenbindungsabschnitte und der zweiten Antigenbindungsabschnitte ein Anti-CD3-Bindungsabschnitt ist und der andere ein Anti-CD20-Bindungsabschnitt ist;
der Anti-CD3-Bindungsabschnitt von einem Anti-CD3-Antikörper abgeleitet ist, umfassend:
a) eine schwere Kette CDR1, die eine Aminosäuresequenz von SEQ ID NO: 13 umfasst,
b) eine schwere Kette CDR2, die eine Aminosäuresequenz von SEQ ID NO: 14 umfasst,
c) eine schwere Kette CDR3, die eine Aminosäuresequenz von SEQ ID NO: 15 umfasst,
d) eine κ-Leichtketten-CDR1, die eine Aminosäuresequenz von SEQ ID NO: 16 umfasst,
e) eine κ-Leichtketten-CDR2, die eine Aminosäuresequenz von SEQ ID NO: 17 umfasst, und
f) eine κ-Leichtketten-CDR3, die eine Aminosäuresequenz von SEQ ID NO: 18 umfasst,
wobei der Anti-CD20-Bindungsabschnitt von einem Anti-CD20-Antikörper abgeleitet ist, umfassend:
a) eine schwere Kette CDR1, die eine Aminosäuresequenz von SEQ ID NO: 19 umfasst,
b) eine schwere Kette CDR2, die eine Aminosäuresequenz von SEQ ID NO: 20 umfasst,
c) eine schwere Kette CDR3, die eine Aminosäuresequenz von SEQ ID NO: 21 umfasst,
d) eine κ-Leichtketten-CDR1, die eine Aminosäuresequenz von SEQ ID NO: 22 umfasst,
e) eine κ-Leichtketten-CDR2, die eine Aminosäuresequenz von SEQ ID NO: 23 umfasst, und
f) eine κ-Leichtketten-CDR3, die eine Aminosäuresequenz von SEQ ID NO: 24 umfasst.

2. Flüssige Formulierung gemäß Anspruch 1, wobei der Anti-CD3-Bindungsabschnitt umfasst:
eine Sequenz der variablen Domäne der schweren Kette, die eine Sequenz von SEQ ID NO: 27 umfasst, und eine Sequenz der variable Domäne der leichten Kette, die eine Sequenz von SEQ ID NO: 28 umfasst; oder eine Sequenz der variablen Domäne der schweren Kette, die eine Sequenz von SEQ ID NO: 27 mit C-terminalen Deletionen der Aminosäuren SS umfasst,
und eine Sequenz der variablen Domäne der leichten Kette, die eine Sequenz von SEQ ID NO: 28 umfasst,
der Anti-CD20-Bindungsabschnitt umfasst:
eine Sequenz der variablen Domäne der schweren Kette, die eine Sequenz von SEQ ID NO: 31 umfasst, und eine Sequenz der variablen Domäne der leichten Kette, die eine Sequenz von SEQ ID NO: 32 umfasst.

3. Flüssige Formulierung gemäß Anspruch 1 oder 2, wobei
der erste Antigenbindungsabschnitt an eine erste Dimerisierungsdomäne gebunden ist, der zweite Antigenbindungsabschnitt an eine zweite Dimerisierungsdomäne gebunden ist und die erste Dimerisierungsdomäne und die zweite Dimerisierungsdomäne assoziiert sind;
gegebenenfalls die Assoziation über einen Linker, eine Disulfidbindung, eine Wasserstoffbrücke, eine elektrostatische Wechselwirkung, eine Salzbrücke oder eine hydrophobe-hydrophile Wechselwirkung oder eine Kombination davon erfolgt;
gegebenenfalls die erste Dimerisierungsdomäne und/oder die zweite Dimerisierungsdomäne mindestens einen Teil einer Antikörper-Scharnierregion, die gegebenenfalls aus IgG1, IgG2 oder IgG4 stammt, umfassen;
gegebenenfalls die erste Dimerisierungsdomäne und/oder die zweite Dimerisierungsdomäne eine Antikörper-CH2-Domäne und/oder eine Antikörper-CH3-Domäne umfassen;
gegebenenfalls die erste Dimerisierungsdomäne funktionsfähig mit der ersten TCR-Konstantenregion (C1) an einer dritten Verbindungsdomäne verbunden ist;
gegebenenfalls die zweite Dimerisierungsdomäne funktionsfähig mit dem C-Terminus der Antikörper-CH1-Konstantregion des zweiten Antigenbindungsabschnitts verbunden ist.

4. Flüssige Formulierung gemäß einem der Ansprüche 1 bis 3, wobei der bispezifische Anti-CD3/CD20-Polypeptidkomplex eine Kombination aus vier Polypeptidsequenzen umfasst: SEQ ID NO: 41, SEQ ID NO: 42, SEQ ID NO: 43 und SEQ ID NO: 44.

5. Flüssige Formulierung gemäß einem der Ansprüche 1 bis 4, wobei der bispezifische Anti-CD3/CD20-Polypeptidkomplex fünf Polypeptidketten umfasst: a) eine erste Polypeptidkette mit einer Sequenz gemäß SEQ ID NO: 41; b) eine zweite Polypeptidkette mit einer Sequenz gemäß SEQ ID NO: 42; c) eine dritte Polypeptidkette mit einer Sequenz gemäß SEQ ID NO: 43 angegeben; d) eine vierte Polypeptidkette mit einer Sequenz gemäß SEQ ID NO: 43; und e) eine fünfte Polypeptidkette mit einer Sequenz gemäß SEQ ID NO: 44.

6. Flüssige Formulierung gemäß einem der Ansprüche 1 bis 5, wobei der Puffer einen Histidin-Histidinhydrochlorid-Puffer, einen Bernsteinsäure-Natriumsuccinat-Puffer und einen Zitronensäure-Natriumcitrat-Puffer umfasst; vorzugsweise der Puffer ein Zitronensäure-Natriumcitrat-Puffer ist; gegebenenfalls der Puffer eine Konzentration von etwa 5 mM bis etwa 20 mM, vorzugsweise etwa 10 mM hat.

7. Flüssige Formulierung gemäß einem der Ansprüche 1 bis 6, wobei der bispezifische Anti-CD3/CD20-Polypeptidkomplex in einer Konzentration von etwa 0,5 mg/ml bis 20 mg/ml oder etwa 1 mg/ml bis etwa 20 mg/ml, vorzugsweise etwa 2 mg/ml bis etwa 10 mg/ml oder etwa 2 mg/ml bis etwa 5 mg/ml vorliegt.

8. Flüssige Formulierung gemäß einem der Ansprüche 1 bis 7, wobei die flüssige Formulierung einen pH-Wert von etwa 5,0 bis etwa 7,5 oder etwa 5,0 bis etwa 6,5, vorzugsweise etwa 6,0 bis etwa 6,5 und noch bevorzugter etwa 6, etwa 6,1 oder etwa 6,2 aufweist.

9. Flüssige Formulierung gemäß einem der Ansprüche 1 bis 8, wobei das Saccharid Trehalose oder Saccharose ist;
vorzugsweise das Saccharid Trehalose ist;
gegebenenfalls das Saccharid in einer Konzentration von etwa 180 mM bis etwa 265 mM, etwa 230 mM bis etwa 240 mM oder etwa 230 mM bis etwa 235 mM, vorzugsweise etwa 233 mM oder etwa 232,6 mM vorliegt.

10. Flüssige Formulierung gemäß einem der Ansprüche 1 bis 9, wobei die flüssige Formulierung ferner eines oder mehrere von Natriumchlorid, Arginin-Hydrochlorid, Glycin und Prolin umfasst;
vorzugsweise die Konzentration von Natriumchlorid, Argininhydrochlorid, Glycin oder Prolin bei etwa 10 mM bis etwa 300 mM liegt, vorzugsweise bei etwa 20 mM bis etwa 200 mM oder bei etwa 50 mM bis etwa 150 mM liegt, noch bevorzugter bei etwa 100 mM bis etwa 110 mM oder bei etwa 100 mM bis etwa 105 mM liegt.

11. Flüssige Formulierung gemäß einem der Ansprüche 1 bis 10, wobei Polysorbat 80 oder Polysorbat 20 in einer Konzentration von etwa 0,005% bis etwa 0,08% (Gew./Vol.) oder etwa 0,01% bis etwa 0,04% (Gew./Vol.), vorzugsweise etwa 0,02% (Gew./Vol.) vorliegt.

12. Flüssige Formulierung gemäß einem der Ansprüche 1 bis 11, wobei die flüssige Formulierung umfasst:
(a) etwa 0,1 mg/ml bis etwa 50 mg/ml bispezifischen Anti-CD3/CD20-Polypeptidkomplex,
(b) etwa 1 mM bis etwa 30 mM Citratpuffer,
(c) etwa 150 mM bis etwa 320 mM Saccharid; gegebenenfalls kann ein oder mehrere der folgenden Bestandteile enthalten sein: etwa 10 mM bis etwa 300 mM Natriumchlorid, etwa 10 mM bis etwa 300 mM Arginin-Hydrochlorid, etwa 10 mM bis etwa 300 mM Glycin und etwa 10 mM bis etwa 300 mM Prolin; und
(d) etwa 0,001% bis etwa 0,1% (Gew./Vol.) Polysorbat 80 oder Polysorbat 20, wobei die flüssige Formulierung einen pH-Wert von etwa 5,0 bis etwa 7,5, vorzugsweise etwa 6,0 bis etwa 6,5, aufweist;
die flüssige Formulierung gegebenenfalls umfasst:
(a) etwa 0,1 mg/ml bis etwa 50 mg/ml bispezifischen Anti-CD3/CD20-Polypeptidkomplex,
(b) etwa 1 mM bis etwa 30 mM Citratpuffer,
(c) etwa 150 mM bis etwa 320 mM Trehalose; gegebenenfalls kann ein oder können mehrere der folgenden Bestandteile enthalten sein: etwa 10 mM bis etwa 300 mM Natriumchlorid, etwa 10 mM bis etwa 300 mM Arginin-Hydrochlorid, etwa 10 mM bis etwa 300 mM Glycin und etwa 10 mM bis etwa 300 mM Prolin; und
(d) etwa 0,001% bis etwa 0,1 % (Gew./Vol.) Polysorbat 80 oder Polysorbat 20, wobei die flüssige Formulierung einen pH-Wert von etwa 5,0 bis etwa 7,5, vorzugsweise etwa 6,0 bis etwa 6,5, aufweist;
die flüssige Formulierung gegebenenfalls umfasst:
(a) etwa 0,5 mg/ml bis etwa 20 mg/ml bispezifischen Anti-CD3/CD20-Polypeptidkomplex,
(b) etwa 5 mM bis etwa 20 mM Citratpuffer,
(c) etwa 180 mM bis etwa 265 mM Trehalose; gegebenenfalls kann ein oder können mehrere der folgenden Bestandteile enthalten sein: etwa 20 mM bis etwa 200 mM Natriumchlorid, etwa 20 mM bis etwa 200 mM Arginin-Hydrochlorid, etwa 20 mM bis etwa 200 mM Glycin und etwa 20 mM bis etwa 200 mM Prolin; und
(d) etwa 0,005% bis etwa 0,08% (Gew./Vol.) Polysorbat 80 oder Polysorbat 20,
wobei die flüssige Formulierung einen pH-Wert von etwa 5,0 bis etwa 7,5, vorzugsweise etwa 6,0 bis etwa 6,5, aufweist;
die flüssige Formulierung gegebenenfalls umfasst:
(a) etwa 0,5 mg/ml bis etwa 20 mg/ml bispezifischen Anti-CD3/CD20-Polypeptidkomplex,
(b) etwa 5 mM bis etwa 20 mM Zitronensäure-Natriumcitrat-Puffer,
(c) etwa 180 mM bis etwa 265 mM Trehalose; gegebenenfalls kann ein oder können mehrere der folgenden Bestandteile enthalten sein: etwa 20 mM bis etwa 200 mM Natriumchlorid, etwa 20 mM bis etwa 200 mM Arginin-Hydrochlorid, etwa 20 mM bis etwa 200 mM Glycin und etwa 20 mM bis etwa 200 mM Prolin; und
(d) etwa 0,005% bis etwa 0,08% (Gew./Vol.) Polysorbat 80 oder Polysorbat 20,
wobei die flüssige Formulierung einen pH-Wert von etwa 5,0 bis etwa 7,5, vorzugsweise etwa 6,0 bis etwa 6,5, aufweist;
die flüssige Formulierung gegebenenfalls umfasst:
(a) etwa 0,1 mg/ml bis etwa 50 mg/ml bispezifischen Anti-CD3/CD20-Polypeptidkomplex,
(b) etwa 1 mM bis etwa 30 mM Histidin-Histidinhydrochlorid- oder Zitronensäure-Natriumcitrat-Puffer,
(c) etwa 150 mM bis etwa 320 mM Saccharid und eines oder mehrere von etwa 10 mM bis etwa 300 mM Natriumchlorid, etwa 10 mM bis etwa 300 mM Arginin-Hydrochlorid, etwa 10 mM bis etwa 300 mM Glycin und etwa 10 mM bis etwa 300 mM Prolin, und
(d) etwa 0,001% bis etwa 0,1% (Gew./Vol.) Polysorbat 80 oder Polysorbat 20, wobei die flüssige Formulierung einen pH-Wert von etwa 5,0 bis etwa 7,5, vorzugsweise etwa 6,0 bis etwa 6,5, aufweist;
die flüssige Formulierung gegebenenfalls umfasst:
(a) etwa 0,5 mg/ml bis etwa 20 mg/ml bispezifischen Anti-CD3/CD20-Polypeptidkomplex,
(b) etwa 5 mM bis etwa 20 mM Histidin-Histidinhydrochlorid- oder Zitronensäure-Natriumcitrat-Puffer,
(c) etwa 180 mM bis etwa 265 mM Trehalose oder Saccharose und etwa 20 mM bis etwa 200 mM Natriumchlorid und/oder etwa 20 mM bis etwa 200 mM Arginin-Hydrochlorid, und
(d) etwa 0,005% bis etwa 0,08% (Gew./Vol.) Polysorbat 20 oder Polysorbat 80,
wobei die flüssige Formulierung einen pH-Wert von etwa 5,0 bis etwa 7,5, vorzugsweise etwa 6,0 bis etwa 6,5, aufweist;
die flüssige Formulierung gegebenenfalls umfasst:
(a) etwa 0,1 mg/ml bis etwa 50 mg/ml bispezifischen Anti-CD3/CD20-Polypeptidkomplex,
(b) etwa 1 mM bis etwa 30 mM Citratpuffer,
(c) etwa 150 mM bis etwa 320 mM Trehalose und etwa 10 mM bis etwa 300 mM Natriumchlorid und
(d) etwa 0,001% bis etwa 0,1% (Gew./Vol.) Polysorbat 80 oder Polysorbat 20, wobei die flüssige Formulierung einen pH-Wert von etwa 5,0 bis etwa 7,5, vorzugsweise etwa 6,0 bis etwa 6,5, aufweist;
die flüssige Formulierung gegebenenfalls umfasst:
(a) etwa 0,5 mg/ml bis etwa 20 mg/ml bispezifischen Anti-CD3/CD20-Polypeptidkomplex,
(b) etwa 5 mM bis etwa 20 mM Citratpuffer,
(c) etwa 180 mM bis etwa 265 mM Trehalose und etwa 20 mM bis etwa 200 mM Natriumchlorid und
(d) etwa 0,005% bis etwa 0,08% (Gew./Vol.) Polysorbat 80 oder Polysorbat 20,
wobei die flüssige Formulierung einen pH-Wert von etwa 5,0 bis etwa 7,5, vorzugsweise etwa 6,0 bis etwa 6,5, aufweist,
die flüssige pharmazeutische Formulierung gegebenenfalls umfasst:
(a) 5 mg/ml bispezifischen Anti-CD3/CD20-Polypeptidkomplex,
(b) 10 mM Citratpuffer,
(c) 230 mM bis 235 mM Trehalose und 100 mM bis 105 mM Natriumchlorid und
(d) 0,02% (w/v) Polysorbat 80,
wobei die flüssige pharmazeutische Formulierung einen pH-Wert von 5,0 bis 7,5, vorzugsweise 6,0 bis 6,5, aufweist;
die flüssige pharmazeutische Formulierung gegebenenfalls umfasst:
(a) 5 mg/ml bispezifischen Anti-CD3/CD20-Polypeptidkomplex,
(b) 10 mM Citratpuffer,
(c) 233 mM Trehalose und 103 mM Natriumchlorid und
(d) 0,02% (w/v) Polysorbat 80,
wobei die flüssige pharmazeutische Formulierung einen pH-Wert von 5,0 bis 7,5, vorzugsweise von 6,0 bis 6,5, aufweist;
die flüssige pharmazeutische Formulierung gegebenenfalls umfasst:
(a) 5 mg/ml bispezifischen Anti-CD3/CD20-Polypeptidkomplex,
(b) 10 mM Zitronensäure-Natriumcitrat-Puffer,
(c) 230 mM bis 235 mM Trehalose und 100 mM bis 105 mM Natriumchlorid und
(d) 0,02% (w/v) Polysorbat 80,
wobei die flüssige pharmazeutische Formulierung einen pH-Wert von 5,0 bis 7,5, vorzugsweise von 6,0 bis 6,5, aufweist;
die flüssige pharmazeutische Formulierung gegebenenfalls umfasst:
(a) 5 mg/ml bispezifischen Anti-CD3/CD20-Polypeptidkomplex,
(b) 10 mM Zitronensäure-Natriumcitrat-Puffer,
(c) 233 mM Trehalose und 103 mM Natriumchlorid und
(d) 0,02% (w/v) Polysorbat 80,
wobei die flüssige pharmazeutische Formulierung einen pH-Wert von 5,0 bis 7,5, vorzugsweise von 6,0 bis 6,5, aufweist.

13. Lyophilisat, das einen bispezifischen Anti-CD3/CD20-Polypeptidkomplex umfasst, wobei das Lyophilisat durch Lyophilisieren der flüssigen Formulierung gemäß einem der Ansprüche 1 bis 12 erhalten wird; oder
das Lyophilisat nach Rekonstitution die flüssige Formulierung gemäß einem der Ansprüche 1 bis 12 bilden kann.

14. Pharmazeutische Zusammensetzung, umfassend einen bispezifischen Anti-CD3/CD20-Polypeptidkomplex, wobei die pharmazeutische Zusammensetzung eine Lösung ist, die durch Rekonstitution des Lyophilisats gemäß Anspruch 13 erhalten wird.

15. Flüssige Formulierung gemäß einem der Ansprüche 1 bis 12, Lyophilisat gemäß Anspruch 13 oder pharmazeutische Zusammensetzung gemäß Anspruch 14 zur Verwendung bei der Behandlung einer CD20-bezogenen Krankheit oder Störung;
wobei gegebenenfalls die CD20-bezogene Krankheit oder Störung Krebs ist; vorzugsweise Krebs ausgewählt aus der Gruppe bestehend aus, aber nicht beschränkt auf Lymphom, Lungenkrebs, Leberkrebs, Gebärmutterhalskrebs, Darmkrebs, Brustkrebs, Eierstockkrebs, Bauchspeicheldrüsenkrebs, Melanom, Glioblastom, Prostatakrebs, Speiseröhrenkrebs und Magenkrebs.

## Revendications

1. Formulation liquide comprenant
(a) un complexe polypeptidique bispécifique anti-CD3/CD20, dans lequel le complexe polypeptidique bispécifique anti-CD3/CD20 est à une concentration d'environ 0,1 mg/mL à environ 50 mg/mL
(b) un tampon, dans lequel le tampon comprend un tampon à base de sel d'histidine, un tampon à base de succinate ou un tampon à base de citrate dans lequel le tampon a une concentration d'environ 1 mM à environ 30 mM ;
(c) un saccharide, dans lequel le saccharide est à une concentration de 150 mM à environ 320 mM ; et
(d) un polysorbate 80 ou un polysorbate 20, dans lequel le polysorbate 80 ou le polysorbate 20 est à une concentration d'environ 0,001 % à environ 0,1 % (p/v) ; dans lequel le terme « environ » signifie +/- 5 %
dans lequel le complexe polypeptidique bispécifique anti-CD3/CD20 comprend une première partie de liaison à l'antigène associée à une deuxième partie de liaison à l'antigène, dans lequel :
la première partie de liaison à l'antigène comprend :
un premier polypeptide, comprenant de l'extrémité N-terminale à l'extrémité C-terminale un premier domaine variable (VH) de chaîne lourde d'un premier anticorps lié de manière fonctionnelle à une première région constante (C1) de récepteur de cellules T (TCR), et
un deuxième polypeptide, comprenant de l'extrémité N-terminale à l'extrémité C-terminale un premier domaine variable de chaîne légère (VL) du premier anticorps lié de manière fonctionnelle à une deuxième région constante du TCR (C2),
dans lequel :
C1 et C2 peuvent former un dimère comprenant au moins une liaison interchaîne non native entre C1 et C2, et la liaison interchaîne non native peut stabiliser le dimère, dans lequel C1 comprend une chaîne Cβ modifiée comprenant la SEQ ID NO : 61, et C2 comprend une chaîne Cα modifiée comprenant la SEQ ID NO : 62,
et
la deuxième partie de liaison à l'antigène comprend :
un deuxième domaine variable de chaîne lourde (VH2) d'un deuxième anticorps lié de manière fonctionnelle à un domaine CH1 de chaîne lourde d'anticorps, et
un deuxième domaine variable de chaîne légère (VL2) du deuxième anticorps lié de manière fonctionnelle à un domaine constant de chaîne légère d'anticorps (CL),
dans lequel :
l'une de la première partie se liant à l'antigène et de la deuxième partie se liant à l'antigène est une partie se liant à l'anti-CD3, et l'autre est une partie se liant à l'anti-CD20 ;
la partie de liaison anti-CD3 est dérivée d'un anticorps anti-CD3, comprenant :
a) une chaîne lourde CDR1 comprenant une séquence d'acides aminés de SEQ ID NO : 13,
b) une chaîne lourde CDR2 comprenant une séquence d'acides aminés de SEQ ID NO : 14,
c) une chaîne lourde CDR3 comprenant une séquence d'acides aminés de SEQ ID NO : 15,
d) une chaîne légère κ CDR1 comprenant une séquence d'acides aminés de SEQ ID NO : 16,
e) une chaîne légère κ CDR2 comprenant une séquence d'acides aminés de SEQ ID NO : 17, et
f) une chaîne légère κ CDR3 comprenant une séquence d'acides aminés de SEQ ID NO : 18,
la partie de liaison anti-CD20 est dérivée d'un anticorps anti-CD20, comprenant :
a) une chaîne lourde CDR1 comprenant une séquence d'acides aminés de SEQ ID NO : 19,
b) une chaîne lourde CDR2 comprenant une séquence d'acides aminés de SEQ ID NO : 20,
c) une chaîne lourde CDR3 comprenant une séquence d'acides aminés de SEQ ID NO : 21,
d) une chaîne légère κ CDR1 comprenant une séquence d'acides aminés de SEQ ID NO : 22,
e) une chaîne légère κ CDR2 comprenant une séquence d'acides aminés de SEQ ID NO : 23, et
f) une chaîne légère κ CDR3 comprenant une séquence d'acides aminés de SEQ ID NO : 24.

2. Formulation liquide selon la revendication 1, dans laquelle
la partie de liaison anti-CD3 comprend : une séquence de domaine variable de chaîne lourde comprenant une séquence de SEQ ID NO : 27, et une séquence de domaine variable de chaîne légère comprenant une séquence de SEQ ID NO : 28 ; ou une séquence de domaine variable de chaîne lourde comprenant une séquence de SEQ ID NO : 27 avec des délétions C-terminales des acides aminés SS, et une séquence de domaine variable de chaîne légère comprenant une séquence de SEQ ID NO : 28,
la partie de liaison anti-CD20 comprend : une séquence de domaine variable de chaîne lourde comprenant une séquence de SEQ ID NO : 31, et une séquence de domaine variable de chaîne légère comprenant une séquence de SEQ ID NO : 32.

3. Formulation liquide selon la revendication 1 ou 2, dans laquelle la première partie de liaison à l'antigène est liée à un premier domaine de dimérisation, la deuxième partie de liaison à l'antigène est liée à un deuxième domaine de dimérisation, et le premier domaine de dimérisation et le deuxième domaine de dimérisation sont associés ;
facultativement, l'association se fait par l'intermédiaire d'un lieur, d'une liaison disulfure, d'une liaison hydrogène, d'une interaction électrostatique, d'un pont salin ou d'une interaction hydrophobe-hydrophile ou d'une combinaison de ceux-ci ;
facultativement, le premier domaine de dimérisation et/ou le deuxième domaine de dimérisation comprennent au moins une partie d'une région charnière d'anticorps, qui est facultativement issue d'IgG1, d'IgG2 ou d'IgG4 ; facultativement, le premier domaine de dimérisation et/ou le deuxième domaine de dimérisation comprennent un domaine CH2 d'anticorps et/ou un domaine CH3 d'anticorps ;
facultativement, le premier domaine de dimérisation est lié de manière fonctionnelle à la première région constante du TCR (C1) au niveau d'un troisième domaine de jonction ;
facultativement, le deuxième domaine de dimérisation est lié de manière fonctionnelle à l'extrémité C-terminale de la région constante CH1 de l'anticorps de la deuxième partie de liaison à l'antigène.

4. Formulation liquide selon l'une quelconque des revendications 1 à 3, dans laquelle le complexe polypeptidique bispécifique anti-CD3/CD20 comprend une combinaison de quatre séquences polypeptidiques : SEQ ID NO : 41, SEQ ID NO : 42, SEQ ID NO : 43 et SEQ ID NO : 44.

5. Formulation liquide selon l'une quelconque des revendications 1 à 4, dans laquelle le complexe polypeptidique bispécifique anti-CD3/CD20 comprend cinq chaînes polypeptidiques : a) une première chaîne polypeptidique ayant une séquence telle que définie dans SEQ ID NO : 41 ; b) une deuxième chaîne polypeptidique ayant une séquence telle que définie dans SEQ ID NO : 42 ; c) une troisième chaîne polypeptidique ayant une séquence telle que définie dans SEQ ID NO : 43 ; d) une quatrième chaîne polypeptidique ayant une séquence telle que définie dans SEQ ID NO : 43 ; et e) une cinquième chaîne polypeptidique ayant une séquence telle que définie dans SEQ ID NO : 44.

6. Formulation liquide selon l'une quelconque des revendications 1 à 5, dans laquelle le tampon comprend un tampon histidine-chlorhydrate d'histidine, un tampon acide succinique-succinate de sodium et un tampon acide citrique-citrate de sodium; de préférence, le tampon est un tampon acide citrique-citrate de sodium;
facultativement, le tampon a une concentration d'environ 5 mM à environ 20 mM, et de préférence d'environ 10 mM.

7. Formulation liquide selon l'une quelconque des revendications 1 à 6, dans laquelle le complexe polypeptidique bispécifique anti-CD3/CD20 est à une concentration d'environ 0,5 mg/mL à 20 mg/mL, ou d'environ 1 mg/mL à environ 20 mg/mL, de préférence d'environ 2 mg/mL à environ 10 mg/mL, ou d'environ 2 mg/mL à environ 5 mg/mL.

8. Formulation liquide selon l'une quelconque des revendications 1 à 7, dans laquelle la formulation liquide a un pH d'environ 5,0 à environ 7,5, ou d'environ 5,0 à environ 6,5, de préférence d'environ 6,0 à environ 6,5, et de préférence encore d'environ 6, d'environ 6,1 ou d'environ 6,2.

9. Formulation liquide selon l'une quelconque des revendications 1 à 8, dans laquelle le saccharide est le tréhalose ou le saccharose ;
de préférence, le saccharide est le tréhalose ;
facultativement, le saccharide est à une concentration d'environ 180 mM à environ 265 mM, d'environ 230 mM à environ 240 mM, ou d'environ 230 mM à environ 235 mM, de préférence d'environ 233 mM ou d'environ 232,6 mM.

10. Formulation liquide selon l'une quelconque des revendications 1 à 9, dans laquelle la formulation liquide comprend en outre un ou plusieurs des composés suivants : chlorure de sodium, chlorhydrate d'arginine, glycine et proline ;
de préférence, le chlorure de sodium, le chlorhydrate d'arginine, la glycine ou la proline sont présents à une concentration d'environ 10 mM à environ 300 mM, de préférence d'environ 20 mM à environ 200 mM, ou d'environ 50 mM à environ 150 mM, de préférence encore d'environ 100 mM à environ 110 mM, ou d'environ 100 mM à environ 105 mM.

11. Formulation liquide selon l'une quelconque des revendications 1 à 10, dans laquelle le polysorbate 80 ou le polysorbate 20 est présent à une concentration d'environ 0,005 % à environ 0,08 % (p/v), ou d'environ 0,01 % à environ 0,04 % (p/v), de préférence d'environ 0,02 % (p/v).

12. Formulation liquide selon l'une quelconque des revendications 1 à 11, dans laquelle la formulation liquide comprend :
(a) environ 0,1 mg/mL à environ 50 mg/mL de complexe polypeptidique bispécifique anti-CD3/CD20,
(b) environ 1 mM à environ 30 mM de tampon citrate,
(c) environ 150 mM à environ 320 mM de saccharide ; facultativement, un ou plusieurs des composants suivants peuvent être compris : environ 10 mM à environ 300 mM de chlorure de sodium, environ 10 mM à environ 300 mM de chlorhydrate d'arginine, environ 10 mM à environ 300 mM de glycine et environ 10 mM à environ 300 mM de proline ; et
(d) environ 0,001 % à environ 0,1 % (p/v) de polysorbate 80 ou de polysorbate 20,
la formulation liquide ayant un pH d'environ 5,0 à environ 7,5, de préférence d'environ 6,0 à environ 6,5 ;
facultativement, la formulation liquide comprend :
(a) environ 0,1 mg/mL à environ 50 mg/mL de complexe polypeptidique bispécifique anti-CD3/CD20,
(b) environ 1 mM à environ 30 mM de tampon citrate,
(c) environ 150 mM à environ 320 mM de tréhalose ; facultativement, un ou plusieurs des composants suivants peuvent être compris : environ 10 mM à environ 300 mM de chlorure de sodium, environ 10 mM à environ 300 mM de chlorhydrate d'arginine, environ 10 mM à environ 300 mM de glycine et environ 10 mM à environ 300 mM de proline ; et
(d) environ 0,001 % à environ 0,1 % (p/v) de polysorbate 80 ou de polysorbate 20,
la formulation liquide ayant un pH d'environ 5,0 à environ 7,5, de préférence d'environ 6,0 à environ 6,5 ;
facultativement, la formulation liquide comprend :
(a) environ 0,5 mg/mL à environ 20 mg/mL de complexe polypeptidique bispécifique anti-CD3/CD20,
(b) environ 5 mM à environ 20 mM de tampon citrate,
(c) environ 180 mM à environ 265 mM de tréhalose ; facultativement, un ou plusieurs des composants suivants peuvent être compris : environ 20 mM à environ 200 mM de chlorure de sodium, environ 20 mM à environ 200 mM de chlorhydrate d'arginine, environ 20 mM à environ 200 mM de glycine et environ 20 mM à environ 200 mM de proline ; et
(d) environ 0,005 % à environ 0,08 % (p/v) de polysorbate 80 ou de polysorbate 20,
la formulation liquide ayant un pH d'environ 5,0 à environ 7,5, de préférence d'environ 6,0 à environ 6,5 ;
facultativement, la formulation liquide comprend :
(a) environ 0,5 mg/mL à environ 20 mg/mL de complexe polypeptidique bispécifique anti-CD3/CD20,
(b) environ 5 mM à environ 20 mM de tampon acide citrique-citrate de sodium,
(c) environ 180 mM à environ 265 mM de tréhalose ; facultativement, un ou plusieurs des composants suivants peuvent être compris : environ 20 mM à environ 200 mM de chlorure de sodium, environ 20 mM à environ 200 mM de chlorhydrate d'arginine, environ 20 mM à environ 200 mM de glycine et environ 20 mM à environ 200 mM de proline ; et
(d) environ 0,005 % à environ 0,08 % (p/v) de polysorbate 80 ou de polysorbate 20,
dans laquelle la formulation liquide a un pH d'environ 5,0 à environ 7,5, de préférence d'environ 6,0 à environ 6,5 ;
facultativement, la formulation liquide comprend :
(a) environ 0,1 mg/mL à environ 50 mg/mL de complexe polypeptidique bispécifique anti-CD3/CD20,
(b) environ 1 mM à environ 30 mM de tampon histidine-chlorhydrate d'histidine ou de tampon acide citrique-citrate de sodium,
(c) environ 150 mM à environ 320 mM de saccharide, et un ou plusieurs des composés suivants : environ 10 mM à environ 300 mM de chlorure de sodium, environ 10 mM à environ 300 mM de chlorhydrate d'arginine, environ 10 mM à environ 300 mM de glycine et environ 10 mM à environ 300 mM de proline, et
(d) environ 0,001 % à environ 0,1 % (p/v) de polysorbate 80 ou de polysorbate 20,
la formulation liquide ayant un pH d'environ 5,0 à environ 7,5, de préférence d'environ 6,0 à environ 6,5 ;
facultativement, la formulation liquide comprend :
(a) environ 0,5 mg/mL à environ 20 mg/mL de complexe polypeptidique bispécifique anti-CD3/CD20,
(b) environ 5 mM à environ 20 mM de tampon histidine-chlorhydrate d'histidine ou de tampon acide citrique-citrate de sodium,
(c) environ 180 mM à environ 265 mM de tréhalose ou de saccharose, et environ 20 mM à environ 200 mM de chlorure de sodium et/ou environ 20 mM à environ 200 mM de chlorhydrate d'arginine, et
(d) environ 0,005 % à environ 0,08 % (p/v) de polysorbate 20 ou de polysorbate 80,
la formulation liquide ayant un pH d'environ 5,0 à environ 7,5, de préférence d'environ 6,0 à environ 6,5 ;
facultativement, la formulation liquide comprend :
(a) environ 0,1 mg/mL à environ 50 mg/mL de complexe polypeptidique bispécifique anti-CD3/CD20,
(b) environ 1 mM à environ 30 mM de tampon citrate,
(c) environ 150 mM à environ 320 mM de tréhalose, et environ 10 mM à environ 300 mM de chlorure de sodium, et
(d) environ 0,001 % à environ 0,1 % (p/v) de polysorbate 80 ou de polysorbate 20,
la formulation liquide ayant un pH d'environ 5,0 à environ 7,5, de préférence d'environ 6,0 à environ 6,5 ;
facultativement, la formulation liquide comprend :
(a) environ 0,5 mg/mL à environ 20 mg/mL de complexe polypeptidique bispécifique anti-CD3/CD20,
(b) environ 5 mM à environ 20 mM de tampon citrate,
(c) environ 180 mM à environ 265 mM de tréhalose, et environ 20 mM à environ 200 mM de chlorure de sodium, et
(d) environ 0,005 % à environ 0,08 % (p/v) de polysorbate 80 ou de polysorbate 20,
la formulation liquide ayant un pH d'environ 5,0 à environ 7,5, de préférence d'environ 6,0 à environ 6,5,
en option, la formulation pharmaceutique liquide comprend :
(a) 5 mg/mL de complexe polypeptidique bispécifique anti-CD3/CD20,
(b) 10 mM de tampon citrate,
(c) 230 mM à 235 mM de tréhalose, et 100 mM à 105 mM de chlorure de sodium, et
(d) 0,02 % (p/v) de polysorbate 80,
la formulation pharmaceutique liquide ayant un pH de 5,0 à 7,5, de préférence de 6,0 à 6,5 ;
facultativement, la formulation pharmaceutique liquide comprend :
(a) 5 mg/mL de complexe polypeptidique bispécifique anti-CD3/CD20,
(b) 10 mM de tampon citrate,
(c) 233 mM de tréhalose, et 103 mM de chlorure de sodium, et
(d) 0,02 % (p/v) de polysorbate 80,
la formulation pharmaceutique liquide ayant un pH compris entre 5,0 et 7,5, de préférence entre 6,0 et 6,5 ;
facultativement, la formulation pharmaceutique liquide comprend :
(a) 5 mg/mL de complexe polypeptidique bispécifique anti-CD3/CD20,
(b) 10 mM de tampon acide citrique-citrate de sodium,
(c) 230 mM à 235 mM de tréhalose, et 100 mM à 105 mM de chlorure de sodium, et
(d) 0,02 % (p/v) de polysorbate 80,
dans laquelle la formulation pharmaceutique liquide a un pH de 5,0 à 7,5, de préférence de 6,0 à 6,5 ;
facultativement, la formulation pharmaceutique liquide comprend :
(a) 5 mg/mL de complexe polypeptidique bispécifique anti-CD3/CD20,
(b) 10 mM de tampon acide citrique-citrate de sodium,
(c) 233 mM de tréhalose et 103 mM de chlorure de sodium, et
(d) 0,02 % (p/v) de polysorbate 80,
la formulation pharmaceutique liquide ayant un pH compris entre 5,0 et 7,5, de préférence entre 6,0 et 6,5.

13. Lyophilisat comprenant un complexe polypeptidique bispécifique anti-CD3/CD20, dans lequel le lyophilisat est obtenu par lyophilisation de la formulation liquide selon l'une quelconque des revendications 1 à 12 ; ou
le lyophilisat peut former la formulation liquide selon l'une quelconque des revendications 1 à 12 après reconstitution.

14. Composition pharmaceutique comprenant un complexe polypeptidique bispécifique anti-CD3/CD20, dans laquelle la composition pharmaceutique est une solution obtenue par reconstitution du lyophilisat selon la revendication 13.

15. La formulation liquide selon l'une quelconque des revendications 1 à 12, le lyophilisat selon la revendication 13 ou la composition pharmaceutique selon la revendication 14 destinés à être utilisés dans le traitement d'une maladie ou d'un trouble lié au CD20 ;
facultativement, la maladie ou le trouble lié au CD20 est un cancer ; de préférence, le cancer est choisi dans le groupe constitué, sans s'y limiter, du lymphome, du cancer du poumon, du cancer du foie, du cancer du col de l'utérus, du cancer du côlon, du cancer du sein, du cancer de l'ovaire, du cancer du pancréas, du mélanome, du glioblastome, du cancer de la prostate, du cancer de l'œsophage et du cancer de l'estomac.
